(19) Europäisches Patentamt
European Patent Office
Office européen des brevets

(11) EP 2 389 221 B1

(12) **EUROPEAN PATENT SPECIFICATION**

(45) Date of publication and mention of the grant of the patent:
**16.08.2023 Bulletin 2023/33**

(21) Application number: **10733939.2**

(22) Date of filing: **22.01.2010**

(51) International Patent Classification (IPC):
***A61K 47/34*** (2017.01) ***A61F 9/007*** (2006.01)
***A61K 31/5575*** (2006.01) ***A61K 45/06*** (2006.01)
***A61F 9/00*** (2006.01) ***A61K 9/00*** (2006.01)

(52) Cooperative Patent Classification (CPC):
**A61K 9/0051; A61F 9/0017; A61F 9/00781; A61K 31/165; A61K 31/216; A61K 31/5575; A61K 45/06; A61K 47/24; A61K 47/34; A61P 27/02; A61P 27/06;** A61F 9/00772

(86) International application number:
**PCT/US2010/021868**

(87) International publication number:
**WO 2010/085696 (29.07.2010 Gazette 2010/30)**

(54) **SUSTAINED RELEASED DELIVERY OF ONE OR MORE AGENTS**

VERZÖGERTE FREIGESETZTE LIEFERUNG VON EINEM ODER MEHR WIRKSTOFFEN

SYSTÈME D'ADMINISTRATION À LIBÉRATION PROLONGÉE D'UN OU PLUSIEURS AGENTS

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **23.01.2009 US 146860 P**
**16.02.2009 US 152909 P**
**27.07.2009 US 228894 P**
**18.09.2009 US 277000 P**

(43) Date of publication of application:
**30.11.2011 Bulletin 2011/48**

(73) Proprietor: **Mati Therapeutics Inc.**
**Austin, TX 78746 (US)**

(72) Inventors:
- **BUTUNER, Zuhal**
  **Oakville, ON L6J 3Y3 (CA)**
- **UTKHEDE, Deepank**
  **Surrey, BC V3S 9C6 (CA)**
- **SIM, Sylvie**
  **La Vista, NE 68128 (US)**
- **WISEMAN, David, J.**
  **Surrey, BC V3V 3C2 (CA)**

(74) Representative: **HGF**
**HGF Limited**
**1 City Walk**
**Leeds LS11 9DX (GB)**

(56) References cited:
**WO-A2-2006/002366**
**US-A1- 2002 028 181**
**US-A1- 2003 152 522**
**US-A1- 2004 071 761**
**US-A1- 2008 181 930**

EP 2 389 221 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

TECHNICAL FIELD

This patent document pertains generally to ophthalmic devices, and particularly to ocular implants. More particularly, but not by way of limitation, this patent document pertains to lacrimal implant delivery systems (e.g., punctal plugs including a drug core), methods of making such implant delivery systems, and methods of treating diseases or disorders, including ocular diseases or disorders using, at least in part, such implant delivery systems.

INTRODUCTION

A variety of challenges face patients and physicians in the area of drug delivery, for example, ocular drug delivery. In particular, the repetitive nature of the therapies (multiple injections, instilling multiple eye drop regimens per day), the associated costs, and the lack of patient compliance may significantly impact the efficacy of the therapies available, leading to reduction in vision and many times blindness.

Patient compliance in taking the medications, for example, instilling the eye drops, can be erratic, and in some cases, patients may not follow the directed treatment regime. Lack of compliance can include, failure to instill the drops, ineffective technique (instilling less than required), excessive use of the drops (leading to systemic side effects), and use of non-prescribed drops or failure to follow the treatment regime requiring multiple types of drops. Many of the medications may require the patient to instill them up to 4 times a day.

For example, glaucoma is a collection of disorders characterized by progressive visual field loss due to optic nerve damage. It is one of the leading causes of blindness in the United States, affecting 1-2% of individuals 60 years of age and older. While there are many risk factors associated with the development of glaucoma (e.g., age, race, myopia, family history, and injury), elevated intraocular pressure, also known as ocular hypertension, is a risk factor successfully manipulatable and correlated with the reduction of glaucomatous optic neuropathy. Public health figures estimate that 2.5 million Americans manifest ocular hypertension.

In order to treat glaucoma and ocular hypertension, drugs can be administered to the eye. A conventional method of drug delivery is by topical drop application to the eye's surface. Topical eye drops, though effective, can be inefficient. For instance, when an eye drop is instilled in an eye, it often overfills the conjunctival sac (i.e., the pocket between the eye and the associated lids) causing a substantial portion of the drop to be lost due to overflow of the lid margin and spillage onto the cheek. In addition, a large portion of the drop remaining on the ocular surface can be washed away into and through a lacrimal canaliculus, thereby diluting the concentration of the drug before it can treat the eye. Further, in some cases, topically applied medications have a peak ocular effect within about two hours, after which additional applications of the medications should be performed to maintain the therapeutic benefit.

To compound ocular management difficulty, subjects often do not use their eye drops as prescribed. Noncompliance rates by drop users of 25% and greater have been previously reported. This poor compliance can be due to, for example, forgetfulness or an initial stinging or burning sensation caused by the eye drop and experience by a subject. Instilling eye drops in one's own eye can be difficult, in part because of the normal reflex to protect the eye.

Therefore, one or more drops may miss the eye. Older subjects may have additional problems instilling drops due to arthritis, unsteadiness, and decreased vision. Pediatric and psychiatric populations pose difficulties as well.

One promising approach to ocular drug delivery is to place an implant that releases a drug in tissue in or near the eye.

WO2006/022366 provides implantable medical devices that are fabricated of biodegradable materials for delivery of bioactive agent to limited access regions of a patient's body, such as the eye, and further provides methods of treatment utilizing the devices.

EXEMPLARY ASPECTS AND EMBODIMENTS OF THE INVENTION

In an aspect, the present invention provides an implant (200) configured for disposition within or adjacent to a body cavity, tissue, duct, or fluid, the implant comprising:
a drug core (214) comprising:

(a) a matrix (254, 362, 454) including a polymer;

(b) a therapeutic agent (252, 360, 452) contained within the matrix, and

(c) an excipient dissolved or dispersed within the matrix, the excipient configured to any of,

(1) modify a release rate of the therapeutic agent into the body cavity, tissue, duct, or fluid relative to a comparable release rate in the absence of an excipient;

(2) increase a loading of the therapeutic agent substantially uniformly dissolved or dispersed within the matrix, relative to a comparable loading of the therapeutic agent that is substantially uniformly dissolved or dispersed, in the absence of an excipient;

(3) increase retention of the agent at or adjacent to a site of release in a living body or increase penetration of adjacent body tissue by the agent, or both, relative to the retention or penetration or both from a comparable implant in the absence of the excipient;

or any combination thereof, wherein an amount of the therapeutic agent in a volumetric portion of the matrix is similar to an amount of the therapeutic agent in any other equal volumetric portion of the matrix; characterized in that the polymer of the matrix is a non-biodegradable polymer; and wherein the implant is an ocular implant, adapted for disposition through a punctum and into a canaliculus of a human eye for release of the therapeutic agent therefrom.
The present inventors have recognized various promising techniques to deliver one or more drugs (also referred to as "therapeutic agents" or "agents"), including, for example, one or more anti-glaucoma agents, to an eye. These techniques include methods for sustained release delivery of a drug, including for example, one or more anti-glaucoma agents from a punctum of a subject onto the ocular surface, and hence ocular tissues, using a lacrimal implant delivery system over an extended period of time. In various embodiments, the drug is an anti-glaucoma therapeutic agent, such as a prostaglandin, for example, latanoprost. The drug, for example latanoprost or other prostaglandin, can be released at one or more therapeutic levels. The drug comprises a medicinal material, a compound or a mixture thereof, that is suitable and medically indicated for treatment of a malcondition in a patient. Specific examples of types or classes of agents for use with the present invention include a glaucoma medication, a muscarinic agent, a beta blocker, an alpha agonist, a carbonic anhydrase inhibitor, or a prostaglandin or prostaglandin analog; an antiinflammatory agent; an anti- infective agent; a dry eye medication; or any combination thereof. More specifically, an example of a glaucoma medication is a prostaglandin or a prostaglandin analog. An example of a muscarinic agent is pilocarpine. an example of a beta blocked is betaxolol. An example of an alpha agonist is brimonidine. Examples of a carbonic anhydrase inhibitor aredorzolamide or brinzolamide. Examples of an antiinflammatory agent include a steroid, a soft steroid, or a non-steroidal antiinflammatory drug (NSAID) such as ibuprofen. An example of an analgesic includes salicylic acid and acetaminophen. An antibiotic (antibacterial) can be a beta-lactam antibiotic, a macrocyclic antibiotic such as erythromycin, a fluoroquinolone, or the like. An antiviral compound can be a reverse transcriptase inhibitor or a viral protease inhibitor. An antimycotic can be a triazole antifungal compound. A dry eye medication can be cyclosporine, olapatadine, a delmulcent, or sodium hyaluronate.

The invention is disclosed by the appended claims.

BRIEF DESCRIPTION OF THE DRAWINGS

This patent document file contains at least one drawing executed in color. Copies of this patent document in the form of a patent or patent application publication with color drawings will be provided by the Office upon request and payment of the necessary fee. In the drawings, like numerals can be used to describe similar components throughout the several views. The drawings illustrate generally, by way of example, but not by way of limitation, various embodiments discussed in the present document.

FIG. 1 illustrates an example of release profile curves, under accelerated dissolution conditions for a time period of about 50 hours, of lacrimal implants including 44 micrograms of an anti-glaucoma agent and lacrimal implants including 21 micrograms of an anti-glaucoma agent.
FIG. 2A illustrates an example of an isometric view of a lacrimal implant configured to be retained at least partially within a lacrimalpunctum and canalicular anatomy.
FIG. 2B illustrates an example of a cross-sectional view of a lacrimal implant taken along a line parallel to a longitudinal axis of the lacrimal implant, such as along line 2B-2B of FIG. 2A.
FIG. 2C illustrates an example of a cross-sectional view of another lacrimal implant taken along a line parallel to a longitudinal axis of the implant.
FIG. 3A illustrates an example of an isometric view of a lacrimal implant configured to be retained at least partially within a lacrimal punctum and canalicular anatomy.
FIG. 3B illustrates an example of a cross-sectional view of a lacrimal implant taken along a line parallel to a longitudinal axis of the implant, such as along line 3B-3B of FIG. 3A, and a dilation of an implant-receiving anatomical tissue structure.

FIG. 4A illustrates an example of an isometric view of a lacrimal implant configured to be retained at least partially within a lacrimal punctum and canalicular anatomy.

FIG. 4B illustrates an example of a cross-sectional view of a lacrimal implant taken along a line parallel to a longitudinal axis of the implant, such as along line 4B-4B of FIG. 4A.

FIG. 5 illustrates an example of a cross-sectional view of a lacrimal implant configured to be retained at least partially within a lacrimal punctum and canalicular anatomy.

FIG. 6 illustrates an example plot of an anti-glaucoma agent content per 0.95 mm cross-section of an agent-filled precursor sheath prepared by cold extrusion.

FIG. 7 illustrates an example method of manufacturing a drug corecomprising about 44 micrograms of an anti-glaucoma agent.

FIGS. 8A-8C illustrate examples of release profile curves, under *in vitro* dissolution conditions for a time period of about 63 days, of lacrimal implants including 44 micrograms of an anti-glaucoma agent.

FIG. 9 illustrates a release profile from an ocular implant of the invention incorporating excipients glycerol, PEG400, EPG, DMPC and DMPE.

FIG. 10 illustrates a release profile from an ocular implant of the invention incorporating excipients DMPC and EPG, relative to a release rate from an implant containing no excipient.

FIG. 11 illustrates a release profile from an ocular implant of the invention incorporating excipients DPPE and long chained PC (PSPC, DPPC, DSPC), both of which exhibit a decreased elution rate, and from ocular implants of the invention incorporating short chained PC (DMPC) and PGs (EPG, POPG, DMPG), which exhibit an increased elution rate.

FIG. 12 illustrates a release profile from an ocular implant of the invention incorporating excipients DOTAP, DODAP, and C16 positively charged lipids.

FIG. 13 illustrates section of filled and cured polyimide sheath containing a composition of the invention comprising therapeutic agent latanoprost in a silicone matrix in the presence of the excipient, either DMPC or EPG. The matrix is macroscopically homogenous with no large inclusions of separated latanoprost.

FIG. 14 illustrates a section of a sheath filled with a control composition identical to the composition of FIG. 13 except lacking the excipient. The extrusions exhibits clear separation between the polymer matrix and latanoprost within the polyimide sheath. The also show the variation that can be observed. The top one shows smaller inclusions of latanoprost. The bottom shows large clear sections of latanoprost.

DETAILED DESCRIPTION

The unit inch employed in this description can be converted as followed: 1 inch = 2,54 cm.

Definitions:

As used herein, the terms "a" or "an" are used, as is common in patent documents, to include one or more than one, independent of any other instances or usages of "at least one" or "one or more."

As used herein, the term "or" is used to refer to a nonexclusive or, such that "A or B" includes "A but not B," "B but not A," and "A and B," unless otherwise indicated.

As used herein, the term "about" is used to refer to an amount that is approximately, nearly, almost, or in the vicinity of being equal to a stated amount. As used herein, the term "adverse event" refers to any undesirable clinical event experienced by a subject undergoing a therapeutic treatment including a drug and/or a medical device, whether in a clinical trial or a clinical practice. Adverse events include a change in the subject's condition or laboratory results, which has or could have a deleterious effect on the subject's health or well-being. For example, adverse events include but are not limited to: device malfunction identified prior to placement, device malposition, device malfunction after placement, persistent inflammation, endophthalmitis, corneal complications (corneal edema, opacification, or graft decompensation), chronic pain, iris pigmentation changes, conjunctival hyperemia, eyelash growth (increased length, thickness, pigmentation, and number of lashes), eyelid skin darkening, intraocular inflammation (iritis/uveitis), macular edema including cystoid macular edema, blurred vision, burning and stinging, foreign body sensation, itching, punctate epithelial keratopathy, dry eye, excessive tearing, eye pain, lid crusting, lid discomfort/pain, lid edema, lid erythema, photophobia, VA decrease, conjunctivitis, diplopia, discharge from the eye, retinal artery embolus, retinal detachment, vitreous hemorrhage from diabetic retinopathy, upper respiratory tract infection/cold/flu, chest pain/angina pectoris, muscle/joint/back pain, and rash/allergic skin reaction, eye pruritus, increase in lacrimation, ocular hyperemia and punctate keratitis.

As used herein, the phrase "consisting essentially of limits a composition to the specified materials or steps and those additional, undefined components that do not materially affect the basic and novel characteristic(s) of the composition.

As used herein, the term "continuous" or "continuously" means unbroken or uninterrupted. For example, continuously administered active agents are administered over a period of time without interruption.

As used herein, the term "eye" refers to any and all anatomical tissues and structures associated with an eye. The eye is a spherical structure with a wall having three layers: the outer sclera, the middle choroid layer and the inner retina. The sclera includes a tough fibrous coating that protects the inner layers. It is mostly white except for the transparent area at the front, the cornea, which allows light to enter the eye. The choroid layer, situated inside the sclera, contains many blood vessels and is modified at the front of the eye as the pigmented iris. The biconvex lens is situated just behind the pupil. The chamber behind the lens is filled with vitreous humour, a gelatinous substance. The anterior and posterior chambers are situated between the cornea and iris, respectively and filled with aqueous humour. At the back of the eye is the light-detecting retina. The cornea is an optically transparent tissue that conveys images to the back of the eye. It includes avascular tissue to which nutrients and oxygen are supplied via bathing with lacrimal fluid and aqueous humour as well as from blood vessels that line the junction between the cornea and sclera. The cornea includes one pathway for the permeation of drugs into the eye. Other anatomical tissue structures associated with the eye include the lacrimal drainage system, which includes a secretory system, a distributive system and an excretory system. The secretory system comprises secretors that are stimulated by blinking and temperature change due to tear evaporation and reflex secretors that have an efferent

parasympathetic nerve supply and secrete tears in response to physical or emotional stimulation. The distributive system includes the eyelids and the tear meniscus around the lid edges of an open eye, which spread tears over the ocular surface by blinking, thus reducing dry areas from developing.

As used herein, the term "implant" or "lacrimal implant" refers to a structure that can be configured to contain or be impregnated with a drug core or a drug matrix, such as those as disclosed in this patent document and in WO 07/115,261. The lacrimal implant is capable of releasing a quantity of a therapeutic active agent, such as latanoprost or other anti-glaucoma agent, into tear fluid for a sustained release period of time when the structure is implanted or inserted at a target location along the path of the tear fluid in the subject. The terms "implant," "plug," and "lacrimal implant" are meant herein to refer to similar structures. Likewise, the terms "implant body" and "plug body" are meant herein to refer to similar structures. The lacrimal implants described herein may be inserted through a punctum of a subject and into an associated canaliculus. The lacrimal implant may be also the drug core or drug matrix itself, which is configured for insertion through a punctum without being housed in a carrier such as a punctal plug occluder, for example, having a polymeric component and an anti-glaucoma agent component, for example, latanoprost, with no additional structure surrounding the polymeric component and agent component. In some embodiments, the lacrimal implant further includes one or more penetration enhancers, for example, benzalkonium chloride.

As used herein, "loss of efficacy" (LoE) is defined as an IOP increase to baseline (post-washout) IOP in either or both eyes while wearing an L-PPDS continuously from Day 0. Subjects were followed for at least 4 weeks before the subject could complete the study due to LoE and LoE was confirmed at 2 sequential visits.

As used herein, a "pharmaceutically acceptable vehicle" is any physiological vehicle known to those of ordinary skill in the art useful in formulating pharmaceutical compositions. Suitable vehicles include polymeric matrices, sterile distilled or purified water, isotonic solutions such as isotonic sodium chloride or boric acid solutions, phosphate buffered saline (PBS), propylene glycol and butylene glycol. Other suitable vehicular constituents include phenylmercuric nitrate, sodium sulfate, sodium sulfite, sodium phosphate and monosodium phosphate. Additional examples of other suitable vehicle ingredients include alcohols, fats and oils, polymers, surfactants, fatty acids, silicone oils, humectants, moisturizers, viscosity modifiers, emulsifiers and stabilizers. The compositions may also contain auxiliary substances, i.e. antimicrobial agents such as chlorobutanol, parabans or organic mercurial compounds; pH adjusting agents such as sodium hydroxide, hydrochloric acid or sulfuric acid; and viscosity increasing agents such as methylcellulose. The final composition should be sterile, essentially free of foreign particles, and have a pH that allows for optimum drug stability.

As used herein, the term "punctum" refers to the orifice at the terminus of the lacrimal canaliculus, seen on the margins of the eyelids at the lateral extremity of the lacus lacrimalis. Puncta (plural of punctum) function to reabsorb tears produced by the lacrimal glands. The excretory part of the lacrimal drainage system includes, in flow order of drainage, the lacrimal puncta, the lacrimal canaliculi, the lacrimal sac and the lacrimal duct. From the lacrimal duct, tears and other flowable materials drain into a passage of the nasal system. The lacrimal canaliculi include an upper (superior) lacrimal canaliculus and a lower (inferior) lacrimal canaliculus, which respectively terminate in an upper and lower lacrimal punctum. The upper and lower punctum are slightly elevated at the medial end of a lid margin at the junction of the ciliary and lacrimal portions near a conjunctival sac. The upper and lower punctum are generally round or slightly ovoid openings surrounded by a connective ring of tissue. Each of the puncta leads into a vertical portion of their respective canaliculus before turning more horizontal at a canaliculus curvature to join one another at the entrance of the lacrimal sac. The canaliculi are generally tubular in shape and lined by stratified squamous epithelium surrounded by elastic tissue, which permits them to be dilated.

The term "subject" refers to animals such as mammals, including, but not limited to, primates (e.g., human beings), cows, sheep, goats, horses, dogs, cats, rabbits, rats, mice and the like. In many embodiments, the subject is

a human being.

An "anti-glaucoma agent" can comprise a drug and may be any of the following or their equivalents, derivatives or analogs, adrenergic agonists, adrenergic antagonists (beta blockers), carbonic anhydrase inhibitors (CAIs, systemic and topical), parasympathomimetics, prostaglandins and hypotensive lipids, and combinations thereof). Other agents or drugs for use with the present invention include antimicrobial agents (e.g., antibiotic, antiviral, antiparacytic, antifungal, etc.), a corticosteroid or other anti-inflammatory (e.g., an NSAID), a decongestant (e.g., vasoconstrictor), an agent that prevents of modifies an allergic response (e.g., an antihistamine, cytokine inhibitor, leucotriene inhibitor, IgE inhibitor, immunomodulator), a mast cell stabilizer, cycloplegic or the like. Examples of conditions that may be treated with agents include but are not limited to glaucoma, pre and post surgical treatments, ocular hypertension, dry eye and allergies.

As used herein, a "penetration enhancer" refers to an agent or other substance that transiently increases a subject's ocular permeability characteristics (e.g., permeability of an eye's cornea). Some characteristics of the ocular penetration enhancer may include one or more of: immediate and unidirectional effect; predictable duration of effect; after removal, the ocular tissues recover their normal barrier property; little to no systemic or toxic effects; little to no irritation or damage to an ocular membrane surface; or physical compatibility with a wide range of anti-glaucoma agents and pharmaceutical excipinets. Exemplary ocular penetration enhancers include, but are not limited to, calcium chelators (e.g., EDTA), surfactants (e.g., nonionic surfactants, including Polyoxyethylene-9-lauryl ether, Tween 80, or Span 60; or Bile acids and salts, including Na-deoxycholate, Na-taurocholate, or Na-taurodeoxycholate), preservatives (e.g., Benzalkonium chloride or Cetylpyridinium chloride), glycosides (e.g., Digitonin or Saponin), fatty acids (e.g., Capric acid, Oleic acid, or Short fatty acid), azones, chitosans, tamarind seed polysaccharides, polycarhophils or derivatives thereof (e.g.,

Polycarbophil or Polycarbophil-cysteine conjugate), cytochalasins, or cyclodextrins. Other penetration enhancers for use in the present invention include, but are not limited to, those as described in the following reference: Touitou, Elka, Barry, Brian W. Enhancement in Drug Delivery, Taylor & Francis Group; 2007: 527-548.

The term "topical" refers to any surface of a body tissue or organ. A topical formulation is one that is applied to a body surface, such as an eye, to treat that surface or organ. Topical formulations include liquid drops such as eye drops; creams, lotions, sprays, emulsions, and gels. Topical formulations as used herein also include formulations that release agents, inlcuding for example anti-glaucoma agents, into the tears to result in topical administration to the eye.

As used herein, the term "treating" or "treatment" of a disease includes: (1) preventing the disease, i.e., causing the clinical symptoms of the disease not to develop in a subject that may be exposed to or predisposed to the disease but who does not yet experience or display symptoms of the disease; (2) inhibiting the disease, i.e., arresting or reducing the development of the disease or its clinical symptoms; or (3) relieving the disease, i.e., causing regression of the disease or its clinical symptoms.

As used herein, an "effective amount" in the context of a therapeutic agent, or a "therapeutically effective amount" of a therapeutic agent refers to an amount of the agent that alleviates, in whole or in part, symptoms associated with the disorder or condition, or halts or slows further progression or worsening of those symptoms, or prevents or provides prophylaxis for the disorder or condition. In particular, an "effective amount" refers to an amount effective, at dosages and for periods of time necessary, to achieve the desired therapeutic result. A therapeutically effective amount is also one in which any toxic or detrimental effects of compounds of the invention are outweighed by the therapeutically beneficial effects. When the term "effective amount" is used in the context of a functional material, such as an effective amount of a dispersant, what is meant is that the amount of the functional material used is effective to achieve the desired result.

A "matrix" is a material comprising a polymer in which the therapeutic agent is dispersed, the combination of which materials, along with an excipient of the invention, makes up the drug core of an implant that serves as the reservoir of the agent from which the agent is released into the surroundings of the implant over a period of time.

A "polymer" as the term is used herein, refers to a macromolecule containing one or more repeating units, as is well known in the art. A "copolymer" refers to a polymer in which there are at least two types of repeating units included. A copolymer can be a block copolymer, in which there are segments containing multiple repeating units of one type, bonded to segments containing multiple repeating units of a second type. A "polymer" or "polymeric material" can be a silicone, a polyurethane, a polyamide, a polyester, a polysaccharide, a polyimide, or the like, or any copolymer thereof. When a polymeric material is to come in contact with a body tissue or fluid, the polymeric material is biocompatible.

A "loading" of a therapeutic agent within the matrix of an implant within the meaning herein refers to the concentration of the agent relative to the matrix within the implant, exclusive of the weight of any sheath, if present.

An "excipient", as the term is used herein, refers to a substance disposed within the matrix in addition to the therapeutic agent that itself does not exert the biological effect of the therapeutic agent. For example, if the therapeutic agent is a prostanoid having a physiological effect on body tissues surrounding the implant, a second prostanoid having the physiological effect would not be an excipient within the meaning herein. Excipients can include agents that modify, such as increase, the rate of the release from the implant into the body tissues. An excipient can include substances that can alter, such as increase, the time of residency of the therapeutic agent in the target tissues surrounding the

location of the implant in a living body. An excipient can also include substances that can alter the properties of uptake of the therapeutic agent into the surrounding tissues, such as by increasing penetration of the cornea by the agent in the presence of the excipient relative to penetration in the absence of the excipient (penetration enhancers). An excipient can also include substances that can alter the physical properties of the implant itself, for example by altering, such as increasing, an amount of therapeutic agent that can be present in the implant while preserving the desirable homogeneity of dispersion of inclusions of the agent within the matrix, relative to an amount of the agent that can be present in the desirable substantially dispersed manner in the matrix without the presence of the excipient. Some examples of excipients within the meaning herein include, but are not limited to lipids such as phospholipids, polyhydric alcohols such as glycerol, and polyethylene glycols of various molecular weights, and benzalkonium chloride.

The term "wherein an amount of the therapeutic agent in a volumetric portion of the matrix is similar to an amount of the therapeutic agent in any other equal volumetric portion of the matrix" refers to a uniformity of distribution of the agent throughout the matrix, whether the agent is homogeneously dissolved or whether it forms separate inclusion bodies, solid or liquid, throughout the matrix polymer, or both. In various embodiments, the therapeutic agent is contained in the matrix such that an amount of the therapeutic agent in a volumetric portion of the drug core is similar to an amount of the therapeutic agent in any other equal volumetric portion of the drug core. For example, the amount of the therapeutic agent in a volumetric portion of the drug core can vary from the amount of the therapeutic agent in any other equal volumetric portion of the drug core by no greater than about 30%. For example, the amount of the therapeutic agent in a volumetric portion of the drug core can vary from the amount of the therapeutic agent in any other equal volumetric portion of the drug core by no greater than about 20%. For example, the amount of the therapeutic agent in a volumetric portion of the drug core can vary from the amount of the therapeutic agent in any other equal volumetric portion of the drug core by no greater than about 10%. For example, the amount of the therapeutic agent in a volumetric portion of the drug core can vary from the amount of the therapeutic agent in any other equal volumetric portion of the drug core by no greater than about 5%. In addition, the concentration of the therapeutic agent in a volumetric portion of the drug core can be the same as any other equal volumetric portion of the drug core, in certain embodiments including those embodiments wherein the agent is present as a uniform, homogeneous dispersion and in embodiments wherein the agent is present in solid or liquid inclusions throughout the matrix.

The agent can be dissolved in the matrix in some embodiments, when the chemical identities of the agent and the matrix, and the concentration of the agent in the matrix, are such that dissolution is achieved. For example, as is known in the art, certain lipophilic steroid derivatives can dissolve at significant concentrations in silicones. In this event, the agent is referred to as being "dissolved" in the polymer, or as being uniformly, homogeneously dispersed throughout the matrix or "dispersed at a molecular level" in the polymer, just as a compound can be dissolved in a solvent, to form a "solid solution" of the agent in the polymeric material of the matrix.

In other embodiments, the agent does not completely dissolve in the matrix, but is present as domains or "inclusions" of the agent within the polymeric matrix. The inclusions can be liquid or solid at about room temperature or at about the temperature of the human body. After the matrix precursor has been cured to form the matrix, the inclusions are non-uniformly distributed in the now-solid or near solid matrix, and are thus prevented at least to some extent from recombining with each other, such as by liquid droplet accretion. This form is referred to as a "heterogeneous" distribution of the agent in the matrix. When inclusions of the agent are present, it is believed that a certain proportion of the agent may also be dissolved in the matrix. However, dissolution is not necessary for operation and function of the invention. Furthermore, the heterogeneous distribution of the agent with the matrix can be managed on a macroscopic level as discussed in connection with the definition of the terms "concentration" and "similar" given below.

A "concentration" of a therapeutic agent, as the term is used herein, refers to a concentration of the agent within a macroscopic volume of the matrix-agent core, that is controlled to have a degree of reproducibility from sample to sample of the core. A concentration of the agent in a macroscopic volume of the core can vary, but only within limits, relative to that in any other equal macroscopic volume of the core. The term does not relate to concentrations at the molecular level, where discontinuous and/or irregular domains or inclusions of the agent in concentrated form may be present, but rather refers to bulk concentrations of the agent in volumes of the core that are greater than at least about 0.1 $mm^3$, for example, a cubic sample of core about 100 micrometers or microns ($\mu m$) on a side, or a 0.1 mm thick slice of a core with cross-sectional area of about 1 mm2.

The term "similar", as in a "similar" concentration of a therapeutic agent, means that within a defined margin, the quantity, such as the concentration of the agent, for example in units of micrograms ($\mu g$)/$mm^3$, only varies within a certain degree from measurement to measurement. The degree of variation is controlled or regulated to provide a degree of uniformity of core material, such that pluralities of cores or inserts are medically suitable in that the dose of the agent they can provide to the tissue is within certain limits from sample to sample. For example, a "similar" concentration between two equal volumes of core material, or between two inserts prepared by from a filled precursor sheath, can vary by no greater than about 30%, or can vary by no greater than about 20%, or can vary by no greater than about 10%, or can vary by no greater than about 5%. The term "similar" also includes solid solutions and uniform homogeneous dispersions, defined herein.

In various embodiments, the core contains the therapeutic agent dispersed in the matrix in the form of solid or liquid inclusion bodies, referred to herein as "inclusions." The inclusions can be of various sizes, and various distributions of sizes of a plurality of the inclusions are possible, as are defined herein. When it is stated that the inclusions are no greater than about 100 μm in diameter, what is meant is that the largest inclusion observed within a drug insert of the invention has a greatest dimension of no greater than about 100 μm. When a particular size distribution of inclusions is recited, what is meant is that a predominant proportion of all the inclusions are of the stated dimension. When an average size or "average diameter" of inclusions within a population of inclusions is stated, such as an "average diameter of about 50 μm," what is meant is a numerical average of the greatest dimensions of all the inclusions. An "average diameter of less than about 50 μm" means this average is less than about the stated value. When a "standard deviation" of the distribution of inclusion diameters with in a population of inclusions is stated, what is meant that the distribution of inclusion diameters is normal or near normal, and that the standard deviation is a measure of the spread of the values, as is well known in the art. A small standard deviation relative to the average diameter denotes a tight distribution of inclusion diameters, a feature of various embodiments of the present invention. A relative uniformity of inclusion size distribution, and a relative uniformity of the amount of agent dispersed per unit volume of the core within the insert, are features of various embodiments according to the present invention.

The size distribution of inclusion diameters can be monodisperse, and can be tightly so. By "monodisperse" is meant herein that the size distribution of the diameters of the plurality of inclusions is relatively tightly clustered around the average inclusion diameter, even if the distribution is not a normal distribution. For example, the distribution can have a fairly sharp upper size limit of inclusions of greater than average diameter, but can trail off in the distribution of inclusions of less than average diameter. Nevertheless, the size distribution can be tightly clustered, or monodisperse.

A "silicone" refers to a polymer including the elements silicon, oxygen, carbon and hydrogen, as is well known in the art. The carbon and hydrogen together form organic groups with which the silicon-oxygen backbone of the polymer can be substituted, and which can intersperse domains of silicon-oxygen backbone, or both. The organic groups can be alkyl groups such as methyl, aryl groups such as phenyl, or any combination thereof.

A "crosslinker" is a reagent that, in formation of the polymer making up the matrix, increased the degree of linking between individual polymer chains. For example, a crosslinker for a silicone polymer can be a tetraalkylorthosilicate, such as tetraethylorthosilicate.

A "polyurethane" refers to a variety of polymer or copolymer containing repeating units bonded covalently through urethane, i.e., carbamate, bonds, -N-C(O)-O- wherein the N and O atoms are attached to an organic radical. The organic radical can be aliphatic, aromatic, or mixed; can contain other functional groups. Each radical, other than the radicals at the ends of the molecular chains, is bonded via two (or more) urethane groups to other radicals. A polyurethane polymer contains only urethane-type groups joining the repeating units. A polyurethane copolymer, such as a polyurethane-silicone copolymer or a polyurethane-carbonate copolymer, contains urethane and other types of groups joining the repeating units, i.e., silicone and carbonate type groups respectively.

A "release profile" or "release rate profile" refers to a rate of release, an amount of the agent as a function of time that moves from an inventive implant into body tissue or fluid, for example the eye or tear fluid. The release profile can in turn govern the concentration of the agent in the eye and surrounding tissue over the time period during which the plug releases the agent. An excipient of the invention can alter this release profile, such as my increasing a rate of release of the agent in the presence of the excipient relative to a rate of release observed from a comparable implant in the absence of the excipient, or in other cases by reducing that rate of release.

Elevated Intraocular Pressure:

Ocular hypertension (OH) and primary open angle glaucoma (POAG) are caused by a build-up of aqueous humor in the anterior chamber primarily due to the eye's inability to properly drain aqueous fluid or an overproduction of aqueous humor. The ciliary body, situated at the root of the iris, continuously produces aqueous humor. It flows into the anterior chamber and then drains via the angle between the cornea and iris through the trabecular meshwork and into a channel in the sclera. In the normal eye, the amount of aqueous humor being produced is equal to the amount that is draining out. However, in an eye in which this mechanism is compromised, intraocular pressure (IOP) rises. Elevated IOP represents a major risk factor for glaucomatous field loss. Results from several studies indicate that early intervention targeted at lowering intraocular pressure retards the progression of optic nerve damage and loss of visual fields that lead to decreased vision and blindness.

Latanoprost:

One anti-glaucoma agent for use in the methods described herein is latanoprost. Latanoprost is a prostaglandin F2α analogue. Its chemical name is isopropyl-(Z)-7 [(1R,2R,3R,5S)3,5-dihydroxy-2-[(3R)-3-hydroxy-5-phenylpentyl]cyclopentyl]-5-heptenoate. Its molecular formula is $C_{26}H_{40}O_5$ and its chemical structure is:

M.W. 432.58 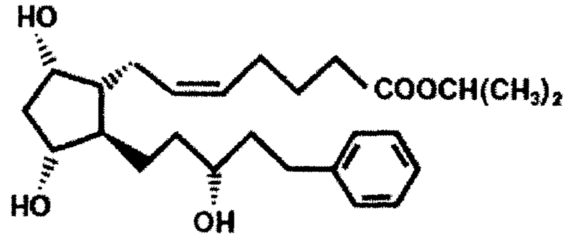

Latanoprost is a colorless to slightly yellow oil that is very soluble in acetonitrile and freely soluble in acetone, ethanol, ethyl acetate, isopropanol, methanol and octanol. It is practically insoluble in water.

Latanoprost is believed to reduce intraocular pressure (IOP) by increasing the outflow of aqueous humor. Studies in animals and man suggest that the main mechanism of action is increased uveoscleral outflow of aqueous fluid from the eyes. Latanoprost is absorbed through the cornea where the isopropyl ester prodrug is hydrolyzed to the acid form to become biologically active. Studies in man indicate that the peak concentration in the aqueous humor is reached about two hours after topical administration.

Xalatan® latanoprost ophthalmic solution is a commercially available product indicated for the reduction of elevated IOP in subjects with open-angle glaucoma or ocular hypertension. The amount of latanoprost in the commercially available product Xalatan® is approximately 1.5 micrograms/drop. As described above, eye drops, though effective, can be inefficient and require multiple applications or treatment regimens to maintain the therapeutic benefit. Low subject compliance compounds these effects.

FIG. 1 illustrates averaged, *in vitro* release profile curves, under accelerated dissolution conditions for a time period of about 50 hours, of three example lacrimal implants each having a drug core including 44 micrograms of latanoprost and three example lacrimal implants each having a drug core including 21 micrograms of latanoprost. The accelerated dissolution conditions were supplied, in part, using 50% isopropyl alcohol. The profile curves illustrate that lacrimal implants having a drug core including 44 micrograms of latanoprost elute the anti-glaucoma agent faster than lacrimal implants having a drug core including 21 micrograms of latanoprost.

The release profile curves of FIG. 1 were obtained using high performance liquid chromatography (HPLC) methods. The latanoprost containing lacrimal implants were placed in a vial containing 1 mL of 1:1 mixture of isopropanol alcohol and phosphate buffered saline pH 7.4 solution, which was placed inside a shaking water bath at 60°C and 100 cycles per minute. Subsequently, 50 microliter samples of the solution were taken at 2, 8, 24 and 48 hours and injected into a reversed phase HPLC system using UV detection at 210 nm to measure the amount of latanoprost released from the lacrimal implant.

FIG. 8A-8C illustrate *in vitro* release profile curves, under *in vitro* dissolution conditions for a time period of about 63 days, of lacrimal implants having a drug core including 44 micrograms of latanoprost. More specifically, FIG. 8A illustrates averaged elution data (in ng) of three example lacrimal implants each having a drug core including 44 micrograms of latanoprost. The release profile curves of FIGS. 8A-8C were obtained using high performance liquid chromatography (HPLC) methods. The latanoprost containing lacrimal implants were placed in a vial containing 1 mL phosphate buffered saline pH 7.4 solution (PBS), which was placed inside a stainless steel sinker in a shaking water bath at 37°C and 100 cycles per minute. At 24 hour sampling intervals (and multiples thereof), the latanoprost containing lacrimal implant was removed and placed into another vial containing 1 mL of PBS and placed into a shaking water bath set at 37°C and 100 cycles per minute. Subsequently, liquid samples were collected and spiked with an internal standard, and injected into a reversed phase HPLC system using UV detection at 210 nm to measure the amount of latanoprost released from the lacrimal implant.

Initially, the lacrimal implants are shown to have a burst elution of latanoprost exceeding 1500 ng; however, this initial burst elution transitions between days 2 through 20 to a relatively constant elution of about 350-450 ng at or near day 21. FIGS. 8B-8C illustrate that the increased amount (in percentage and ng, respectively) of latanoprost eluted from the drug core over time, especially after about 5 days, is substantially linear. Advantageously, these linear trends can be beneficial in determining or predicting the effective release duration of a given lacrimal implant (e.g., punctum plug).

The lacrimal implants used to generate the profile curves shown in FIGS. 8A-8C each include a drug core having a diameter of about 0.0165 inches. As noted below, the level of release of latanoprost or other anti-glaucoma agent can be changed by altering an exposed surface area (e.g., a diametric surface area) of the drug core.

Effecting Latanoprost or other Anti-Glaucoma Agent Release Rates:

Work in relation to embodiments of the present invention indicates that molecular weight and solubility in water can each effect the release rate of the anti-glaucoma agent, such as latanoprost or other prostaglandin, from a solid drug core matrix. For example, lower molecular weight may increase diffusion through a solid matrix material, e.g.,

through silicone, such that low molecular weight compounds may be released more quickly. Also, solubility in water can also effect the release rate of the anti-glaucoma agent, and in some instances increased water solubility of the agent may increase the rate of release from the solid drug core matrix, for example via transport from the solid matrix material to the bodily liquid, such as tear liquid. In accordance with these embodiments, anti-glaucoma agents with higher molecular weight than fluorescein and with lower water solubility than fluorescein, for example cyclosporin and prostaglandins, may be released from the solid core at lower rates. Surfactants may also effect the rate of release of the anti-glaucoma agent from the drug core into the surrounding bodily tissue and/or fluid, for example tear film fluid.

Work in relation to embodiments of the present invention suggests that radicals generated in the sterilization process may crosslink the drug core matrix material so as to inhibit the initial release rate of anti-glaucoma agent from the drug core matrix material. In specific embodiments with e-beam sterilization, this crosslinking may be limited to the surface and/or near the surface of the drug core matrix. In some embodiments, a known Mylar bag can be penetrated with the e-beam to sterilize the surface of the drug core. In some embodiments, other sterilization techniques that effect sterilization can be used, for example gamma ray sterilization, and that are not limited to the surface of the drug core and fully and/or uniformly penetrate the drug core material.

Work in relation to embodiments of the present invention suggests that known salts, for example sodium chloride can effect the rate of elution from the drug core.

Work in relation with the present invention indicates that naturally occurring surfactants in the tear film, for example surfactant D and phospholipids, may effect transport of the anti-glaucoma agent dissolved in the solid matrix from the core to the tear film. The drug core can be adapted in response to the surfactant in the tear film to provide sustained delivery of the anti-glaucoma agent into the tear film at therapeutic levels. For example, empirical data can be generated from a patient population, for example 10 patients whose tears are collected and analyzed for surfactant content. Elution profiles in the collected tears for a drug that is sparingly soluble in water, for example cyclosporine, can also be measured and compared with elution profiles in buffer and surfactant such that an *in vitro* model of tear surfactant is developed. An *in vitro* solution with surfactant based on this empirical data can be used to adjust the drug core in response to the surfactant of the tear film.

In some embodiments, the silicone or other solid drug core material may comprise an inert filler to add rigidity to the cured matrix. Work in relation to embodiments of the present invention suggests that the filler material may increase the rate of release of the therapeutic agent. The MED-4011 and MED-6385 materials are commercially available with the filler material. The MED-4011 material may comprise an inert silica filler material to add rigidity to the cured silicone matrix. The MED-4385 may comprise inert diatomaceous earth filler material to add rigidity to the cured silicone matrix.

Work in relation to embodiments of the present invention suggests that the level of release of anti-glaucoma agent can be decreased with a decreased exposed surface area of the drug core, thereby allowing for selective release of the agent at one or more therapeutic levels for sustained periods.

<u>Adverse Events in Clinical Trials and Clinical Practice:</u>

Based on Xalatan® product information, the most frequently reported ocular adverse events associated with latanoprost in clinical trials were blurred vision, burning and stinging, conjunctival hyperemia, foreign body sensation, itching, increased pigmentation of the iris, and punctate keratopathy. These events occurred in 5% to 15% of subjects. Less than 1% of subjects required discontinuation of therapy because of intolerance to conjunctival hyperemia. Dry eye, excessive tearing, eye pain, lid crusting, lid discomfort/pain, lid edema, lid erythema, and photophobia were reported in 1% to 4% of subjects. Conjunctivitis, diplopia, and discharge from the eye were reported in <1% of subjects. Retinal artery embolus, retinal detachment, and vitreous hemorrhage from diabetic retinopathy were rarely reported.

The most common systemic adverse events in clinical trials were upper respiratory tract infection/cold/flu, which occurred at a rate of approximately 4%. Chest pain/angina pectoris, muscle/joint/back pain, and rash/allergic skin reaction each occurred at a rate of 1% to 2%.

In clinical practice, the following adverse events associated with latanoprost have been noted: asthma and exacerbation of asthma; corneal edema and erosions; dyspnea; eyelash and vellus hair changes (increased length, thickness, pigmentation, and number); eyelid skin darkening; herpes keratitis; intraocular inflammation (iritis/uveitis); keratitis; macular edema, including cystoid macular edema; misdirected eyelashes sometimes resulting in eye irritation; dizziness, headache, and toxic epidermal necrolysis.

<u>Subject Noncompliance:</u>

Numerous studies have been published showing high noncompliance by subjects using eye drops for treatment of various ocular disorders. One study showed only 64% of subjects used the eye drops as directed (Winfield et al., 1990). Another study showed that 41% of subjects using eye drops for glaucoma missed six or more doses over a 30-day period (Norell and Granstrom 1980).

The disclosure described herein provides methods to treat diseases and disorders, for example, glaucoma, that avoid at least some of the problem of noncompliance associated with eye drop administration. In some embodiments, the methods of the disclosure reduce subject noncompliance significantly compared to eye drop administration, by at least 10%, at least 20%, at least 30%, at least 40%, at least 50%, at least 60%, at least 70%, at least 80%, or at least 90%. In some embodiments, overall subject noncompliance with the methods described herein is about 5%, about 10%, about 15%, about 20%, or about 25%. Subject noncompliance may occur if a lacrimal implant delivery system of the invention is intentionally removed by a subject or if the subject does not seek reinsertion of the implant delivery system after such implant has been unintentionally lost from a punctum of the subject.

Benzalkonium Chloride:

Benzalkonium chloride, also known as B AK, alkyldimethylbenzylammonium chloride and ADBAC, is a mixture of alkylbenzyldimethylammonium chlorides of various even-numbered alkyl chain lengths. Benzalkonium chloride is a nitrogenous cationic surface-acting agent belonging to the quaternary ammonium group. It has three main categories of use; as a biocide, a cationic surfactant and phase transfer agent in the chemical industry.

Benzalkonium chloride is readily soluble in ethanol and acetone. Although dissolution in water is slow, aqueous solutions are easier to handle for example. Solutions are neutral to slightly alkaline, with color ranging from clear to a pale yellow. Solutions foam profusely when shaken, have a bitter taste and a faint almond-like odor which is only detectable in concentrated solutions.

Benzalkonium chloride solutions are rapidly acting biocidal agents with a moderately long duration of action. They are active against bacteria and some viruses, fungi, and protozoa. Bacterial spores are considered to be resistant. Solutions are bacteriostatic or bactericidal according to their concentration. Gram-positive bacteria are generally more susceptible than Gram-negative bacteria. Activity is not greatly affected by pH, but increases substantially at higher temperatures and prolonged exposure times. HypoTears® is a commercially available artificial tear product containing benzalkonium chloride. The product safety information states that short-lived stinging and/or burning and local irritation may occur.

It has been reported that 59% of glaucoma subjects have signs and symptoms of ocular surface disease, and that the chance of having such signs or symptoms is increased 2-fold with the use of each additional BAK-containing eye drop (Leung et al. 2008). Through in vitro studies, it has been found that BAK enhances corneal penetration. Beyond BAK, it is believed that one or more penetration enhancers, such as at least one of a calcium chelator (e.g., EDTA), a surfactant (e.g., nonionic surfactants, including Polyoxyethylene-9-lauryl ether, Tween 80, or Span 60; or Bile acids and salts, including Na-deoxycholate, Na-taurocholate, or Na-taurodeoxycholate), a preservative (e.g., Cetylpyridinium chloride), a glycoside (e.g., Digitonin or Saponin), a fatty acid (e.g., Capric acid, Oleic acid, or Short fatty acid), an azone, a chitosan, a tamarind seed polysaccharide, a polycarhophil or derivative thereof (e.g., Polycarbophil or Polycarbophil-cysteine conjugate), a cytochalasin, or a cyclodextrin, can promote the successful delivery of one or more anti-glaucoma agents through an eye's complex series of defense mechanisms, which make it difficult to achieve an effective agent concentration within a target area of the eye.

Artificial Tears:

In humans, under normal conditions, it had been reported that the tear volume in the conjunctival cul-de-sac is approximately 7 to 9 microlitres, with a turnover rate of 0.5 to 2.2 microlitres/min. The maximum volume that the conjunctival cul-de-sac can contain is estimated to be about 30 microlitres. Commercial eyedrops typically deliver between 25.1 and 56.4 microlitres; with an average drop volume of 35 microlitres. This bolus of fluid can serve as a vehicle for the active ingredient in topical medications and provides that the eye drop fluid covers the ocular surface and has adequate access to the cornea. In some embodiments of the present invention, for example in those with subjects with suboptimal basal tear volume, excess fluid from one or more drops of artificial tears used in conjunction with the lacrimal implants of the present invention may serve as a means to augment a subject's basal tear in order to assist in dispersion and adequate access for the anti-glaucoma medication to reach the cornea from the lacrimal implants. In some embodiments of the present invention, the artificial tears for use with the present invention include eye lubricant formulations. In some embodiments, the eye lubricants include gels, liquidgels, ointments, emollients, sprays and eyedrops. In some embodiments, the artificial tears for use with the present invention include a penetration enhancer. In other embodiments, artificial tears for use with the present invention do not include a penetration enhancer. Examples of artificial tears for use with the present invention, may include, but are not limited to lubricating formulations such as Hypotears™, Refresh™ Tears, Visine™ Tears, Bion™ Tears, Advanced Eye Relief™, Clarymist™, Oasis™ Tears, Soothe™, Similasan™, Genteal™ Gel, Refresh™ Liquigel, Systane™ Lubricant Eyedrops, Systane™ Free liquid gel, Lacri-Lube™, Refresh PM™, Tears Naturale™, Tears Again™, Dwelle™, Lacrisert™, and the like.

Methods of treatment not forming part of the invention:

Described herein are methods to treat glaucoma, elevated intraocular pressure, or glaucoma-associated elevated intraocular pressure with an anti-glaucoma agent. In many embodiments, a method of treating an eye with an anti-glaucoma agent, for example, latanoprost, is provided. In some embodiments, the anti-glaucoma agent is released to the eye over a sustained period of time. In an embodiment, the sustained period of time is approximately 90 days. In some embodiments, the method comprises inserting through a punctum a lacrimal implant including a body and a drug core, such that the drug core is retained near the punctum. In some embodiments, the method comprises inserting through a punctum a lacrimal implant having a body impregnated with an anti-glaucoma agent. An exposed surface of the drug core or impregnated body located near a proximal end of the implant contacts the tear or tear film fluid and the latanoprost or other anti-glaucoma agent migrates from the exposed surface to the eye over a sustained period of time while the drug core and body is at least partially retained within the punctum. In many embodiments, a method of treating an eye with an anti-glaucoma agent, for example, latanoprost, is provided, the method comprising inserting through a punctum into a canalicular lumen an implant having an optional retention structure so that the implant body is anchored to a wall of the lumen by the retention structure. The implant releases effective amounts of the anti-glaucoma agent from a drug core or other agent supply into a tear or tear film fluid of the eye. In some embodiments, the drug core may be removed from the retention structure while the retention structure remains anchored within the lumen. A replacement drug core can then be attached to the retention structure while the retention structure remains anchored. At least one exposed surface of the replacement drug core releases the anti-glaucoma agent, for example, latanoprost, at therapeutic levels over a sustained period.

A replacement drug core can be attached to the retention structure approximately every 90 days to result in continuous release of the drug to the eye for a period of time of approximately 180 days, approximately 270 days, approximately 360 days, approximately 450 days, approximately 540 days, approximately 630 days, approximately 720 days, approximately 810 days or approximately 900 days. In some embodiments, a replacement implant can be inserted through the punctum approximately every 90 days to achieve release of the drug to the eye for extended periods of time, including up to about 180 days, about 270 days, about 360 days, about 450 days, about 540 days, about 630 days, about 720 days, about 810 days or about 900 days.

A method for treating an eye with latanoprost or other anti-glaucoma agent is provided, the method comprising inserting a drug core or other implant body at least partially through at least one punctum of the eye. The drug core may or may not be associated with a separate implant body structure. The drug core or agent-impregnated implant body provides sustained release delivery of latanoprost at therapeutic levels. In some embodiments, the sustained release delivery of latanoprost or other anti-glaucoma agent continues for up to 90 days.

A method for treating an eye with an anti-glaucoma agent, for example, latanoprost, is provided, the method comprising inserting a distal end of an implant body through at least one punctum of the eye. In some embodiment, a retention structure of the implant can be expanded so as to inhibit expulsion of the implant. The expansion of the retention structure can help to occlude a flow of tear fluid through the punctum. In some embodiments, the implant body is configured such that, when implanted, an at least 45 degree angled intersection exists between a first axis, defined by a proximal end of the implant, and a second axis, defined by the distal end of the implant body, to inhibit expulsion of the implant body. Latanoprost or other anti-glaucoma agent is delivered from a proximal end of the implant body to the tear fluid adj acent the eye. Delivery of the latanoprost or other anti-glaucoma agent is inhibited distally of the proximal end.

The methods provide sustained release of latanoprost or other anti-glaucoma agent. In some embodiments, the latanoprost is released from the implant for at least one week, at least two weeks, at least three weeks, at least four weeks, at least five weeks, at least six weeks, at least seven weeks, at least eight weeks, at least nine weeks, at least ten weeks, at least eleven weeks, at least twelve weeks, at least thirteen weeks, at least fourteen weeks, at least fifteen weeks, or at least sixteen weeks. In an embodiment, the latanoprost is released for at least twelve weeks. In another embodiment, the methods of treatment according to the present invention as described above further comprises an adjunctive therapy with a latanoprost-delivering eye drop solution, for example, Xalatan®.

The amount of the anti-glaucoma agent, for example, latanoprost, associated with the implant may vary depending on the desired therapeutic benefit and the time during which the device is intended to deliver the therapy. Since the devices of the present invention present a variety of shapes, sizes and delivery mechanisms, the amount of drug associated with the device will depend on the particular disease or condition to be treated, and the dosage and duration that is desired to achieve the therapeutic effect. Generally, the amount of latanoprost is at least the amount of drug that, upon release from the device, is effective to achieve the desired physiological or pharmacological local or systemic effects.

Embodiments of the implants of the present invention can be configured to provide delivery of latanoprost or other anti-glaucoma agent at a daily rate that is substantially below the therapeutically effective drop form of treatment so as to provide a large therapeutic range with a wide safety margin. For example, many embodiments treat the eye with therapeutic levels for extended periods that are no more than 5 or 10 per cent of the daily drop dosage. In specific embodiments, the quantity can be less than 5% of the recommended drop-administered quantity. Consequently, during

an initial bolus or washout period of about one to three days, the implant can elute latanoprost at a rate that is substantially higher than the sustained release levels and well below the daily drop form dosage. For example, with an average sustained release level of 100 ng per day, and an initial release rate of 1000 to 1500 ng per day, the amount of drug initially released is less than the 2500 ng of drug that may be present in a drop of drug delivered to the eye. This use of sustained release levels substantially below the amount of drug in one or more drops administered daily allows the device to release a therapeutically beneficial amount of drug to achieve the desired therapeutic benefit with a wide safety margin, while avoiding an inadequate or excessive amount of drug at the intended site or region.

For comparison purposes, standard treatment with drops such as Xalatan® drops delivers about 1.5 micrograms of latanoprost, assuming a 35 microliter drop volume. In contrast, the implant of the instant invention will deliver an amount of drug that will be significantly less than conventional drop administration described above. Although the sustained release amount of latanoprost released each day can vary, a sustained release of approximately 100 ng per day using the implant of the invention corresponds to about 6% of the latanoprost applied with a single drop of a 0.005% or more solution.

Methods of inserting and removing the implant are known to those of skill in the art. For instance, tools for insertion and removal/extraction of implants are described in U.S. Patent Application No. 60/970,840 (filed September 7, 2007 and entitled Insertion and Extraction Tools for Punctal Implants). Generally, for placement, the size of a lacrimal implant to be used may be determined by using suitable magnification or, if provided, using a sizing gauge. The subject's punctum may be dilated if necessary to fit the lacrimal implant. A drop of lubricant may be applied if necessary to facilitate placement of the implant into the punctum. Using an appropriate placement instrument, the implant may be inserted through the superior or inferior punctum of the eye.

After placement, the cap of the implant may be visible. This process may be repeated for the subject's other eye. For removal of the implant, small ophthalmic or medical forceps may be used to securely grasp the implant at the tube section below the cap. Using a gentle tugging motion the implant may be gently retrieved.

<u>Implant:</u>

In various embodiments, latanoprost or other anti-glaucoma agent is administered for a sustained period of time by a drug core which may or may not be associated with a separate implant body structure. In certain embodiments, a lacrimal implant for use in the methods described herein is provided. The lacrimal implant can be configured, when implanted at a target location along the path of tear fluid in the subject, to release a quantity of latanoprost or other anti-glaucoma agent into the tear fluid each day for a sustained release period of days, weeks, or months. The lacrimal implant can be one of any number of different designs that releases latanoprost or other anti-glaucoma agent for a sustained period of time.

The disclosures of the following patent documents, describe example implant embodiments for use in the methods of the current invention and methods of making those implants: U.S. Application Serial No. 60/871,864 (filed December 26, 2006 and entitled Nasolacrimal Drainage System Implants for Drug Therapy); U.S. Application Serial No. 11/695,537 (filed April 2, 2007 and entitled Drug Delivery Methods, Structures, and Compositions for Nasolacrimal System); U.S. Application Serial No. 60/787,775 (filed March 31, 2006 and entitled Nasolacrimal drainage system implants for drug therapy); U.S. Application Serial No. 11/695,545 (filed Apr 2, 2007 and entitled Nasolacrimal drainage system implants for drug therapy); U.S. Application Serial No.

**[0083]** 60/970,696 (filed September 7, 2007 and entitled Expandable Nasolacrimal Drainage System Implants); U.S. Application Serial No. 60/974,367 (filed September 21, 2007 and entitled Expandable Nasolacrimal Drainage System Implants); U.S. Application Serial No. 60/970,699 (filed September 7, 2007 and entitled Manufacture of Drug Cores for Sustained Release of Therapeutic Agents); U.S. Application Serial No. 60/970,709 (filed September 7, 2007 and entitled Nasolacrimal Drainage System Implants for Drug Delivery); U.S. Application Serial No. 60/970,720 (filed September 7, 2007 and entitled Manufacture of Expandable Nasolacrimal Drainage System Implants); U.S. Application Serial No. 60/970,755 (filed September 7, 2007 and entitled Prostaglandin Analogues for Implant Devices and Methods); U.S. Application Serial No. 60/970,820 (filed September 7, 2007 and entitled Multiple Drug Delivery Systems and Combinations of Drugs with Punctal Implants); U.S. Application Serial No. 61/049,347 (filed April 30, 2008 and entitled Lacrimal Implants and Related Methods); U.S. Application Serial No. 61/049,360 (filed April 30, 2008 and entitled Lacrimal Implants and Related Methods); U.S. Application Serial No. 61/209,630 (filed March 9, 2009 and entitled Lacrimal Implants and Related Methods); U.S. Application Serial Number unknown, reference no. 2755.023PV9 (filed herewith and entitled Lacrimal Implants and Related Methods); U.S. Application Serial No. 61/036,816 (filed March 14, 2008 and entitled Lacrimal Implants and Related Methods); U.S. Application Serial No. 61/049,337 (filed April 30, 2008 and entitled Lacrimal Implants and Related Methods); U.S Application Serial No. 61/049,329 (filed April 30, 2008 and entitled Composite Lacrimal Insert); U.S Application Serial No. 61/049,317 (filed April 30, 2008 and entitled Drug-Releasing Polyurethane Lacrimal Insert); U.S. Application Serial No. 61/050,901 (filed May 6, 2008 and entitled Lacrimal implant Detection); U.S. Application Serial No. 12/231,989 (filed September 5, 2008 and entitled Lacrimal Implants and Related Methods); U.S. Application

Serial No. 61/134,271 (filed July 8, 2008 and entitled Lacrimal Implant Body Including Comforting Agent); U.S. Application Serial No. 12/231,986 (filed September 5, 2008 and entitled Drug Cores for Sustained Release of Therapeutic Agents); U.S. Application Serial No. 10/825,047 (filed April 15, 2004 and entitled Drug Delivery via Punctal Plug); International Published Application WO 2006/014434; International Application Serial No. PCT/US2007/065789 (filed March 31, 2006, published as WO 2007/115259 and entitled Nasolacrimal Drainage System Implants for Drug Therapy); International Application Serial No. PCT/US2008/010487 (filed September 5, 2008 and entitled Drug Cores for Sustained Release of Therapeutic Agents); International Application Serial No. PCT/US2008/010479 (filed September 8, 2008 and entitled Lacrimal Implants and Related Methods); U.S. Application Serial No. 61/139,456 (filed December 19, 2008 and entitled Substance Delivering Punctum Implants and Methods.)

Generally, the lacrimal implant comprises an implant body. In some embodiments, the implant body has a distal end portion and a proximal end portion. The distal end portion of the body is at least partially insertable through a punctum and into an associated canalicular lumen of the subject. The implant body may be at least impregnated with latanoprost or otherwise comprise latanoprost or latanoprost plus a corneal penetration enhancer (for example, benzalkonium chloride), such as within a matrix drug core that is inserted into the implant body. Exposure of the matrix drug core or impregnated body to a tear fluid causes an effective latanoprost release into the tear fluid over a sustained period. The implant may include a sheath disposed over at least a portion of the drug core to inhibit release of latanoprost from certain portions thereof. The implant body may have an outer surface configured to engage luminal wall tissues so as to inhibit expulsion when disposed therein.

In many embodiments, an integral feedback or other projection is connected around the sheath near the proximal end of the drug core. In an embodiment, the feedback or other projection includes one or more wings sized to remain outside the punctum so as to retain the proximal end of the drug core near the punctum. In other embodiments, the feedback or other projection includes a full or partial (e.g., trimmed) collar connected around the sheath near the proximal end of the drug core. The collar can be sized to remain outside the punctum so as to retain the proximal end of the drug core near the punctum.

In some embodiments, the implant comprises a drug core alone, lacking an additional structure surrounding the core. In some embodiments, the drug core comprises a latanoprost or other anti-glaucoma agent matrix comprising a pharmaceutically acceptable vehicle, for example, a non-bioabsorbable polymer, for example silicone in a non-homogenous mixture with the latanoprost. The non-homogeneous mixture in the drug core may comprise a silicone matrix saturated with the latanoprost or with inclusions of latanoprost. The inclusions in the drug core are a concentrated form of latanoprost, and the silicone matrix encapsulates the inclusions in the drug core. In specific embodiments, the latanoprost inclusions encapsulated within the silicone matrix comprise an inhomogeneous mixture of the inclusions encapsulated within the silicone matrix. The drug core inclusions can comprise latanoprost oil.

It is also within the scope of this invention to modify or adapt the implant device to deliver a high release rate, a low release rate, a bolus release, a burst release, or combinations thereof. A bolus of the drug may be released by the formation of an erodable polymer cap that is immediately dissolved in the tear or tear film. As the polymer cap comes in contact with the tear or tear film, the solubility properties of the polymer enable the cap to erode and the latanoprost is released all at once. A burst release of latanoprost can be performed using a polymer that also erodes in the tear or tear film based on the polymer solubility. In this example, the drug and polymer may be stratified along the length of the device so that as the outer polymer layer dissolves, the drug is immediately released. A high or low release rate of the drug could be accomplished by changing the solubility of the erodable polymer layer so that the drug layer released quickly or slowly. Other methods to release the latanoprost or other anti-glaucoma agent could be achieved through porous membranes, soluble gels (such as those in typical ophthalmic solutions), microparticle encapsulations of the drug, or nanoparticle encapsulation.

Sheath Body:

The sheath body can comprise appropriate shapes and materials to control the migration of latanoprost or other anti-glaucoma agent from the drug core. In some embodiments, the sheath body houses the drug core and can fit snugly against the core. The sheath body is made from a material that is substantially impermeable to the latanoprost or other anti-glaucoma agent so that the rate of migration of the agent may be largely controlled by the exposed surface area of the drug core that is not covered by the sheath body. In many embodiments, migration of the latanoprost or other anti-glaucoma agent through the sheath body can be about one tenth of the migration of latanoprost or other anti-glaucoma agent through the exposed surface of the drug core, or less, often being one hundredth or less. In other words, the migration of the latanoprost or other anti-glaucoma agent through the sheath body is at least about an order of magnitude less that the migration of latanoprost or other anti-glaucoma agent through the exposed surface of the drug core. Suitable sheath body materials include polyimide, polyethylene terephthalate (hereinafter "PET"). The sheath body has a thickness, as defined from the sheath surface adjacent the core to the opposing sheath surface away from the core, from about 0.00025" to about 0.0015". The total diameter of the sheath that extends across the core ranges from about 0.2

mm to about 1.2 mm. The core may be formed by dip coating the core in the sheath material. Alternatively or in combination, the sheath body can comprise a tube and the core introduced into the sheath, for example as a liquid or solid that can be slid, injected or extruded into the sheath body tube. The sheath body can also be dip coated around the core, for example dip coated around a pre-formed core.

The sheath body can be provided with additional features to facilitate clinical use of the implant. For example, the sheath may receive a drug core that is exchangeable while the implant body, retention structure and sheath body remain implanted in the subject. The sheath body is often rigidly attached to the retention structure as described above, and the core is exchangeable while the retention structure retains the sheath body. In specific embodiments, the sheath body can be provided with external protrusions that apply force to the sheath body when squeezed and eject the core from the sheath body. Another drug core can then be positioned in the sheath body. In many embodiments, the sheath body or retention structure may have a distinguishing feature, for example a distinguishing color, to show placement such that the placement of the sheath body or retention structure in the canaliculus or other body tissue structure can be readily detected by the subject. The retention element or sheath body may comprise at least one mark to indicate the depth of placement in the canaliculus such that the retention element or sheath body can be positioned to a desired depth in the canaliculus based on the at least one mark.

Retention Structure:

In many embodiments, a retention structure is employed to retain the implant in the punctum or canaliculus. The retention structure is attached to or integral with the implant body. The retention structure comprises an appropriate material that is sized and shaped so that the implant can be easily positioned in the desired tissue location, for example, the punctum or canaliculus. In some embodiments, the drug core may be attached to the retention structure via, at least in part, the sheath. In some embodiments, the retention structure comprises a hydrogel configured to expand when the retention structure is placed in the punctum. The retention structure can comprise an attachment member having an axially oriented surface. In some embodiments, expansion of the hydrogel can urge against the axially oriented surface to retain the hydrogel while the hydrogel is hydrated. In some embodiments, the attachment member can comprise at least one of a protrusion, a flange, a rim, or an opening through a portion of the retention structure. In some embodiments, the retention structure includes an implant body portion size and shape to substantially match an anatomy of the punctum and canaliculus.

The retention structure may have a size suitable to fit at least partially within the canalicular lumen. The retention structure can be expandable between a small profile configuration suitable for insertion and a large profile configuration to anchor the retention structure in the lumen, and the retention structure can be attached near the distal end of the drug core. In specific embodiments, the retention structure can slide along the drug core near the proximal end when the retention structure expands from the small profile configuration to the large profile configuration. A length of the retention structure along the drug core can be shorter in the large profile configuration than the small profile configuration.

In some embodiments, the retention structure is resiliently expandable. The small profile may have a cross section of no more than about 0.2 mm, and the large profile may have a cross section of no more than about 2.0 mm. The retention structure may comprise a tubular body having arms separated by slots. The retention structure can be disposed at least partially over the drug core.

In some embodiments, the retention structure is mechanically deployable and typically expands to a desired cross sectional shape, for example with the retention structure comprising a super elastic shape memory alloy such as Nitinol™. Other materials in addition to Nitinol™ can be used, for example resilient metals or polymers, plastically deformable metals or polymers, shape memory polymers, and the like, to provide the desired expansion. In some embodiments polymers and coated fibers available from Biogeneral, Inc. of San Diego, CA may be used. Many metals such as stainless steels and non-shape memory alloys can be used and provide the desired expansion. This expansion capability permits the implant to fit in hollow tissue structures of varying sizes, for example canaliculae ranging from 0.3 mm to 1.2 mm (i.e. one size fits all). Although a single retention structure can be made to fit canaliculae from 0.3 to 1.2 mm across, a plurality of alternatively selectable retention structures can be used to fit this range if desired, for example a first retention structure for canaliculae from 0.3 to about 0.9 mm and a second retention structure for canaliculae from about 0.9 to 1.2 mm. The retention structure has a length appropriate to the anatomical structure to which the retention structure attaches, for example a length of about 3 mm for a retention structure positioned near the punctum of the canaliculus. For different anatomical structures, the length can be appropriate to provide adequate retention force, e.g. 1 mm to 15 mm lengths as appropriate.

Although the implant body may be attached to one end of the retention structure as described above, in many embodiments the other end of the retention structure is not attached to the implant body so that the retention structure can slide over the implant body including the sheath body and drug core while the retention structure expands. This sliding capability on one end is desirable as the retention structure may shrink in length as the retention structure expands in width to assume the desired cross sectional width. However, it should be noted that many embodiments may employ

a sheath body that does not slide in relative to the core.

In many embodiments, the retention structure can be retrieved from tissue. A projection, for example a hook, a loop, or a ring, can extend from a portion of the implant body to facilitate removal of the retention structure.

In some embodiments the sheath and retention structure can comprise two parts.

Occlusive Element:

An occlusive element can be mounted to and expandable with the retention structure to inhibit tear flow. An occlusive element may inhibit tear flow through the lumen, and the occlusive element may cover at least a portion of the retention structure to protect the lumen from the retention structure. The occlusive element comprises an appropriate material that is sized and shaped so that the implant can at least partially inhibit, even block, the flow of fluid through the hollow tissue structure, for example lacrimal fluid through the canaliculus. The occlusive material may be a thin walled membrane of a biocompatible material, for example silicone, that can expand and contract with the retention structure. The occlusive element is formed as a separate thin tube of material that is slid over the end of the retention structure and anchored to one end of the retention structure as described above. Alternatively, the occlusive element can be formed by dip coating the retention structure in a biocompatible polymer, for example silicone polymer. The thickness of the occlusive element can be in a range from about 0.01 mm to about 0.15 mm, and often from about 0.05 mm to 0.1 mm.

Drug core:

The drug core may be inserted into an implant body, or may serve as the implant itself, without any additional structural components. The drug core comprises one or more active agents, in some embodiments, one or more anti-glaucoma agent, for example, latanoprost, and materials to provide sustained release of the agent. Optionally, the drug core can further include a penetration enhancer, for example, benzalkonium chloride. In some embodiments, the drug core comprises a sustained release formulation, which formulation consists of or consists essentially of latanoprost and silicone as a carrier. The latanoprost or other anti-glaucoma agent or other agent migrates from the drug core to the target tissue, for example ciliary muscles of the eye. The drug core may optionally comprise latanoprost or other anti-glaucoma agent or other agent in a matrix, wherein the latanoprost or other agent is dispersed or dissolved within the matrix. The latanoprost or other anti-glaucoma agent or other agent may be only slightly soluble in the matrix so that a small amount is dissolved in the matrix and available for release from the surface of the drug core. As the latanoprost or other anti-glaucoma agent or other agent diffuses from the exposed surface of the core to the tear or tear film, the rate of migration from the core to the tear or tear film can be related to the concentration of latanoprost or other agent dissolved in the matrix. In addition or in combination, the rate of migration of latanoprost or other anti-glaucoma agent or other agent from the core to the tear or tear film can be related to properties of the matrix in which the latanoprost or other agent is dissolved.

In some embodiments, a sheath body as described above houses the drug core and can fit snugly against the core. Suitable sheath body materials include polyimide and polyethylene terephthalate (hereinafter "PET"). The sheath body can comprise a tube and the drug core is introduced into the sheath, for example as a liquid or solid that can be slid, injected or extruded into the sheath body tube.

In specific embodiments, the rate of migration from the drug core to the tear or tear film can be based on a silicone formulation. In some embodiments, the concentration of an agent, such as an anti-glaucoma agent, for example, latanoprost, dissolved in the drug core may be controlled to provide the desired rate of release of the agent. The agent included in the core can include liquid (such as oil), solid, solid gel, solid crystalline, solid amorphous, solid particulate, or dissolved forms. In some embodiments, the drug core may comprise liquid or solid inclusions, for example liquid latanoprost droplets dispersed in the silicone matrix.

Table 1 shows drug insert silicones that may be used and associated cure properties, according to embodiments of the present invention. The drug core insert matrix material can include a base polymer comprising dimethyl siloxane, such as MED-4011, MED 6385 and MED 6380, each of which is commercially available from NuSil. The base polymer can be cured with a cure system such as a platinum-vinyl hydride cure system or a tin-alkoxy cure system, both commercially available from NuSil. In many embodiments, the cure system may comprise a known cure system commercially available for a known material, for example a known platinum vinyl hydride cure system with known MED-4011. In a specific embodiment shown in Table 2, 90 parts of MED-4011 can be combined with 10 parts of the crosslinker, such that the crosslinker comprises 10% of the mixture. A mixture with MED-6385 may comprise 2.5% of the crosslinker, and mixtures of MED-6380 may comprise 2.5% or 5% of the crosslinker.

**Table 1. Drug Insert Silicone Selections**

| Material | Base Polymer | Cure System | Crosslinker Percent |
|---|---|---|---|
| MED-4011 | Dimethyl siloxane<br>Silica filler material | Platinum vinyl hydride system 10% | 10% |
| MED-6385 | Dimethyl siloxane<br>Diatomaceous earth filler material | Tin-Alkoxy 2.5 % | 2.5% |
| MED-6380 | Dimethyl siloxane without filler material | Tin-Alkoxy | 2.5 to 5 % |

It has been determined according to the present invention that the cure system and type of silicone material can affect the curing properties of the solid drug core insert, and may potentially affect the yield of anti-glaucoma agent from the drug core matrix material. In specific embodiments, curing of MED-4011 with the platinum vinyl hydride system can be inhibited with relatively high concentrations of drug/prodrug, for example over 20% drug, such that a solid drug core may not be formed. In specific embodiments, curing of MED-6385 or MED 6380 with the tin alkoxy system can be slightly inhibited with relatively high concentrations, e.g. 20%, of drug/prodrug. This slight inhibition of curing can be compensated by increasing the time or temperature of the curing process. For example, embodiments of the present invention can make drug cores comprising 40% drug and 60% MED-6385 with the tin alkoxy system using appropriate cure times and temperatures. Similar results can be obtained with the MED-6380 system the tin-alkoxy system and an appropriate curing time or temperature. Even with the excellent results for the tin alkoxy cure system, it has been determined according to the present invention that there may be an upper limit, for example 50% drug/prodrug or more, at which the tin-alkoxy cure system may not produce a solid drug core. In many embodiments, the latanoprost in the solid drug core may be at least about 5%, for example a range from about 5% to 65%, and can be from about 20% to about 65% by weight of the drug core. For other anti-glaucoma agent, an equivalent therapeutic amount based upon therapeutic equivalency to latanoprost can be employed. Therapeutic equivalency can be determined by reference to the "Physician's Desk Reference."

The drug core or other anti-glaucoma agent supply (e.g., implant impregnated body) can comprise one or more biocompatible materials capable of providing sustained release of an agent, such as an anti-glaucoma agent, for example, latanoprost. Although the drug core is described above with respect to an embodiment comprising a matrix with a substantially non-biodegradable silicone matrix with inclusions of latanoprost located therein that dissolve, the drug core can include structures that provide sustained release of a drug, for example latanoprost, from for example a biodegradable matrix, a porous drug core, liquid drug cores and solid drug cores.

A matrix that contains latanoprost or other anti-glaucoma agent or agent can be formed from either biodegradable or non-biodegradable polymers. A non-biodegradable drug core can include silicone, acrylates, polyethylenes, polyurethane, polyurethane, hydrogel, polyester (e.g., DACRON.R™. from E. I. Du Pont de Nemours and Company, Wilmington, Del.), polypropylene, polytetrafluoroethylene (PTFE), expanded PTFE (ePTFE), polyether ether ketone (PEEK), nylon, extruded collagen, polymer foam, silicone rubber, polyethylene terephthalate, ultra high molecular weight polyethylene, polycarbonate urethane, polyurethane, polyimides, stainless steel, nickel-titanium alloy (e.g., Nitinol), titanium, stainless steel, cobalt-chrome alloy (e.g., ELGILOY.R™. from Elgin Specialty Metals, Elgin, Ill.; CONICHROME.R™. from Carpenter Metals Corp., Wyomissing, Pa.).

A biodegradable drug core can comprise one or more biodegradable polymers, such as protein, hydrogel, polyglycolic acid (PGA), polylactic acid (PLA), poly(L-lactic acid) (PLLA), poly(L-glycolic acid) (PLGA), polyglycolide, poly-L-lactide, poly-D-lactide, poly(amino acids), polydioxanone, polycaprolactone, polygluconate, polylactic acid-polyethylene oxide copolymers, modified cellulose, collagen, polyorthoesters, polyhydroxybutyrate, polyanhydride, polyphosphoester, poly(alpha-hydroxy acid) and combinations thereof. In some embodiments the drug core can comprise at least one hydrogel polymer.

Specific Implant Embodiments:

Various embodiments of lacrimal implants that may be employed in the lacrimal implant delivery systems and methods described herein are as follows (*see also*, the Example section below). In some embodiments, a drug core insertable within the lacrimal implants includes a thin-walled polyimide tube sheath body that is filled with latanoprost or other anti-glaucoma agent dispersed in NuSil 6385, a cured medical grade solid silicone, for example. The cured silicone can serve as the solid, non-erodible matrix from which latanoprost slowly elutes. The drug insert can be sealed at the distal end with a cured film of solid Loctite 4305 medical grade adhesive (cyanoacrylate). The polyimide tube sheath body is inert and, together with the adhesive, can provide structural support and a barrier to both lateral drug diffusion and drug diffusion through the distal end of the drug insert. The drug insert can be seated in a bore or other cavity of

the lacrimal implant and can be held in place via an interference fit. In some embodiments, a body of the lacrimal implant is at least partially impregnated with an anti-glaucoma agent, such as latanoprost. Optionally, the drug core can further include a penetration enhancer, for example, benzalkonium chloride.

FIG. 2A illustrates an example embodiment of a lacrimal implant (e.g., punctal plug) 200 that is insertable into a lacrimal punctum. The insertion of the lacrimal implant 200 into the lacrimal punctum allows for one or more of inhibition or blockage of tear flow therethrough (e.g., to treat dry eyes) or the sustained delivery of an anti-glaucoma agent to an eye (e.g., to treat one or more of infection, inflammation, glaucoma or other ocular diseases). In this embodiment, the lacrimal implant 200 comprises an implant body 202 extending from a proximal end portion 204 to a distal end portion 206 and having a retention structure 208.

In various embodiments, the implant body 202 can comprise an elastic material, such as silicone, polyurethane or other urethane-based material, or an acrylic of a non-biodegradable, partially biodegradable or biodegradable nature (i.e., erodeable within the body) allowing at least one portion of the retention structure to deform outward. In some embodiments, the biodegradable elastic materials include cross-linked polymers, such as poly (vinyl alcohol). In some embodiments, different portions of the implant body 202 are made of different materials. For instance, the implant body proximal end portion 204 can comprise a silicone/polyurethane co-polymer and the implant body distal end portion 206 can comprise a polyurethane hydrogel or other solid hydrogel. In certain embodiments, the implant body proximal end portion 204 can comprise silicone and the implant body distal end portion 206 can comprise a hydrophilic silicone mixture. Other co-polymers that can be used to form the implant body 302 include silicone/urethane, silicone/poly(ethylene glycol) (PEG), and silicone/2hydroxyethyl methacrylate (HEMA).

In certain embodiments, the implant body 202 can include a cylindrical-like structure having a first chamber 210 at or near the proximal end and a second chamber 212 at or near the distal end. A latanoprost drug core 214 can be disposed in the first chamber 210, while a hydrogel or other expandable retention element 216 of a biodegradable or non-biodegradable nature can be disposed in the second chamber 216. In some embodiments, the biodegradable retention elements include salt and cellulose based mixtures. In some embodiments, the non-biodegradable retention elements include hydrogels or other synthetic polymers. An implant body septum 218 can be positioned between the first chamber 210 and the second chamber 216 and can be used to inhibit or prevent communication of a material between the drug core 214 and the hydrogel retention element 216.

In various ways, the expandable, hydrogel retention element 216 can be substantially encapsulated, such as within a portion of the retention structure 208. In various embodiments, the retention structure 208 can include a fluid permeable retainer allowing fluid to be received into and absorbed or otherwise retained by the hydrogel retention element 216, such as upon its insertion into the punctum. The hydrogel retention element 216 can be configured to expand, such as to a size or shape that urges one or more outer surface portions of the retention structure 208 to contact a wall of the lacrimal canaliculus, thereby retaining or helping retain a least a portion of the implant within the punctum. In some embodiments, the fluid permeable retainer can include a fluid permeable aperture 220, such as disposed in a lateral wall of the retention structure 208. In some embodiments, the fluid permeable retainer can include a fluid permeable or hydrophilic cap member 222 or other membrane. In some embodiments, the fluid permeable retainer can include a fluid permeable or hydrophilic implant body portion 224. These examples of fluid permeable retainers 220, 222, and 224 can also inhibit the hydrogel retention element 216 from appreciably protruding out of the retention structure 208 during and upon expansion.

The implant body 202 can include a feedback or other projection 226, such as extending laterally at least partially from or around (e.g., a removal loop) a proximal end portion 204 of the implant body 202. In some embodiments, the projection 226 can include a removal loop. In some embodiments, the projection 226 can be configured to seat against or near (e.g., via a ramped portion 260) the punctum opening, such as for inhibiting or preventing the lacrimal implant 200 from passing completely within the canaliculus, or for providing tactile or visual feedback information to an implanting user regarding the same. In some embodiments, a proximal end of the projection 226 can include a convex such as for helping provide comfort to a subject when implanted. In some embodiments, the projection 226 can include a convex radius of about 0.8 millimeters. In some embodiments, the projection 226 is between about 0.7 millimeters to about 0.9 millimeters in diameter. In some embodiments, the projection 226 can include a non-concave shape of about 0.5 millimeters to about 1.5 millimeters in diameter, and 0.1 millimeters to about 0.75 millimeters in thickness. In some embodiments, the projection 226 has a wing-like shape, in which a column-like projection extends from opposite sides of the implant proximal end 204. In some examples, the projection 226 includes a partially trimmed collar extending 360 degrees around the proximal end 204 from an outer implant body surface. In some examples, such the projection 226 includes a full collar extending 360 degrees around the proximal end 204 from an outer implant body surface. In an example, the projection 226 includes a cross-sectional shape similar to a flat disk (i.e., relatively flat top and bottom surfaces). A drug or other agent elution port 228 can extend though the projection 226, such as to provide sustained release of a drug core 214 agent onto an eye.

FIG. 2B illustrates a cross-sectional view of an example embodiment of a lacrimal implant (e.g., punctal plug) 200 taken along a line parallel to a longitudinal axis of the implant, such as along line 2B-2B of FIG. 2A. As shown in

FIG. 2B, the lacrimal implant can include an implant body 202 having a retention structure 208 substantially encapsulating a hydrogel retention element 216 at or near an implant body distal end portion 206, and a latanoprost drug core 214 disposed within the implant body, for example at or near a proximal end portion 204. In this embodiment, the drug core 214 is disposed in a first implant body chamber 210 and the hydrogel retention element 216 is disposed in a second implant body chamber 212. As discussed above, the hydrogel retention element 216 can be configured to expand to a size or shape that retains or helps retain at least a portion of the implant 200 within the lacrimal punctum. In some embodiments, a hydrogel retention element 250 can also be coated or otherwise provided on an outer surface portion of the implant body 202 providing another (e.g., secondary) mechanism for retaining or helping to retain at least a portion of the implant 200 at least partially within the lacrimal punctum.

The retention structure 208, which can be used to substantially encapsulate the hydrogel retention element 216, can be of varying sizes relative to an implant body 202 size. In some embodiments, the retention structure 208 is at least about one fifth the length of the implant body 202. In some embodiments, the retention structure 208 is at least about one fourth the length of the implant body 202. In some embodiments, the retention structure 208 is at least about one third the length of the implant body 202. In some embodiments, the retention structure 208 is at least about one half the length of the implant body 202. In some embodiments, the retention structure 208 is at least about three quarters the length of the implant body 202. In some embodiments, the retention structure 208 is about the full length of the implant body 202.

As shown in the example embodiment of FIG. 2B, the hydrogel retention element 216 can have a non-expanded, "dry" state, which aids insertion through the punctum and into the lacrimal canaliculus. Once placed in the canaliculus, the hydrogel retention element 216 can absorb or otherwise retain canalicular or other fluid, such as via a fluid permeable retainer 220, 222, 224 (FIG. 2A) to form an expanded structure. In some embodiments, the hydrogel retention element 216 can include a material that is non-biodegradable. In some embodiments, the hydrogel retention element 216 can include a material that is biodegradable. Other options for the hydrogel retention element 216 can also be used. For instance, the hydrogel retention element 216 can be molded with the retention structure 208 in a single piece, or can be formed separately as one piece and subsequently coupled to the retention structure 208.

In some examples, the drug core 214 disposed at or near the proximal end portion 204 of the implant body 202 can include a plurality of latanoprost inclusions 252, which can be distributed in a matrix 254. In some embodiments, the inclusions 252 comprise a concentrated form of the latanoprost (e.g., a crystalline agent form). In some embodiments, the matrix 254 can comprise a silicone matrix or the like, and the distribution of inclusions 252 within the matrix can be non-homogeneous. In some embodiments, the agent inclusions 252 include droplets of an oil, such as latanoprost oil. In still other embodiments, the agent inclusions 252 comprise solid particles. The inclusions can be of many sizes and shapes. For instance, the inclusions can be microparticles having dimensions on the order of about 1micrometers to about 100 micrometers.

In the embodiment shown, the drug core 214 has a sheath body 256 disposed over at least a portion thereof such as to define at least one exposed surface 258 of the drug core. The exposed surface 258 can be located at or near the proximal end portion 204 of the implant body such as to contact a tear or a tear film fluid and release the latanoprost at one or more therapeutic levels over a sustained time period when the lacrimal implant 200 is inserted through the punctum.

FIG. 2C illustrates a cross-sectional view of an example embodiment of a lacrimal implant 200 taken along a line parallel to a longitudinal axis of the implant. As shown in FIG. 2C, the lacrimal implant includes an implant body 202 without a feedback or other projection 226 (FIG. 2A). In this way, the implant 200 can be completely inserted inside the lacrimal punctum. In some embodiments, the first chamber 210 can include dimensions of about 0.013 inches x about 0.045 inches. In some embodiments, the second chamber 212 can include dimensions of about 0.013 inches by about 0.020 inches.

FIG. 3A illustrates another embodiment of a lacrimal implant 300 that can be insertable into a lacrimal punctum. The insertion of the lacrimal implant 300 into the lacrimal punctum can allow for one or more of: inhibition or blockage of tear flow therethrough (e.g., to treat dry eyes) or the sustained delivery of an anti-glaucoma agent to an eye (e.g., to treat an infection, inflammation, glaucoma or other ocular disease or disorder), a nasal passage (e.g., to treat a sinus or allergy disorder) or an inner ear system (e.g., to treat dizziness or a migraine).

In this embodiment, the lacrimal implant 300 comprises an implant body 302 including first 304 and second 306 portions. The implant body 302 extends from a proximal end 308 of the first portion 304 to a distal end 310 of the second portion 306. In various embodiments, the proximal end 308 can define a longitudinal proximal axis 312 and the distal end 310 can define a longitudinal distal axis 314. The implant body 300 can be configured such that, when implanted, an at least 45 degree angled intersection 316 exists between the proximal axis 312 and the distal axis 314 for biasing at least a portion of the implant body 302 against at least a portion of a lacrimal canaliculus located at or more distal to a canaliculus curvature. In some embodiments, the implant body 302 can be configured such that the angled intersection 316 is between about 45 degrees and about 135 degrees. In this embodiment, the implant body 302 is configured such that the angled intersection 316 is approximately about 90 degrees. In various embodiments, a distal end 326 of the first

portion 304 can be integral with the second portion 306 at or near a proximal end 328 of the second portion 306.

In certain embodiments, the implant body 302 can include angularly disposed cylindrical-like structures comprising one or both of a first cavity 318 disposed near the proximal end 308 or a second cavity 320 disposed near the distal end 310. In this embodiment, the first cavity 318 extends inward from the proximal end 308 of the first portion 304, and the second cavity 320 extends inward from the distal end 310 of the second portion 306. A first drug-releasing drug supply 322 can be disposed in the first cavity 318 to provide a sustained drug release to an eye, while a second drug-releasing or other agent-releasing drug supply 324 can be disposed in the second cavity 320 to provide a sustained drug or other agent release to a nasal passage or inner ear system, for example. An implant body septum 330 can be positioned between the first cavity 318 and the second cavity 320, and can be used to inhibit or prevent communication of a material between the first drug supply 322 and the second drug supply 324.

In some embodiments, the drug or other agent release can occur, at least in part, via an exposed surface of the drug supply 322, 324. In some embodiments, by controlling geometry of the exposed surface, a predetermined drug or agent release rate can be achieved. For instance, the exposed surface can be constructed with a specific geometry or other technique appropriate to control the release rate of the drug or other agent onto an eye, such as on an acute basis, or on a chronic basis between outsubject doctor visits, for example. Further description regarding effective release rates of one or more drugs or other agents from a drug supply 322, 324 can be found in commonly-owned DeJuan et al., U.S. Application Serial No. 11/695,545 (filed Apr 2, 2007 and entitled Nasolacrimal Drainage System Implants for Drug Therapy), including its description of obtaining particular release rates. In some embodiments, the exposed surface of the drug supply 322, 324 can be flush or slightly below the proximal end 308 of the first portion 304 or the distal end 310 of the second portion 306, respectively, such that the drug supply does not protrude outside of the implant body 302. In some embodiments, the exposed surface of the drug supply 322, for instance, can be positioned above the proximal end 308 such that the drug supply 322 at least partially protrudes outside of the implant body 302.

The implant body 302 can include an integral feedback or other projection 332, such as projections extending laterally at least partially from or around a proximal end 308 of the first implant body portion 304. In some embodiments, the projection 332 can include a set of wings for use in removing the lacrimal implant 300 from an implant position. The removal set of wings can be configured without migration in mind, as the non-linear configuration of the implant body 302 can prevent migration by assuming a size or shape of the canaliculus curvature and optionally, the lacrimal canaliculus ampulla. In some embodiments, the projection 332 can be configured to seat against or near the punctal opening such as for inhibiting or preventing the lacrimal implant 300 from passing completely within the lacrimal canaliculus, or for providing tactile or visual feedback information to an implanting user, e.g., as to whether the implant is fully implanted. The projection 332 can extend laterally in a direction parallel to or away from an eye when implanted. This will reduce irritation to the eye as compared to a case in which a portion of the projection extends toward the eye. In addition, a lateral extension direction of the projection 332 from the proximal end 308 can be substantially the same as a lateral extension direction of the second implant body portion 306 relative to the distal end 326 of the first implant body portion 304. This can also avoid extension toward the eye. A drug or other agent elution port can extend though a collar-projection 332, such as to provide sustained release of the drug supply 322 agent onto an eye.

In various embodiments, the implant body 302 can be molded using an elastic material, such as silicone, polyurethane, NuSil (e.g., NuSil 4840 with 2% 6-4800) or an acrylic of a non-biodegradable, partially biodegradable or biodegradable nature (i.e., erodeable within the body) allowing a non-linear extending implant body 302 to be formed. In some embodiments, the biodegradable elastic materials can include cross-linked polymers, such as poly (vinyl alcohol). In some embodiments, the implant body 302 can comprise a silicone/ polyurethane co-polymer. Other co-polymers that can be used to form the implant body 302 include, but are not limited to, silicone/urethane, silicone/poly (ethylene glycol) (PEG), and silicone/2hydroxyethyl methacrylate (HEMA). As discussed in commonly-owned Jain et al., Application Serial No. 61/049,317 (filed April 30, 2008 and entitled Drug-Releasing Polyurethane Lacrimal Insert), urethane-based polymer and copolymer materials allow for a variety of processing methods and bond well to one another.

FIG. 3B illustrates an example embodiment of a cross-sectional view of a lacrimal implant 300 taken along a line parallel to a longitudinal axis of the implant, such as along line 3B-3B of FIG. 3A. As shown in FIG. 3B, the lacrimal implant 300 can include an implant body 302 including first 304 and second 306 portions. The implant body 302 extends from a proximal end 308 of the first portion 304 to a distal end 310 of the second portion 306. In various embodiments, the proximal end 308 can defines a longitudinal proximal axis 312 and the distal end 310 can define a longitudinal distal axis 314. The implant body 300 can be configured such that, when implanted, an at least 45 degree angled intersection 316 exists between the proximal axis 312 and the distal axis 314 for biasing at least a portion of the implant body 302 against at least a portion of a lacrimal canaliculus located at or more distal to a canaliculus curvature. In this embodiment, the implant body 300 is configured such that the angled intersection 316 is approximately about 90 degrees.

In various embodiments, a distal end 326 of the first portion 304 can be integral with the second portion 306 at or near a proximal end 328 of the second end 326. In some embodiments, the second portion 306 can include a length having a magnitude less than four times a length of the first portion 304. In one embodiment, the second portion 306 can include a length of less than about 10 millimeters, such as is shown in FIG. 3B. In another embodiment, the second

portion 306 can include a length less than about 2 millimeters.

In certain embodiments, the second portion 306 can comprise an integral dilator 350 to dilate anatomical tissue 352, such one or both of a lacrimal punctum or canaliculus to a sufficient diameter as the lacrimal implant 300 is being implanted. In this way, the lacrimal implant 300 can be implanted in various size ocular anatomies without the need for pre-dilation via a separate enlarging tool. The dilator 350 can be formed so as to not be traumatic to an inner lining of the punctum and the canaliculus. In some embodiments, a lubricious coating disposed on, or impregnated in, an outer surface of the implant body 302 can be used to further aid insertion of the lacrimal implant 300 into the anatomical tissue 352. In one embodiment, the lubricious coating can include a silicone lubricant.

As shown, the dilator 350 can generally narrow from a location near the proximal end 328 of the second portion 306 to the distal end 310 of the second portion 306, such as from a diameter of about 0.6 millimeters to a diameter of about 0.2 millimeters. In some embodiments, an outer surface slope of the dilator 350, as measured from the location near the proximal end 328 of the second portion 306 to the distal end 310 of the second portion 306, can be between about 1 degree to about 10 degrees (e.g., 2 degrees, 3 degrees, 4 degrees, or 5 degrees) with respect to the longitudinal distal axis 314. In some embodiments, the slope of the dilator 350 can be less than 45 degrees with respect to the longitudinal distal axis 314. Among other factors, a determination of a desirable dilator 350 slope for a given implant situation can be made by balancing an implant body 302 strength desirable for implant with a desire to have a soft, flexible and conforming implant body (e.g., to conform to a lacrimal canaliculus anatomy) upon implantation. In some embodiments, a diameter of a dilator tip 354 can be between about 0.2 millimeters to about 0.5 millimeters.

In certain embodiments, the proximal end 328 of the second implant body portion 306 can include a lead extension 356 configured to bias against at least a portion of a lacrimal canaliculus ampulla when implanted. In this embodiment, the lead extension 356 projects proximally from the intersection between the first 304 and second 306 implant body portions, such as in an opposite direction as the extension of the dilator 350.

In certain embodiments, the implant body 302 can include a first cavity 318 disposed near the proximal end 308. In this embodiment, the first cavity 318 extends inward about 2 millimeters or less from the proximal end 308, and houses a first drug-releasing or other agent-releasing drug supply 322 to provide a sustained drug or other agent release to an eye. In some embodiments, the drug supply 322 can include a plurality of anti-glaucoma agent inclusions 360, which can be distributed in a matrix 362. In some embodiments, the inclusions 360 can comprise a concentrated form of the anti-glaucoma agent (e.g., a crystalline agent form). In some embodiments, the matrix 362 can comprise a silicone matrix or the like, and the distribution of inclusions 360 within the matrix can be non-homogeneous. In some embodiments, the agent inclusions 360 can include droplets of oil, such as latanoprost oil. In still other embodiments, the agent inclusions 360 can comprise solid particles, such as Bimatoprost particles in crystalline form. The inclusions can be of many sizes and shapes. For instance, the inclusions can include microparticles having dimensions on the order of about 1micrometer to about 100 micrometers.

In the embodiment shown, the drug supply 322 includes a sheath body 366 disposed over at least a portion thereof such as to define at least one exposed surface 368 of the drug supply. The exposed surface 368 can be located at or near the proximal end 308 of the implant body 302 such as to contact a tear or a tear film fluid and release the anti-glaucoma agent at one or more therapeutic levels over a sustained time period when the lacrimal implant 300 is inserted through the lacrimal punctum.

FIG. 4A illustrates an embodiment of a lacrimal implant 400 that can be insertable into a lacrimal punctum. In various embodiments, the lacrimal implant 400 comprises an implant body 402, including first 404 and second 406 portions, which is sized and shaped for at least partial insertion into a lacrimal punctum. The first portion 404 is formed from a polymer and includes a first diameter 408. The second portion 406 is also formed from a polymer and includes a base member 412 (e.g., mandrel or spine-like member) having a second diameter 410, which is less than the first diameter 408. In an embodiment, the first 404 and second 406 portions are integrally coupled and comprise a unitary implant body 402. In an embodiment, the first 404 and second 406 portions are separate elements, which can be coupled to one another via an engagement between a coupling void and a coupling arm, for instance.

An expandable retention member 414, such as a swellable material, can be bonded or otherwise coupled over the base member 412 such that it envelops, at least in part, a portion of the base member 412. In an embodiment, the expandable retention member substantially envelops the base member 412. As the expandable retention member 414 absorbs or otherwise retains lacrimal or other fluid, such as upon insertion into a lacrimal punctum, its size increases and its shape may change thereby urging itself against and slightly biasing a wall of the associated canaliculus. It is believed that the expandable retention member 414 will provide retention comfort to a subject and may improve lacrimal implant 400 implant retention via controlled biasing of the canaliculus wall.

The positioning of the expandable retention member 414 over a portion of the implant body 402 allows the retention member 414 to be freely exposed to lacrimal fluid in situ, thereby allowing for a wide range of potential expansion rates. Further, the base member 412 provides an adequate coupling surface area to which the expandable retention member 414, for example, can adhere such that the material of the expandable retention member 414 does not remain in a lacrimal punctum after the lacrimal implant 400 is removed from the subject. As shown in this embodiment, the

expandable retention member 414 can include a non-expanded, "dry or dehydrated" state, which aids insertion through a lacrimal punctum and into the associated lacrimal canaliculus. Once placed into a lacrimal canaliculus, the expandable retention member 414 can absorb or other retain lacrimal fluid to form an expanded structure.

In some embodiments, the implant body 402 can include a cylindrical-like structure comprising a cavity 416 disposed near a proximal end 418 of the first portion 404. In this embodiment, the cavity 416 extends inward from the proximal end 418 and includes a first drug-releasing or other agent-releasing drug supply 420 to provide a sustained drug or other agent release to an eye. The drug or other agent release can occur, at least in part, via an exposed surface of the drug supply 420. In an embodiment, the exposed surface of the drug supply 420 can be positioned above the proximal end 418 such that the drug supply 420 at least partially protrudes outside of the implant body 402. In some embodiments, the exposed surface of the drug supply 420 can be flush or slightly below the proximal end 418 such that the drug supply 420 does not protrude outside of the implant body 402.

In some embodiments, by controlling geometry or a drug concentration gradient near the exposed surface, a predetermined drug or agent release rate can be achieved. For instance, the exposed surface can be constructed with a specific geometry or other technique appropriate to control the release rate of the drug or other agent onto an eye, such as on an acute basis, or on a chronic basis between outsubject doctor visits, for example.

The implant body 402 can include an integral feedback or other projection 422, such as projections extending laterally at least partially from or around the proximal end 418 of the first implant body portion 404. In an embodiment, the projection 422 includes a partially trimmed collar extending 360 degrees around the proximal end 418 from an outer implant body surface. In an embodiment, the projection 422 includes a full collar extending 360 degrees around the proximal end 418 from an outer implant body surface. In an embodiment, the projection 422 includes a cross-sectional shape similar to a flat disk (i.e., relatively flat top and bottom surfaces). In various embodiments, the projection 422 can be configured to seat against or near a punctal opening when the second portion 406 of the implant body 402 is positioned within the associated canalicular lumen, such as for inhibiting or preventing the lacrimal implant 400 from passing completely within the canalicular lumen, for providing tactile or visual feedback information to an implanting user (e.g., as to whether the implant is fully implanted), or for removing the lacrimal implant 400 from an implant position. In an embodiment, the projection 422 includes a portion having a diameter of about 0.5-2.0 mm to prevent the lacrimal implant 400 from passing down into the canaliculus.

FIG. 4B illustrates an example embodiment of a cross-sectional view of a lacrimal implant 400 taken along a line parallel to a longitudinal axis of the implant, such as along line 4B-4B of FIG. 4A. As shown in FIG. 4B, the lacrimal implant 400 comprises an implant body 402, including first 404 and second 406 portions, which is sized and shaped for at least partial insertion into a lacrimal punctum. The first portion 404 is formed from a polymer and includes a first diameter 408. The second portion 406 is also formed from a polymer and includes a base member 412 (e.g., mandrel or spine) having a second diameter 410, which is less than the first diameter 408. In an embodiment, the base member 412 is at least about one-third the total length of the implant body 402. In an embodiment, the base member 412 is at least about one-half the total length of the implant body 402. In the embodiment shown, the implant body 402 also includes an integral feedback or other projection 422, such as a projection extending laterally at least partially from or around a proximal end 418 of the first implant body portion 404.

In various embodiments, the implant body 402 can be molded or otherwise formed using an elastic material, such as silicone, polyurethane or other urethane-based material, or combinations thereof. In an embodiment, one or both of the first 404 and second 406 portions include a urethane-based material. In an embodiment, one or both of the first 404 and second 406 portions include a silicone-based material, such as 4840® or PurSil®. In an embodiment, one or both of the first 404 and second 406 portions include a copolymer material, such as polyurethane/silicone, urethane/carbonate, silicone/ polyethylene glycol (PEG) or silicone/2hydroxyethyl methacrylate (HEMA). In various embodiments, the implant body 402 is configured to be non-absorbable in situ and is sufficiently strong to address issues of cutting strength (e.g., during insertion and removal of the lacrimal implant 400) and dimensional stability.

An expandable retention member 414, such as a swellable material, can be bonded or otherwise coupled over the base member 412 such that it envelops, at least in part, a portion of the base member 412. As the expandable retention member absorbs or otherwise retains lacrimal fluid, such as upon insertion into a lacrimal punctum, its size increases and its shape may change thereby urging itself against and slightly biasing a wall of the associated canaliculus. In various embodiments, the expandable retention member 414 can be molded or otherwise formed using a swellable material. In an embodiment, the expandable retention member 414 includes a polyurethane hydrogel, such as TG-2000®, TG-500®, or other urethane-based hydrogel. In an embodiment, the expandable retention member 414 includes a thermoset polymer, which may be configured to swell anisotropically. In an embodiment, the expandable retention member 414 includes a gel, which does not maintain its shape upon expansion, but rather conforms to fit the shape of a canaliculus lumen wall or other surrounding structure.

In some embodiments, the lacrimal implant 400 includes a base member 412 including polyurethane or other urethane-based material and an expandable retention member 414 including a polyurethane or other urethane-based swellable material. In an embodiments, a polyurethane hydrogel is coupled directly to an outer surface, such as a plasma-treated outer surface, of the base member 412.

In some embodiments, the lacrimal implant 400 includes an intermediate member 450 positioned between a portion of the implant body 402, such as the base member 412, and a portion of the expandable retention member 414. The intermediate member 450 can include a material configured to absorb, when implanted, a greater amount of lacrimal fluid than the polymer of the base member 412 but less lacrimal fluid than the swellable polymer of the expandable retention member 414. The intermediate member 450 can provide the lacrimal implant 400 with integrity, such as between a substantially non-swelling polymer of the implant body 402 and a swelling polymer of the expandable retention member 414. For instance, when the polymer of the expandable retention member 414 swells upon exposure to moisture, it is possible that the expanding polymer will, in the absence of the intermediate member 450, swell away from the underlying, non-swelling polymer of the base member 412. In an embodiment, the intermediate member 450 includes PurSil® and is dip or otherwise coated onto an outer surface of the base member 412. In an embodiment, the intermediate member 450 includes a polyurethane configured to absorb about 10% to about 500% water, such as Tecophilic® urefhanes or Tecophilic® solution grade urethanes. Further discussion regarding the use of an intermediate member 450 positioned between a portion of a first polymer material and a portion of a second polymer material, typically different than the first polymer material, can be found in commonly-owned Sim et al., U.S Application Serial No. 61/049,329 (filed April 30, 2008 and entitled Composite Lacrimal Insert).

In certain embodiments, the implant body 402 can include a cavity 416 disposed near the proximal end 418 of the first portion 404. In an embodiment, the first cavity 416 extends inward about 2 millimeters or less from the proximal end 418, and houses a first drug-releasing or other agent-releasing drug supply 420 to provide a sustained drug or other agent release to an eye. In an embodiment, the first cavity 416 extends through the implant body 402, and houses a first drug-releasing or other agent-releasing drug supply 420. In various embodiments, the drug supply 420 stores and slowly dispenses an agent to one or both of the eye or the nasolacrimal system as they are leached out, for example, by tear film fluid or other lacrimal fluid. In an embodiment, the drug supply 420 includes a plurality of anti-glaucoma agent inclusions 452, which can be distributed in a matrix 454. In an embodiment, the inclusions 452 comprise a concentrated form of the anti-glaucoma agent (e.g., a crystalline agent form). In an embodiment, the matrix 454 comprises a silicone matrix or the like, and the distribution of inclusions 452 within the matrix are homogeneous or non-homogeneous. In an embodiment, the agent inclusions 452 include droplets of oil, such as latanoprost oil. In still another embodiment, the agent inclusions 452 include solid particles, such as Bimatoprost particles in crystalline form. The inclusions can be of many sizes and shapes. For instance, the inclusions can include microparticles having dimensions on the order of about 1 micrometer to about 100 micrometers.

In the embodiment shown, the drug supply 420 includes a sheath body 456 disposed over at least a portion thereof such as to define at least one exposed surface 458 of the drug supply. In an embodiment, the sheath body 456 comprises polyimide. The exposed surface 458 can be located at or near the proximal end 418 of the implant body 402 such as to contact a tear or a tear film fluid and release the anti-glaucoma agent at one or more therapeutic levels over a sustained time period when the lacrimal implant 400 is inserted through a lacrimal punctum.

In certain embodiments, the expandable retention member can include a second drug-releasing or other agent-releasing drug supply 460 to provide a sustained drug or other agent release to one or both of a wall of a lacrimal canaliculus or a nasolacrimal system. The drug supply 460 can be configured to store and slowly dispense an agent after contact with lacrimal fluid within a lacrimal canaliculus. In an embodiment, the agent included in the expandable retention member can comprise medicaments, anti-glaucoma agents (e.g., latanoprost), or antimicrobials (e.g., silver).

FIG. 5 illustrates an example embodiment of a cross-sectional view of a lacrimal implant 500 taken along a line parallel to a longitudinal axis of the implant. As shown in FIG. 5, the lacrimal implant 500 comprises an implant body 502. In the embodiment shown, the implant body 502 includes an integral feedback or other projection 522, such as a projection extending laterally at least partially from or around a proximal end 518 of the implant body 502. The projection 522 is in the form of a collarette extending radially outwardly from the implant body 502 to a degree sufficient so that at least a portion of the collarette will extend beyond and be exterior to the punctum after insertion of implant body 502 distal portions into the canaliculus.

In this embodiment, the implant body 502 is at least partially impregnated with a drug-releasing or other agent-releasing drug supply 520. In certain embodiments, the drug supply 520 is disposed within, dispersed throughout, or otherwise contained in the implant body 502. As discussed in commonly-owned Odrich, Application Serial No. 10/825,047 (filed April 15, 200 and entitled Drug Delivery via Punctal Plug), the agent of the drug supply 520 can be released from the implant body 502 into tear fluid of the eye or into the nasolacrimal duct system. In some embodiments, an impermeable sheath is disposed over portions of the implant body 502 to control drug supply 520 release therefrom.

Making the Lacrimal Implant:

Those of skill in the art will be familiar with various methods useful for making the lacrimal implants described herein. Particular methods are described in the above- identified patent documents.

For example, drug cores as described above may be fabricated with different cross sectional sizes of 0.006 inches, 0.012 inches, 0.0125 inches, 0.0165 inches, 0.0220 inches or 0.025 inches. Wall thickness of the tubing can vary; in some embodiments it may be about 0.005 - 0.0015 inches. Drug concentrations in the core may be about 5%, about 10%, about 20%, about 30%, about 33%, about 60%, about 63% or about 93% in a silicone matrix. These drug cores can be made with a syringe tube and cartridge assembly, mixing latanoprost or other anti-glaucoma agent with silicone, and injecting the mixture into a polyimide tube which is cut to desired lengths and sealed. In some embodiments, the length of the drug cores can be approximately 0.80 to 0.95 mm, which for a diameter of 0.012 inches (0.32 mm) corresponds to total latanoprost or other agent content in the drug cores of approximately 3.5 micrograms, 7 micrograms, 14 micrograms, 21 micrograms, or 25 micrograms. In some embodiments, drug cores of the present invention provide for concentrations of therapeutic agent of 5%, 10%, 20%, 30%, 33% and 34% by weight relative to the total weight drug core components. Optionally, the drug cores can include one or more penetration enhancers, for example, benzalkonium chloride.

Syringe Tube and Cartridge Assembly: 1. Polyimide tubing of various diameters (for example 0.006 inches, 0.012 inches, 0.0125 inches, 0.0165 inches, 0.0220 inches or 0.025 inches) can be used. The wall thickness of the tubing can vary from about 0.0005 to about 0.0015 inches. The tubing can be about 30 cm in length, or can be cut to about 15 cm in length. For example, when making drug cores containing 81 micrograms latanoprost, a 30 cm length polyimide tube can be used. When making drug cores containing 44 micrograms latanoprost, the tubing can be cut into 15 cm sections. 2. The polyimide tubes can be inserted into a Syringe Adapter. 3. The polyimide tube can be adhesive bonded into luer adapter (Loctite, low viscosity UV cure). 4. The end of the assembly can then be trimmed. 5. The cartridge assembly can be cleaned using distilled water and then with methanol and dried in oven at 60 degrees C.

In various embodiments, the latanoprost or other anti-glaucoma agent can be mixed with silicone. Latanoprost may be provided as a 1% solution in methylacetate. The appropriate amount of solution can be placed into a dish and using a nitrogen stream, the solution can be evaporated until only the latanoprost or other anti-glaucoma agent remains. The dish with the latanoprost oil, for example, can be placed under vacuum for 30 minutes. This latanoprost can then be combined with silicone, with different concentrations of latanoprost (for example, about 5%, 10%, 20%, 30%, 33%, or 34%) in silicone NuSil 6385 being injected into tubing of different diameters (for example, 0.006 inches, 0.012 inches, 0.0125 inches, 0.0165 inches, 0.0220 inches or 0.025 inches) to generate matrixes. The percent of latanoprost to silicone is determined by the total weight of the drug matrix. Calculation: Weight of latanoprost/(weight of latanoprost + weight of silicone) x 100 = percent drug.

The tube can then be injected: 1. The cartridge and polyimide tubes assembly can be inserted into a 1 ml syringe. 2. One drop of catalyst (MED-6385 Curing Agent) can be added in the syringe. 3. Excess catalyst can be forced out of the polyimide tube with clean air. 4. The syringe can then be filled with silicone drug matrix. 5. The tube can then be injected with drug matrix until the tube is filled or the syringe plunger becomes too difficult to push. 6. The distal end of the polyimide tube can be closed off and pressure can be maintained until the silicone begins to solidify. 7. Allow to cure at room temperature for 12 hours. 8. Place under vacuum for 30 minutes. 9. The tube can then be place in the correct size trim fixture (prepared in house to hold different size tubing) and drug inserts can be cut to length (0.80-0.95 mm).

In some embodiments, a silicone implant body is molded. A filament comprising a solid material, for example a coil, is wound, and heat sets the filament. The filament comprising the heat set coil is placed in a mold. The implant body is molded with the coil embedded therein. The implant body may comprise sleeves, tubes, retention structures and/or at least one chamber. The filament may comprise at least one of a heat activated material, Nitinol, a shape memory material, a polymer, polypropylene, polyester, nylon, natural fibers, stainless steel, polymethylmethacrylate or polyimide. In some embodiments, the filament may comprise an absorbable thermoplastic polymer, for example at least one of polylactic acid (PLA), poly glycolic acid (PGA) or poly-lactic-co-glycolic acid (PLGA). The heat setting of the filament can be optimized by appropriately controlling the time and/or temperature of the heat filament based on empirical data from a sample of heat set filaments, for example 10 filaments. The molding of the implant can be optimized in several ways, such as appropriate time and temperature, hard tooling of the mold, a multiple cavity mold, and mold equipment parameters. In some embodiments, a filament for removal of the drug core insert can be molded with the implant body such that the filament is embedded in the implant body and positioned near the channel that receives the drug core insert.

In some embodiments, the lacrimal implant body is molded with a coil. A hydrogel rod is molded. The hydrogel rod component is inserted into a channel of the implant body component. Windings of coil are extended over the hydrogel rod. The hydrogel rod and implant body are dip coated, for example in a hydrogel coating solution comprising for example a 5% solution of hydrogel by weight. A needle may be placed in a channel of the implant body to hold the body while the hydrogel rod and implant body are dipped in the solution.

In some embodiments, for manufacturing a drug core insert, a syringe assembly is prepared to inject a drug matrix into a polyimide tubing. A drug core matrix is prepared and injected into the tubing. The matrix is cured inside the polyimide tubing. The polyimide tubing and cured matrix is cut to a length and an adhesive is applied.

Known commercially available syringes can be used in the syringe assembly. The syringe assembly may comprise a syringe tube and cartridge assembly. The syringe tube and cartridge assembly may comprise a tube attached to a modified needle tip that attaches that attaches to a syringe. The syringe can be connected to a syringe pump or other mechanism to pressurize the tube. The syringe assembly can be used for injection of the drug core mixture and/or material into the polyimide tubing. In some embodiments, multiple syringes can be used, for example with the manufacture of drug inserts that comprise two or more drug cores. In some embodiments, the syringe assembly may comprise a manifold with two or more injection pots that can be used to with separate syringes in which each syringe includes a different drug core mixture.

The polyimide tubing is prepared for injection by attaching a 30cm or 15 cm length of polyimide tubing to a luer. The luer can be connected to the syringe for injection of the drug core mixture and/or material. In some embodiments, the tubing connected to the syringe may comprise PMMA and/or PET. In many embodiments the tubing comprises a material that inhibits release of the anti-glaucoma agent from the drug core through the tubing, for example a material that is substantially impermeable to the flow of the anti-glaucoma agent through the tubing, such that the flow of anti-glaucoma agent is directed toward the exposed end of the drug core. In some embodiments, for example drug core inserts comprising two or more concentric drug cores, the tubing may comprise concentric tubes, for example concentric polyimide tubes, with an outer tube arranged to receive and outer drug core mixture, and an inner tube arranged to receive an inner drug core mixture. With an annular drug core as described above, concentric tubes may be used to form the annular drug core, with an inner tube that can be removed after the drug core matrix material has solidified.

In some embodiments, a filament for removal of the drug core insert can be embedded in the drug core. The filament may be run through the sheath, for example tubing, and the mixture injected into the tubing. The matrix material is then cured with the filament embedded in the matrix.

A drug core mixture comprising an anti-glaucoma agent with a matrix material, for example silicone, is formed. In some embodiments, the anti-glaucoma agent may comprise at least one of latanoprost, bimatoprost or travaprost. Embodiments can use silicones that comprise dimethylsiloxane, for example Med-4011, Med-6385 and Med-6380 each of which is commercially available from NuSil of Lafayette, CA. In some embodiments, two or more drug core mixtures are prepared, each for injection for a separate drug core, for example two mixtures one for an inner drug core and one for an outer drug core.

In a specific embodiment, a drug core mixture comprising inclusions of latanoprost oil in silicone is prepared. The anti-glaucoma agent and drug core matrix material can be prepared prior to mixing the anti-glaucoma agent with the drug core matrix material. In one embodiment, latanoprost oil can be provided as a 1% solution in methyl acetate. An appropriate amount of the 1 % solution can be placed in a dish. A stream of dry nitrogen can be used to evaporate the solution until only the latanoprost remains. The dish with latanoprost oil can be placed under vacuum for 30 minutes. In some embodiments, for example those which use bimatoprost available as crystals as the anti-glaucoma agent, the evaporation and vacuum may not be used to prepare the anti-glaucoma agent.

In some embodiments with solid anti-glaucoma agent, for example bimatoprost crystals, the anti-glaucoma agent can be ground and passed through a sieve, prior to mixing with the matrix material. In some embodiments, the sieve may comprise a 120 sieve (125 um) and/or a 170 sieve (90 um). Work in relation to embodiments of the present invention indicates that a sieve may remove a very small fraction of anti-glaucoma agent and that many embodiments will work with inclusions of anti-glaucoma agent having a size greater than the optional sieve. In many embodiments, the release rate is independent of the size and/or distribution of size of the inclusions, and the release rate can be independent of particle size for particles from about 0.1 micrometer to about 100 micrometer. In some embodiments, the size and/or distribution of sizes of the particles and/or inclusions can be characterized with at least one of a sieve, light scatter measurements of the core, light microscopy of the core, scanning electron microscopy of the core or transmission electron microscopy of sections of the core. A sieve can generally be used to create desirable particle sizes and/or exclude undesirable particle sizes before mixing with the matrix. The exemplary sieve comprises a fine mesh that passes only the desired size particles or smaller, thereby limiting the anti-glaucoma agent to finer drug particles. This can be used to produce a more homogenous drug core and/or drug particle size that is easier to mix with the silicone matrix than one with excessively large particles, although significant variations among particle sizes may remain. A variety of sieves may be used. For example, a Sieve # 120 can be used so that the largest particle diameter passed is about .0049 inches. Sieve # 170 may pass particles of .0035 inch diameter or smaller. A Sieve # 70 will allow a particle size of .0083 inch diameter to pass through. Sieves may optionally be used in series.

In some embodiments, silicone, for example NuSil 6385, can be obtained from the manufacturer in a sealed container. An appropriate amount of silicone can be weighed based on the lot size of the build. The anti-glaucoma agent, for example, latanoprost, can be combined with silicone, based on the intended and/or measured percentage of anti-glaucoma agent in the drug core matrix. The percent of latanoprost to silicone can be determined by the total weight of the drug matrix. The anti-glaucoma agent, for example latanoprost, is incorporated into the silicone by weighing out the appropriate amount of the components. The following formula can be used to determine the percentage of anti-glaucoma agent in the drug core matrix:

$$\text{Percent Drug} = (\text{weight of drug}) / (\text{weight of drug} + \text{weight of silicone}) \times 100$$

For the specific example of latanoprost in silicone the percentage of latanoprost is silicone is given by:

$$(20 \text{ mg of latanoprost}) / (20 \text{ mg of latanoprost} + 80 \text{ mg of silicone}) \times 100 = 20\%.$$

The anti-glaucoma agent, for example latanoprost, is combined and mixed with the silicone using known methods and apparatus for mixing silicones. In some embodiments, the anti-glaucoma agent comprising latanoprost oil may form a micro emulsion comprising inclusions that may scatter light and appear white.

When an anti-glaucoma agent such as latanoprost, which is in a liquid physical state at about room temperature (22°C), and thus is also in a liquid physical state at human body temperature (37°C), is used, the agent and the matrix material can be mixed by techniques that bring about a high degree of dispersion of the liquid latanoprost droplets in the matrix material in which it can be substantially insoluble. Mixing techniques should provide for a dispersion of the droplet within the matrix material, such that when curing takes place, the liquid anti-glaucoma agent is present as relatively small, relatively homogeneously dispersed discrete droplets within the matrix of solid silicone material. For example, mixing can include sonication, i.e., the use of ultrasonic frequencies, such as are generated by an ultrasonic probe. The probe can be put in contact with the mixture of matrix material and liquid anti-glaucoma agent to prepare an intimate mixture of the two substantially immiscible materials. See FIG. 6 for an illustration of latanoprost content per 0.95 mm section of a filled precursor sheath. As can be seen, a uniform distribution of anti-glaucoma agent latanoprost in the silicone matrix is provided along the entire length of the 13 cm precursor sheath, which was subsequently divided into 0.95 mm sections. It is desirable to maintain a uniform content of the anti-glaucoma agent among a plurality of drug inserts manufactured by this method.

In some embodiments, the mixture of anti-glaucoma agent and silicone is injected into the tubing. A syringe, for example a 1 ml syringe, can be connected to the syringe tube and cartridge assembly. A drop of catalyst appropriate for the silicone, for example MED-6385 curing agent, can be placed into the syringe and the syringe is then filled with the uncured mixture of silicone and anti-glaucoma agent, or silicone drug matrix. The mixture, i.e., mixture of the uncured silicone and agent still liquid enough to flow or pump, can be chilled to subambient temperatures. For example, the mixture can be chilled to temperatures of less than 20°C. For example, the mixtures can be chilled to 0°C, to -5°C or to -25°C. The polyimide tube is injected with the drug/matrix mixture until the tube is filled. The tube and associated apparatus can also be chilled to maintain the subambient temperature of the mixture throughout the process of filling or injecting the sheath with the mixture. In various embodiments, the polyimide tube, or sheath, is filled with the drug matrix mixture under pressure, for example through use of a high pressure pump. For instance, the drug/matrix mixture, such as can be obtained in mixtures of latanoprost with MED-6385 Part A to which amounts of catalyst Part B have been added, can be pumped into the tube under at least about 40 psi pressure. The tube can be filled at any suitable rate, for example, at rates of less than about 0.5 linear cm/sec. Without being bound by theory, it is believed that filling the tube relatively rapidly under a relatively high head of pressure can reduce the degree of phase separation of the substantially immiscible latanoprost oil and silicone monomer material, such that upon polymerization ("curing") to provide the final silicone polymeric product, the latanoprost droplets are finely dispersed in the solid matrix in which they are only slightly soluble.

Curing takes place in the presence of the catalyst ("Part B") of the NuSil MED-6385, and can be carried out at temperatures of at least about 40°C, at relative humidity (RH) of at least about 80%, or both. Curing can be initiated directly after filling the tube and clamping the ends of the filled tube to prevent the formation of voids and loss of the precursor material from the tube ends.

After curing, which can be complete in about 16-24 hours at 40°C and 80% RH, the clamps can be removed from the ends of the tubing, as the silicone is fully set up. The tubing can then be cut into sections of suitable length for use as drug inserts, for example, lengths of about 1 mm.

When the extrusion is carried out at subambient temperatures, small and more uniform inclusions of the agent can result. For example, when the agent is latanoprost, a liquid at room temperature, extrusion at -5°C provides significantly smaller and more uniform inclusion droplets. In an example, cold extrusion yielded a drug core comprising a silicone matrix with latanoprost droplets of average diameter of 6 $\mu$m, with a standard deviation of diameter of 2 $\mu$m. In comparison, an extrusion carried out at room temperature yielded a drug core comprising a silicone matrix with latanoprost droplets of average diameter of 19 $\mu$m, with a standard deviation of droplet diameter of 19 $\mu$m, It is apparent that the cold extrusion technique provides smaller, more uniform inclusions than does extrusion at room temperature. This in turn results in a more uniform concentration of drug throughout the core, or the insert containing the core, which is desirable for medical applications as uniformity of dose is improved.

The open end of the polyimide tube can be closed off until the silicone begins to solidify. In some embodiments

with two or more drug cores, two or more separate mixtures can each be separately injected from two or more syringes.

The amount of time and temperature of the cure may be controlled, and empirical data can be generated to determine ideal times and temperatures of the curing. Work in relation with embodiments of the present invention indicates that the silicone material and drug loading of the core, for example a percentage of anti-glaucoma agent in the core, may effect the optimal time and temperature of the cure. In some embodiments, empirical data can be generated for each silicone matrix material and percentage of each anti-glaucoma agent to determine an optimal amount of time to cure the injected mixture. In some embodiments with two or drug cores in a drug core insert, two or more mixtures can be cured together to cure the drug cores of the insert.

Release of Latanoprost or Other Anti-Glaucoma Agents or other agents at Effective Levels:

The rate of release of latanoprost or other anti-glaucoma agents or other agents can be related to the concentration of latanoprost or other agent dissolved in the drug core. In some embodiments, the drug core comprises non-therapeutic agents that are selected to provide a desired solubility of the agent, for example, latanoprost, in the drug core. The non-therapeutic agent of the drug core can comprise polymers as described herein, and additives. A polymer of the core can be selected to provide the desired solubility of the latanoprost in the matrix. For example, the core can comprise hydrogel that may promote solubility of hydrophilic treatment agent. In some embodiments, functional groups can be added to the polymer to provide the desired solubility of the latanoprost in the matrix. For example, functional groups can be attached to silicone polymer. Optionally, one or more excipients, for example, a penetration enhancer, for example, benzalkonium chloride, can co-elute with the latanoprost or other agent in use.

Additives may be used to control the concentration of agent, for example, latanoprost, by increasing or decreasing solubility of the latanoprost in the drug core so as to control the release kinetics of the latanoprost. The solubility may be controlled by providing appropriate molecules or substances that increase or decrease the content of latanoprost in the matrix. The latanoprost content may be related to the hydrophobic or hydrophilic properties of the matrix and latanoprost. For example, surfactants and salts can be added to the matrix and may increase the content of hydrophobic latanoprost in the matrix. In addition, oils and hydrophobic molecules can be added to the matrix and may increase the solubility of hydrophobic treatment agent in the matrix.

Instead of or in addition to controlling the rate of migration based on the concentration of latanoprost dissolved in the matrix, the surface area of the drug core can also be controlled to attain the desired rate of drug migration from the core to the target site. For example, a larger exposed surface area of the core will increase the rate of migration of the treatment agent from the drug core to the target site, and a smaller exposed surface area of the drug core will decrease the rate of migration of the latanoprost from the drug core to the target site. The exposed surface area of the drug core can be increased in any number of ways, for example by any of castellation of the exposed surface, a porous surface having exposed channels connected with the tear or tear film, indentation of the exposed surface, protrusion of the exposed surface. The exposed surface can be made porous by the addition of salts that dissolve and leave a porous cavity once the salt dissolves. Hydrogels may also be used, and can swell in size to provide a larger exposed surface area. Such hydrogels can also be made porous to further increase the rate of migration of the latanoprost.

Further, an implant may be used that includes the ability to release two or more drugs in combination, such as the structure disclosed in U.S. Pat. No. 4,281,654 (Shell). For example, in the case of glaucoma treatment, it may be desirable to treat a subject with multiple prostaglandins or a prostaglandin and a cholinergic agent or an adrenergic antagonist (beta blocker), such as Alphagan R™., or latanoprost and a carbonic anhydrase inhibitor. In some embodiments herein, an implant is contemplated that releases both latanoprost and benzalkonium chloride or other penetration enhancer or artificial tear in combination.

In addition, drug impregnated meshes may be used such as those disclosed in US Patent Publication No. 2002/0055701 (serial no. 77/2693) or layering of biostable polymers as described in US Patent Publication No. 2005/0129731 (serial no. 97/9977). Certain polymer processes may be used to incorporate latanoprost into the devices of the present invention; such as so-called "self-delivering drugs" or PolymerDrugs (Polymerix Corporation, Piscataway, N.J.) are designed to degrade only into therapeutically useful compounds and physiologically inert linker molecules, further detailed in US Patent Publication No. 2005/0048121 (serial no. 86/1881; East). Such delivery polymers may be employed in the devices of the present invention to provide a release rate that is equal to the rate of polymer erosion and degradation and is constant throughout the course of therapy. Such delivery polymers may be used as device coatings or in the form of microspheres for a drug depot injectable (such as a reservoir of the present invention). A further polymer delivery technology may also be configured to the devices of the present invention such as that described in US Patent Publication No. 2004/0170685 (serial no. 78/8747; Carpenter), and technologies available from Medivas (San Diego, CA).

In specific embodiments, the drug core matrix comprises a solid material, for example silicone, that encapsulates inclusions of the drug, for example latanoprost. The drug comprises molecules which are very insoluble in water and slightly soluble in the encapsulating drug core matrix. The inclusions encapsulated by the drug core can be micro-particles having dimensions from about 1 micrometer to about 100 micrometers across. The drug inclusions can comprise droplets of oil, for example latanoprost oil. The drug inclusions can dissolve into the solid drug core matrix and substantially saturate the drug core matrix with the drug, for example dissolution of latanoprost oil into the solid drug core matrix. The drug dissolved in the drug core matrix is transported, often by diffusion, from the exposed surface of the drug core into the tear film. As the drug core is substantially saturated with the drug, in many embodiments the rate limiting step of drug delivery is transport of the drug from the surface of the drug core matrix exposed to the tear film. As the drug core matrix is substantially saturated with the drug, gradients in drug concentration within the matrix are minimal and do not contribute significantly to the rate of drug delivery. As surface area of the drug core exposed to the tear film is nearly constant, the rate of drug transport from the drug core into the tear film can be substantially constant. It has been determined according to the present invention that the solubility of the latanoprost in water and molecular weight of the drug can affect transport of the drug from the solid matrix to the tear. In many embodiments, the latanoprost is nearly insoluble in water and has a solubility in water of about 0.03% to 0.002% by weight and a molecular weight from about 400 grams/mol. to about 1200 grams/mol.

In many embodiments the latanoprost has a very low solubility in water, for example from about 0.03% by weight to about 0.002% by weight, a molecular weight from about 400 grams per mole (g/mol) to about 1200 g/mol, and is readily soluble in an organic solvent. Latanoprost is a liquid oil at room temperature, and has an aqueous solubility of 50 micrograms/mL in water at 25 degrees C., or about 0.005% by weight and a M.W. of 432.6 g/mol.

It has been determined according to the present invention that naturally occurring surfactants in the tear film, for example surfactant D and phospholipids, may effect transport of the drug dissolved in the solid matrix from the core to the tear film. The drug core can be configured in response to the surfactant in the tear film to provide sustained delivery of latanoprost into the tear film at therapeutic levels. For example, empirical data can be generated from a subject population, for example 10 subjects whose tears are collected and analyzed for surfactant content. Elution profiles in the collected tears for a drug that is sparingly soluble in water can also be measured and compared with elution profiles in buffer and surfactant such that an *in vitro* model of tear surfactant is developed. An *in vitro* solution with surfactant based on this empirical data can be used to adjust the drug core in response to the surfactant of the tear film.

FIG. 7 illustrates a method 700 of manufacturing a drug core comprising about 44 micrograms of latanoprost, for example. At 702, latanoprost is combined with a silicone formulation. In various embodiments, the silicone formulation includes a two part system, such as part A and part B. Part A can include the silicone and a crosslinker, while part B can contain a tin catalyst, for example, to promote crosslinking. In one embodiment, the two parts are combined in a final ratio of 200:1 (part A: part B). Additional crosslinker can be added to assist in the formation of a solid drug core. The crosslinker used in the system can be tetrapropyl orthosilicate, among other, which can be purchased from NuSil as part a three part system (MED5-6382), with the crosslinker being supplied separately. At 704, appropriate amounts of latanoprost, crosslinker and MED6385 part A and B can be dispensed onto a glass slide and mixed for approximately 2 minutes using a plastic mini spatula.

After the silicone/latanoprost mixing is complete, the mixture can loaded in the barrel of the syringe extrusion system at 706. A plunger is inserted and excess air is removed. The extrusion apparatus can be an all stainless steel jacketed tube within a tube, sanitary welded heat exchanger and includes a gas purge that is internally cooled by coiling inside the coolant side of the heat exchanger. The operating temperature of the cooling system can be 5 to -20°C, depending on the settings and capacity of the re-circulating chiller used. The temperature inside the heat exchanger should be relatively stable at extrusion temperature within $\pm$2.5°C. The steady state temperature of the cooling system can be verified prior to insertion of syringe and tubing.

In various examples, the tubing inside the heat exchanger is cooled all the way down to the sight glass, and the inner surface remains dry and protected by a slight overpressure nitrogen gas purge. The top part of the syringe cooling system can be tri-clamped to the HP7x high pressure syringe adaptor, which is connected to the EFD. The EFD is connected to compressed air source.

After setup, the EFD is activated and a silicone/latanoprost mixture is extruded down the length of the polyimide tubing at 708. The pressure can be increased gradually from 5 to 40 psi over the course of approximately 3 minutes and held at 40 psi until the mixture reaches the end of the tubing. Once the mixture reaches the bottom of the tubing, the syringe including tubing can be removed from the cooling system. The syringe can be removed by cutting the tubing with a razor blade; then, at 710, the tubing can be clamped on both ends. At 712, the clamped section of tubing can be placed in a humidity chamber to be cured at 40°C/80% RH for approximately 16-24 hours, for example. After curing, the tubing can be cut at 714 into 0.95mm long sections, cyanoacrylate glue can be applied at 716 and cured to one end of the insert, and then, at 718, inserted into a punctal plug to produce the final product, for example. The final product can then be packaged at 720 and optionally sterilized at 722.

In one embodiment, a drug core comprising about 44 micrograms of latanoprost was prepared using the following procedure. The silicone/latanoprost mixture was prepared by combining 16.8mg of latanoprost, 33.2mg of silicone MED6385 part A, 0.15$\mu$L of silicone MED6385 part B and 1.0$\mu$L of additional crosslinker on a glass slide. The components were mixed for approximately 2 minutes using a small plastic spatula. After mixing, the mixture was loaded

into the barrel of the syringe extrusion system and the plunger was inserted and depressed to remove excess air. The syringe was then loaded into the chilled extrusion apparatus and allowed to equilibrate for 2 minutes to the extrusion temperature of -10°C. The EFD was activated and the pressure was gradually extruded from 5 to 40psi until the mixture was extruded down the length of the polyimide tubing to the end. The syringe is then removed from the extrusion apparatus, the tubing is cut using a razor blade and then clamped on both ends. The tubing was then cured at 40°C/80% RH for approximately 16-24 hours in a control humidity and temperature chamber. After curing the tubing was unclamped and cut into 0.95mm long sections. Glue was applied and cured to one end of the insert and then inspected for length and physical attributes. The inserts were inserted into in the bore of the punctal plug. The assembled punctal plug was packaged into a Tyvec pouch and heat sealed. The Tyvec pouch was then packaged into a aluminum laminate pouch which was backfilled with nitrogen before heatsealing. The packaged punctal plugs were then sent out for sterilization by e-beam irradiation.

The drug cores may also be modified to utilize carrier vehicles such as nanoparticles or microparticles depending on the size of the molecule to be delivered such as latent-reactive nanofiber compositions for composites and nanotextured surfaces (Innovative Surface Technologies, LLC, St. Paul, Minn.), nanostructured porous silicon, known as BioSilicon.R™., including micron sized particles, membranes, woven fivers or micromachined implant devices (pSividia, Limited, UK) and protein nanocage systems that target selective cells to deliver a drug (Chimeracore).

In many embodiments, the drug insert comprises of a thin-walled polyimide tube sheath with a drug core comprising latanoprost dispersed in NuSil 6385 (MAF 970), a medical grade solid silicone that serves as the matrix for drug delivery. The distal end of the drug insert is sealed with a cured film of solid Loctite 4305 medical grade adhesive. The drug insert may be placed within the bore of the lacrimal implant, the Loctite 4305 adhesive does not come into contact with either tissue or the tear film. The inner diameter of the drug insert can be 0.32 mm; and the length can be 0.95 mm. In embodiments of the present invention, various latanoprost concentrations in the finished drug product can be employed: Drug cores can comprise 3.5, 7, 14, 21, 44 or 81 micrograms of latanoprost, with per cent by weight concentrations of 5, 10, 20, 30, or 34%, respectively. Assuming an overall elution rate of approximately 100 ng/day, the drug core comprising 14 micrograms of latanoprost is configured to deliver drug for approximately at least 100 days, for example 120 days. The overall weight of the drug core, including latanoprost, can be about 70 micrograms. The weight of the drug insert including the polyimide sleeve can be approximately 100 micrograms.

In many embodiments, the drug core may elute with an initial elevated level of latanoprost followed by substantially constant elution of the latanoprost. In many instances, an amount of latanoprost released daily from the core may be below the therapeutic levels and still provide a benefit to the subject. An elevated level of eluted latanoprost can result in a residual amount of latanoprost or residual effect of the latanoprost that is combined with a sub-therapeutic amount of latanoprost to provide relief to the subject. In embodiments where therapeutic level is about 80 ng per day, the device with a 44 microgram drug core may deliver about 1500ng-3000ng on the first day, between 500-1000ng on days 2 through 7, and 300-500ng of latanoprost thereafter for at least 90 days or 120 days in total. As the amount of drug delivered can be precisely controlled, an initial elevated dose may not result in complications or adverse events to the subject.

In certain embodiments, the methods of the invention result in a percentage reduction in intraocular pressure of approximately 28%. In some embodiments, the methods of the invention results in a percentage reduction or decrease in intraocular pressure of approximately 30%, approximately 29%, approximately 28%, approximately 27%, approximately 26%, approximately 25%, approximately 24%, approximately 23%, approximately 22%, approximately 21%, or approximately 20%. In certain embodiments, the methods of the invention result in a percentage reduction or decrease in intraocular pressure of at least 30%, at least 29%, at least 28%, at least 27%, at least 26%, at least 25%, at least 24%, at least 23%, at least 22%, at least 21%, at least 20%, at least 15%, or at least 10%.

In certain embodiments, the methods of the invention result in a reduction in intraocular pressure from baseline of about 9 mmHg, about 8.5 mmHg, about 8 mmHg, about 7.5 mmHg, about 7 mmHg, about 6.5 mmHg, about 6 mmHg, about 5.5 mmHg, about 5 mmHg, about 4.5 mmHg, about 4 mmHg, about 3.5 mmHg, about 3 mmHg, about 2.5 mmHg, or about 2 mmHg. In certain embodiments, the methods of the invention result in a reduction in intraocular pressure from baseline of at least 2 mmHg, at least 3 mmHg, at least 4 mmHg, at least 5 mmHg, at least 6 mmHg, or at least 7 mmHg. In some embodiments, intraocular pressure is reduced to less than or equal to 21 mmHg, less than or equal to 20 mmHg, less than or equal to 19 mmHg, less than or equal to 18 mmHg, less than or equal to 17 mmHg, less than or equal to 16 mmHg, less than or equal to 15 mmHg, less than or equal to 14 mmHg, less than or equal to 13 mmHg, or less than or equal to 12 mmHg.

In an embodiment, the implants and methods of the invention provide a 90-day course of treatment. In some embodiments, effective levels of latanoprost release during the entire course of treatment. In a further embodiment, the variability in intraocular pressure over the course of treatment is less than about 1 mmHg. In other embodiments, the variability in intraocular pressure over the course of treatment is less than about 2 mmHg. In other embodiments, the variability in intraocular pressure over the course of treatment is less than about 3 mmHg.

The implants described herein may be inserted through the superior punctum, the inferior punctum, or both,

and may be inserted into one or both eyes of the subject. In some embodiments, the lacrimal implant delivery system is inserted bilaterally into the lower (inferior) puncta of the eyes.

Lacrimal Implants containing excipients:

The present invention is also directed to various embodiments of lacrimal implants containing therapeutic agents for use in implant bodies adapted for disposition in a body tissue, fluid, cavity, or duct, and containing one or more excipient as disclosed herein that can alter one or more of several properties of the implant. These properties include an amount of the therapeutic agent that can be substantially uniformly dispersed in the matrix, a rate of the release of the agent from the matrix after emplacement in living body tissues, and the pharmacokinetic behavior of the agent in the tissue.

The implants can be adapted to be disposed in or adjacent to an eye of a patient. The implants release the agent to the body, for example, into an eye or surrounding tissues, or both, over a period of time, for treatment of a malcondition in the patient for which use of the therapeutic agent is medically indicated. The invention is also directed to various embodiments of methods of manufacture of the drug inserts, and to methods of treatment of patients using implants containing the drug inserts.

The drug cores, implants, and methods of manufacturing the same, as described herein, can take any one of a number of different designs, configurations, or arrangements, such as are described in the following patent documents: U.S. Application Serial No. 60/871,864 (filed December 26, 2006 and entitled Nasolacrimal Drainage System Implants for Drug Therapy); U.S. Application Serial No. 11/695,537 (filed April 2, 2007 and entitled Drug Delivery Methods, Structures, and 60/970,709 (filed September 7, 2007 and entitled Nasolacrimal Drainage System Implants for Drug Delivery); U.S. Application Serial No. 60/970,720 (filed September 7, 2007 and entitled Manufacture of Expandable Nasolacrimal Drainage System Implants); U.S. Application Serial Compositions for Nasolacrimal System); U.S. Application Serial No. 60/787,775 (filed March 31, 2006 and entitled Nasolacrimal drainage system implants for drug therapy); U.S. Application Serial No. 11/695,545 (filed Apr 2, 2007 and entitled Nasolacrimal drainage system implants for drug therapy); U.S. Application Serial No. 60/970,696 (filed September 7, 2007 and entitled Expandable Nasolacrimal Drainage System Implants); U.S. Application Serial No. 60/974,367 (filed September 21, 2007 and entitled Expandable Nasolacrimal Drainage System Implants); U.S. Application Serial No. 60/970,699 (filed September 7, 2007 and entitled Manufacture of Drug Cores for Sustained Release of Therapeutic Agents); U.S. Application Serial No. 60/970,709 (filed September 7, 2007 and entitled Nasolacrimal Drainage System Implants for Drug Delivery); U.S. Application Serial No. 60/970,720 (filed September 7, 2007 and entitled Manufacture of Expandable Nasolacrimal Drainage System Implants); U.S. Application Serial No. 60/970,755 (filed September 7, 2007 and entitled Prostaglandin Analogues for Implant Devices and Methods); U.S. Application Serial No. 60/970,820 (filed September 7, 2007 and entitled Multiple Drug Delivery Systems and Combinations of Drugs with Punctal Implants); U.S. Application Serial No. 61/049,347 (filed April 30, 2008 and entitled Lacrimal Implants and Related Methods); U.S. Application Serial No. 61/049,360 (filed April 30, 2008 and entitled Lacrimal Implants and Related Methods); U.S. Application Serial No. 61/209,630 (filed March 9, 2009 and entitled Lacrimal Implants and Related Methods); U.S. Application Serial Number unknown, reference no. 2755.023PV9 (filed herewith and entitled Lacrimal Implants and Related Methods); U.S. Application Serial No. 61/036,816 (filed March 14, 2008 and entitled Lacrimal Implants and Related Methods); U.S. Application Serial No. 61/049,337 (filed April 30, 2008 and entitled Lacrimal Implants and Related Methods); U.S Application Serial No. 61/049,329 (filed April 30, 2008 and entitled Composite Lacrimal Insert); U.S Application Serial No. 61/049,317 (filed April 30, 2008 and entitled Drug-Releasing Polyurethane Lacrimal Insert); U.S. Application Serial No. 61/050,901 (filed May 6, 2008 and entitled Lacrimal implant Detection); U.S. Application Serial No. 12/231,989 (filed September 5, 2008 and entitled Lacrimal Implants and Related Methods); U.S. Application Serial No. 61/134,271 (filed July 8, 2008 and entitled Lacrimal Implant Body Including Comforting Agent); U.S. Application Serial No. 12/231,986 (filed September 5, 2008 and entitled Drug Cores for Sustained Release of Therapeutic Agents); U.S. Application Serial No. 10/825,047 (filed April 15, 2004 and entitled Drug Delivery via Punctal Plug); International Published Application WO 2006/014434; International Application Serial No. PCT/US2007/065789 (filed March 31, 2006, published as WO 2007/115259 and entitled Nasolacrimal Drainage System Implants for Drug Therapy); International Application Serial No. PCT/US2008/010487 (filed September 5, 2008 and entitled Drug Cores for Sustained Release of Therapeutic Agents); International Application Serial No. PCT/US2008/010479 (filed September 8, 2008 and entitled Lacrimal Implants and Related Methods); U.S. Application Serial No. 61/139,456 (filed December 19, 2008 and entitled Substance Delivering Punctum Implants and Methods).

It has been surprisingly discovered that addition of certain excipients to the drug core, which is composed of the polymeric matrix and the therapeutic agent, can unexpectedly alter the performance or behavior of the drug core when emplaced within a living body. For example, it has been unexpectedly found that certain excipients can increase a rate of release of the agent after emplacement of the implant containing the core in a living body, such as in the punctum of a human eye. Other excipients can act to retard the release of the agent under the same conditions.

Some excipients can provide benefits in administration of the agent, such as latanoprost, to a tissue such as

the eye in terms of a longer residence time of the agent adjacent to the implant, for example in tear fluids or disposed upon the eye surface. Or, some excipients can enhance penetration of adjacent tissue by the agent after emplacement of the implant, for example, increase corneal penetration by a prostanoid.

In some embodiments, there is provided a high degree of homogeneity of the therapeutic agent throughout the matrix of the drug core. Many agents do not dissolve at any appreciable concentration in the polymeric material, but rather form inclusion bodies, either solid particles or liquid droplets, within the polymeric matrix. It is desirable to maintain a high degree of uniformity of dispersion of the agent within the matrix at sub-millimeter levels, to provide for uniformity of the often physically small (e.g. 1 mm length) drug cores, such as those adapted for emplacement in the punctum of the eye. On the other hand, it can be advantageous to achieve a high level of concentration of the agent or drug in the matrix, to provide effective quantities to the target tissues over a period of time, such as a period of days or weeks.

It has been surprisingly discovered that certain excipients of the invention can allow for a higher loading of the therapeutic agent within the drug core matrix while preserving desirable homogeneity of dispersion of inclusion bodies within the polymeric matrix.

In various embodiments, the invention provides a drug core adapted for disposition within a sheath and of that sheathed core within an implant body to provide the complete implant assembly. This implant is adapted for disposition within or adjacent to an eye of a patient, for providing sustained release of a therapeutic agent to the eye or surrounding tissues or both, wherein the sheath serves to spatially limit release of the agent into surrounding tissues. For example, a cylindrical sheath open only at one end can limit the release of the agent to the open end, provided the sheath is substantially impermeable itself to diffusion of the agent therethrough.

In some embodiments, the drug core comprises a therapeutic agent, one or more excipients as disclosed herein, and a matrix wherein the matrix comprises a polymer, wherein an amount of the therapeutic agent in a volumetric portion of the drug core is similar to an amount of the therapeutic agent in any other equal volumetric portion of the drug core.

In some embodiments, the drug core comprises excipients that modify the release rate of the agent to the body tissue such as by increasing or decreasing the release rate, or increase the residence time of the agent in the adjacent tissue, or provide for enhanced tissue penetration, such as corneal penetration in the eye. The excipients can also allow a higher drug loading to be achieved in the drug core composition while preserving the desirable attribute of a substantially homogeneous distribution of inclusions of the agent within the polymeric matrix forming the core.

In various embodiments, the invention provides an implant configured for disposition within or adjacent to a body cavity, tissue, duct, or fluid, the implant comprising:
a drug core comprising:

(a) a matrix including a polymer;
(b) a therapeutic agent dissolved or dispersed within the matrix, and
(c) an excipient dissolved or dispersed within the matrix, the excipient configured to any of

(1) modify a release rate of the therapeutic agent into the body cavity, tissue, duct, or fluid relative to a comparable release rate in the absence of an excipient; or
(2) increase a loading of the therapeutic agent substantially uniformly dissolved or dispersed within the matrix, relative to a comparable loading of the therapeutic agent that is substantially uniformly dissolved or dispersed, in the absence of an excipient;
(3) increase retention of the agent at or adjacent to a site of release in a living body or increase penetration of adjacent body tissue by the agent, or both, relative to the retention or penetration or both from a comparable implant in the absence of the excipient;

or any combination thereof;

wherein an amount of the therapeutic agent in a volumetric portion of the matrix is similar to an amount of the therapeutic agent in any other equal volumetric portion of the matrix; characterized in that the polymer of the matrix is a non-biodegradable polymer; and wherein the implant is an ocular implant, adapted for disposition through a punctum and into a canaliculus of a human eye for release of the therapeutic agent therefrom;

and, optionally an implant body adapted to receive the drug core therewithin for placement within the body cavity, tissue, duct, or fluid.

As is disclosed in published PCT application PCT/US2008/010487, filed September 5, 2008, published as WO 2009/035562 on March 19, 2009 and, a drug core comprises a matrix and a therapeutic agent. As is disclosed and claimed herein, the drug core can further comprise an excipient that unexpectedly modifies the properties of the drug

core, for example in terms of the drug loading achievable in the core, and in the release of the drug from the core following emplacement within or adjacent to living tissue of a patient.

A therapeutic agent or drug for use in the inventive insert or core can include anti-glaucoma medications, (e.g. adrenergic agonists, adrenergic antagonists (beta blockers), carbonic anhydrase inhibitors (CAIs, systemic and topical), parasympathomimetics, prostaglandins such as latanoprost, and hypotensive lipids, and combinations thereof), antimicrobial agent (e.g., antibiotic, antiviral, antiparasitic, antimycotic, etc.), a corticosteroid or other anti-inflammatory (e.g., an NSAID or other analgesic and pain management compounds) such as cyclosporine or olopatidine, a decongestant (e.g., vasoconstrictor), an agent that prevents of modifies an allergic response (e.g., an antihistamine, cytokine inhibitor, leucotriene inhibitor, IgE inhibitor, immunomodulator such as cyclosporine), a mast cell stabilizer, cycloplegic, mydriatic or the like.

Examples of agents further include, but are not limited to, thrombin inhibitors; antithrombogenic agents; thrombolytic agents; fibrinolytic agents; vasospasm inhibitors; vasodilators; antihypertensive agents; antimicrobial agents, such as antibiotics (such as tetracycline, chlortetracycline, bacitracin, neomycin, polymyxin, gramicidin, cephalexin, oxytetracycline, chloramphenicol, rifampicin, ciprofloxacin, tobramycin, gentamycin, erythromycin, penicillin, sulfonamides, sulfadiazine, sulfacetamide, sulfamethizole, sulfisoxazole, nitrofurazone, sodium propionate), antifungals (such as amphotericin B and miconazole), and antivirals (such as idoxuridine trifluorothymidine, acyclovir, gancyclovir, interferon); inhibitors of surface glycoprotein receptors; antiplatelet agents; antimitotics; microtubule inhibitors; anti-secretory agents; active inhibitors; remodeling inhibitors; antisense nucleotides; anti-metabolites; antiproliferatives (including antiangiogenesis agents); anticancer chemotherapeutic agents; anti-inflammatories (such as cyclosporine, olopatidine, hydrocortisone, hydrocortisone acetate, dexamethasone 21-phosphate, fluocinolone, medrysone, methylprednisolone, prednisolone 21-phosphate, prednisolone acetate, fluoromethalone, betamethasone, triamcinolone, triamcinolone acetonide); non steroidal anti-inflammatories (NSAIDs) (such as salicylate, indomethacin, ibuprofen, diclofenac, flurbiprofen, piroxicam indomethacin, ibuprofen, naxopren, piroxicam and nabumetone). Examples of such anti-inflammatory steroids contemplated for use with the present punctum plugs, include triamcinolone acetonide and corticosteroids that include, for example, triamcinolone, dexamethasone, fluocinolone, cortisone, prednisolone, flumetholone, and derivatives thereof.); antiallergenics (such as sodium chromoglycate, antazoline, methapyriline, chlorpheniramine, cetrizine, pyrilamine, prophenpyridamine); anti proliferative agents (such as 1,3-cis retinoic acid, 5-fluorouracil, taxol, rapamycin, mitomycin C and cisplatin); decongestants (such as phenylephrine, naphazoline, tetrahydrazoline); miotics and anticholinesterase (such as pilocarpine, salicylate, carbachol, acetylcholine chloride, physostigmine, eserine, diisopropyl fluorophosphate, phospholine iodine, demecarium bromide); antineoplastics (such as carmustine, cisplatin, fluorouracil); immunological drugs (such as vaccines and immune stimulants); hormonal agents (such as estrogens, estradiol, progestational, progesterone, insulin, calcitonin, parathyroid hormone, peptide and vasopressin hypothalamus releasing factor); immunosuppressive agents, growth hormone antagonists, growth factors (such as epidermal growth factor, fibroblast growth factor, platelet derived growth factor, transforming growth factor beta, somatotrapin, fibronectin); inhibitors of angiogenesis (such as angiostatin, anecortave acetate, thrombospondin, anti-VEGF antibody); dopamine agonists; radiotherapeutic agents; peptides; proteins; enzymes; extracellular matrix; components; ACE inhibitors; free radical scavengers; chelators; antioxidants; anti polymerases; photodynamic therapy agents; gene therapy agents; and other therapeutic agents such as prostaglandins, antiprostaglandins, prostaglandin precursors, including antiglaucoma drugs including beta-blockers such as timolol, betaxolol, levobunolol, atenolol, and prostaglandin analogues such as bimatoprost, travoprost, latanoprost etc; carbonic anhydrase inhibitors such as acetazolamide, dorzolamide, brinzolamide, methazolamide, dichlorphenamide, diamox; and neuroprotectants such as lubezole, nimodipine and related compounds; and parasympathomimetrics such as pilocarpine, carbachol, physostigmine and the like.

Additional agents that can be used with the present implants include, but are not limited to, drugs that have been approved under Section 505 of the United States Federal Food, Drug, and Cosmetic Act or under the Public Health Service Act, some of which can be found at the U.S. Food and Drug Administration (FDA) website: http://www.accessdata.fda.gov/scripts/cder/drugsatfda/index. The present punctum plugs can also be used with drugs listed in the Orange Book, either in paper or in electronic form, which can be found at the FDA Orange Book website (http://www.fda.gov/cder/ob/)), that has or records the same date as, earlier date than, or later date than, the filing date of this patent document. For example, these drugs can include, among others, dorzolamide, olopatadine, travoprost, bimatoprost, cyclosporin, brimonidine, moxifloxacin, tobramycin, brinzolamide, aciclovir timolol maleate, ketorolac tromethamine, prednisolone acetate, sodium hyaluronate, nepafenac, bromfenac,diclofenac, flurbiprofen, suprofenac, binoxan, patanol, dexamethasone/tobramycin combination, moxifloxacin, or acyclovir.

In various embodiments, the agent can be a prostaglandin analog, such as latanoprost, bimatoprost, or travoprost, and the amount of the agent in the drug insert can be about 10-100 $\mu$g. In various embodiments, the drug core, exclusive of the sheath, if present, can comprises about 0.1 wt% to about 50 wt% of the agent such as latanoprost.

A matrix can comprise a polymeric material, for example, the matrix can include a silicone, a polyurethane, or any non-biodegradable polymer wherein the agent has at least sufficient solubility to diffuse therethrough. The matrix can comprise other materials, including but not limited to other types of polymers such as polyolefins, polyamides,

polyesters, polyvinyl alcohol or acetate, ethylene-vinyl acetate copolymers, polysaccharides such as cellulose or chitin, or the like, provided the material is biocompatible. Accordingly, selection of a material for the matrix can be made at least in part based on the agent selected for the particular application intended, such that a sufficient degree of solubility of the agent in the matrix can be achieved for a therapeutic level of the agent in the target tissue can be maintained over a period of time. In various embodiments, the invention provides an implant wherein the matrix comprises a silicone, optionally crosslinked, or a polyurethane polymer. For example, the matrix can comprise a MED6385 silicone polymer crosslinked with tetraethylorthosilicate.

In various embodiments, the implant can comprise a sheath partially surrounding the drug core, at least a portion of the sheath body intermediate a drug core surface and a wall of the implant body lumen. The drug core can be partially covered by a sheath, but whether or not a sheath is present, the drug core can be emplaced within a lumen of an implant body adapted to receive it. The implant body can then be emplaced within or adjacent to living tissue of a patient for controlled or sustained release of the agent therefrom. For example, the implant body can be adapted for disposition within a punctum of a human eye.

In various embodiments, a drug core comprising a composite of a therapeutic agent and a matrix is partially contained within or surrounded by a sheath, the sheath being substantially impermeable to the agent. The sheath can cover part, but not all, of the surface of the core comprising the drug and the matrix material, the core having an exposed surface such that the therapeutic agent can be released therethrough. In various embodiments, the sheath body can comprise a polymer comprising at least one of polyimide, PMMA, or PET, wherein the polymer is extruded or cast; or a metal comprising stainless steel or titanium.

The drug core and its sheath together are adapted for inclusion within an implant structure that is itself adapted for implantation within a body of a patient, such as within a body cavity, tissue, duct, or fluid. For example, the implant can be an ocular implant, adapted for disposition in or around the eye, such as a punctal plug, adapted to disposition within the canaliculus of the eye such that the agent can be released through the punctum of the eye to contact the orb and surrounding tissues, for example the sclera, the conjunctiva, the cul-de-sac of the eyelid, the trabecular meshwork, the ciliary body, the cornea, the choroid, the suprachoroidal space, or tissues within the orb such as the vitreous humor, aqueous humor and retina.

In various embodiments, the implant can be an ocular implant, adapted for disposition within a punctum of a human eye for release of the therapeutic agent therefrom.

In various embodiments, the therapeutic agent is substantially uniformly and homogeneously dissolved in the matrix or the agent at least partially forms solid or liquid inclusions, the inclusions having an average diameter less than about 50 microns, the inclusions being substantially uniformly dispersed throughout the matrix on a sub-millimeter scale.

In various embodiments, the agent is insufficiently soluble in the matrix to form a solid solution. In these embodiments, the agent can be distributed at least in part as a plurality of solid or liquid inclusions throughout the matrix, the inclusions comprising, at a temperature of about 20°C, droplets of the agent of no greater than about 50 $\mu$m diameter when the agent is a liquid at about 20°C, or particles of the agent of no greater than about 50 $\mu$m diameter when the agent is a solid at about 20°C; wherein the inclusions of the agent are dispersed throughout each drug core.

The size and size distribution of the inclusions can have an effect on a rate of release of the agent from the drug core to the patient. For example, smaller, more uniform inclusions can serve to infuse the bulk matrix with the agent more effectively, at a higher rate, due to a more favorable surface area to volume ratio. Accordingly, inventive methods provide for control or regulation of the average inclusion diameter or the distribution of inclusion diameters. In various embodiments, the distribution of diameters of the inclusions can be a monodisperse distribution. In various embodiments, the inclusions predominantly comprise a cross-sectional size within a range from about 0.1 $\mu$m to about 50 $\mu$m. It is believed that tight, or monodisperse, distributions of inclusion diameter are favorable from the point of view of therapeutic aspects of the drug core or a drug insert containing the core.

Various embodiments of the invention also provide a drug core or an insert containing a drug core wherein the agent forms inclusions in the matrix that are in a liquid physical state at about 20°C. For example, substantially all the inclusions can be droplets of the agent of less than about 50 $\mu$m in diameter within the matrix. An example of an agent in a liquid physical state at about 20°C is latanoprost.

Various embodiments of the invention also provide a drug core or an insert containing a drug core wherein the agent forms inclusions in the matrix that are in a solid physical state at about 20°C. For example, substantially all the inclusions can be particles of the agent of less than about 50 $\mu$m in diameter within the matrix. For example, an average particle diameter within the matrix can be about 5-50 $\mu$m. Examples of agents in a solid physical state at about 20°C include bimatoprost, olopatadine, or cyclosporine.

In various embodiments, the therapeutic agent, such as latanoprost, is contained in the matrix such that an amount of the therapeutic agent in a volumetric portion of the drug core is similar to an amount of the therapeutic agent in any other equal volumetric portion of the drug core. For example, the amount of the therapeutic agent in a volumetric portion of the drug core can vary from the amount of the therapeutic agent in any other equal volumetric portion of the drug core by no greater than about 30%. For example, the amount of the therapeutic agent in a volumetric portion of

the drug core can vary from the amount of the therapeutic agent in any other equal volumetric portion of the drug core by no greater than about 20%. For example, the amount of the therapeutic agent in a volumetric portion of the drug core can vary from the amount of the therapeutic agent in any other equal volumetric portion of the drug core by no greater than about 10%. For example, the amount of the therapeutic agent in a volumetric portion of the drug core can vary from the amount of the therapeutic agent in any other equal volumetric portion of the drug core by no greater than about 5%. In addition, the concentration of the therapeutic agent in a volumetric portion of the drug core can be the same as any other equal volumetric portion of the drug core, in certain embodiments including those embodiments wherein the agent is present as a uniform, homogeneous dispersion and in embodiments wherein the agent is present in solid or liquid inclusions throughout the matrix.

In various embodiments, the drug core can comprise an excipient comprising a phospholipid, a polyhydric alcohol, a polyethyleneglycol, or any combination thereof. The excipient can modify a release rate of the agent into tissue after emplacement within or adjacent to the tissue, or the excipient can alter the pharmacokinetic properties of the agent within the tissue, such as tissue residence time or penetration. For example, an excipient can increase a degree of corneal penetration of an agent that is released into tear fluid on the surface of the eye.

For example, an excipient can modify a release rate of a drug wherein the release rate is a release rate into tear fluid of the therapeutic agent from an implant disposed in a punctum of a patient and the release rate is increased relative to a release rate in the absence of the excipient. More specifically, the release rate can be increased over a period of time of about 1 to about 10 days following disposition of the ocular implant within the punctum.

In various embodiments, when inclusions of the therapeutic agent in the drug core are present in the matrix, the inclusions are of a more uniform size and are more uniformly dispersed in the matrix relative to a size and dispersion of inclusions within a matrix in a comparable drug core with a comparable loading of the agent in the absence of the excipient.

In some embodiments, an excipient can increase an achievable drug loading, such that the therapeutic agent is present at a higher loading or concentration within the matrix of the drug core in the presence of the excipient than could be achieved with a comparably homogeneous dispersion in a comparable matrix in the absence of an excipient. As discussed above, a high degree of homogeneity of dispersion of the agent within the matrix is desirable, and drug cores that do not maintain a suitable degree of uniformity are unsuitable for use as controlled or sustained release implants in living body tissue. At higher drug loadings within the matrix, a substantially uniform dispersion of the drug within the matrix is difficult to achieve. Phase separation can occur. Referring to FIGS. 13 and 14, it can be seen that in the absence of an inventive drug core comprising an excipient (FIG. 14), the drug core containing latanoprost in a crosslinked MED6385 silicone, prepared as described in Example 2 ("control") without an excipient exhibits significant in-homogeneity of dispersion of the latanoprost. Discrete liquid droplets of latanoprost are observed within the filled sheath following extrusion of the silicone/latanoprost mixture into the polyimide sheath. At comparable latanoprost loadings in the same matrix, the presence of excipients DMPC or EPG is surprisingly found to result in maintenance of a substantially uniform or homogeneous dispersion of the latanoprost droplets within the matrix, such that macroscopic droplets are not visible, as seen in FIG. 13.

In various embodiments, the excipient can be adapted to enhance corneal penetration of the therapeutic agent, or can be adapted to enhance retention of the therapeutic agent on the surface of the eye or within the tissue of the eye. For example, the excipient can act as a penetration enhancer of a drug, such as latanoprost, into corneal tissue, where it can exert its anti-glaucoma effect. Alternatively or in addition, the excipient can delay washout of the agent from the surface of the eye by tear fluid, such as by increasing adsorption of the drug onto the surface of the cornea.

In various embodiments, a drug core of an implant of the invention can comprise a phospholipid. The phospholipid can be a negatively charged phospholipid, for example, the phospholipid can be egg phosphatidylglycerol (EPG). EPG is a negatively charged lipid in that the molecule contains an anionic phosphate group, but no cationic group. As is shown in FIGS. 9 and 10, EPG has been unexpectedly found to increase a rate of release of latanoprost from a silicone matrix (Formulation 2) into an aqueous medium such as tear fluid compared to a rate of release of the latanoprost in the absence of an excipient.

In various embodiments, the phospholipid can comprise a zwitterionic phospholipid containing fatty acyl moieties of 16 carbon atoms or less. For example, the phospholipid excipient can be dimyristoylphosphatidylcholine (DMPC). DMPC is a zwitterionic phospholipid in that the molecule includes a negatively charge phosphate oxygen atom and a positively charged choline (trimethylammoniumethanol) moiety. Formulation 1 (Example 1) contains DMPC, and as can be seen from FIGS. 9 and 10, DMPC increases a release rate of latanoprost from a silicone matrix into an aqueous medium such as tear fluid compared to a rate of release of the latanoprost in the absence of an excipient.

In various embodiments, the excipient can be a polyhydric alcohol, such as glycerol. FIG. 9 shows a release rate of latanoprost from a crosslinked silicone matrix in the presence of 10% glycerol. It is apparent that the rate is increased relative to control.

In various embodiments, the excipient can comprise a polyethyleneglycol, such as PEG-400. FIG. 9 shows a release rate of latanoprost from a crosslinked silicone matrix in the presence of 5% PEG400. It is apparent that the rate

is increased relative to control.

In various embodiments, the invention provides a method for preparing an ocular implant of the invention wherein the matrix polymer is crosslinked silicone, the therapeutic agent is latanoprost, and the excipient comprises a phospholipid, a polyhydric alcohol, or a polyethyleneglycol, or any combination thereof, the method comprising:

combining with mixing a silicone part A, the latanoprost, and the excipient, then
adding with mixing a silicone part B and the crosslinker, then,
extruding under pressure, for example, at sub-ambient temperature, the mixture into a tube, the tube comprising an impermeable material, then
curing the mixture in the tube, then
cutting the cured, filled tube into sections, each section being a drug core for an implant.

In various embodiments, the crosslinker can be tetraethylorthosilicate.

In various embodiments, the phospholipid is a phosphatidylglycerol or is dimyristoyl phosphatidylcholine.

In various embodiments, the polyhydric alcohol is glycerol.

In various embodiments, the polyethyleneglycol is PEG400.

In various embodiments, for a drug core of an implant prepared by a method of the invention, the release rate is a release rate into tear fluid of the therapeutic agent from an implant disposed in a punctum of a patient and the release rate is increased relative to a release rate in the absence of the excipient. For example, the release rate can be increased over a period of time of about 1 to about 10 days following disposition of the ocular implant within the punctum.

In various embodiments, for a drug core of an implant prepared by a method of the invention, the therapeutic agent is present at a higher loading or concentration within the matrix of the drug core in the presence of the excipient than could be achieved with a comparably homogeneous dispersion in a comparable matrix in the absence of an excipient.

In various embodiments, for a drug core of an implant prepared by a method of the invention, the inclusions of the therapeutic agent in the drug core are present in the matrix, and the inclusions are of a more uniform size and are more uniformly dispersed in the matrix relative to a size and dispersion of inclusions within a matrix in a comparable drug core with a comparable loading of the agent in the absence of the excipient.

In various embodiments, for a drug core of an implant prepared by a method of the invention, the excipient is adapted to enhance corneal penetration of the therapeutic agent, or is adapted to enhance retention of the therapeutic agent on the surface of the eye or within the tissue of the eye.

In various embodiments, for a drug core of an implant prepared by a method of the invention, the amount of the therapeutic agent in a volumetric portion of the matrix varies from the amount of the therapeutic agent in any other equal volumetric portion of the matrix by no greater than about 30%, or by no greater than about 20%, or by no greater than about 10%, or by no greater than about 5%.

The above Detailed Description includes references to the accompanying drawings, which form a part of the Detailed Description. The drawings show, by way of illustration, specific embodiments in which the invention can be practiced. These embodiments are also referred to herein as "examples."

Concentrations, amounts, etc. of various components of this invention are often presented in a range format throughout this patent document. The description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as about 42 micrograms to about 44 micrograms of latanoprost should be considered to have specifically disclosed subranges such as about 42 micrograms to about 43 micrograms, about 43 micrograms to about 44 micrograms, etc., as well as individual numbers within that ranges, such as 42 micrograms, 43 micrograms, and 44 micrograms. This construction applies regardless of the breadth of the range and in all contexts throughout this patent document.

The above description is intended to be illustrative, and not restrictive. For example, the above-described examples (or one or more features thereof) can be used in combination with each other. Other embodiments can be used, such as by one of ordinary skill in the art upon reviewing the above description. Also, in the above Detailed Description, various features can be grouped together to streamline the disclosure. This should not be interpreted as intending that an unclaimed disclosed feature is essential to any claim. Rather, inventive subject matter can lie in less than all features of a particular disclosed embodiment. Thus, the following claims are hereby incorporated into the Detailed Description, with each claim standing on its own as a separate embodiment. The scope of the invention should be determined with reference to the appended claims.

Abbreviations used throughout include:

DMPC dimyristoyl phosphatidylcholine

DMPE dimyristoyl phosphatidylethanolamine

DMPG dimyristoyl phosphatidylglycerol

DOTAP 1 ,2-dipalmitoyl-3 -trimethylammonium-propane

DODAP 1 ,2-dipalmitoyl-3 -dimethylammonium-propane

DPPC dipalmitoyl phosphatidylcholine

DPPE dipalmitoyl phosphatidylethanolamine

DSPC distearoyl phosphatidylcholine

EPG egg phosphatidylglycerol

PEG polyethyleneglycol

POPG palmitoyl oleoyl phosphatidylglycerol

PSPC palmitoyl stearoyl phosphatidylcholine

The invention can be further described by the following, non-limiting examples.

## Example 1

An open-label, Phase 2 study of a Latanoprost Punctal Plug Delivery System (L-PPDS) lacrimal implant containing a drug core comprising 44 micrograms latanoprost is conducted in subjects with Ocular Hypertension (OH) or Open-Angle Glaucoma (OAG). After an appropriate washout period from previous treatment (or no washout if treatment naive), approximately 40 subjects are fitted with the L-PPDS and followed for safety and efficacy for 12 weeks.

After treatment with the L-PPDS, subjects are given Xalatan® (latanoprost ophthalmic solution, 0.005%) for a 4 week run-out period to confirm a response to topical prostaglandin treatment.

Introduction:

The L-PPDS formulation in this Example includes 44 μg of latanoprost and uses a proprietary, punctal plug design that has been designed to have improved retention characteristics. This is an open-label study to gather preliminary safety and efficacy data on the 44 μg strength L-PPDS composed of the new punctal plug design.

The total amount of latanoprost in the L-PPDS for this study (44 μg) is equivalent to the amount in 29 drops of Xalatan®, and is intended to be delivered over several months.

Study Procedures and Assessments:

The trial and treatment punctal plugs are composed of medical grade silicone, polyimide, and cyanoacrylate medical grade adhesive.

At the start of the study, each subject has the L-PPDS inserted bilaterally into the lower puncta and inspected thereafter at each visit. The L-PPDS is removed at the Week 12 visit. Following completion of L-PPDS treatment, subjects are dispensed Xalatan® and instructed to instill 1 drop into each eye in the evening. Xalatan® (latanoprost ophthalmic solution, 0.005%) is supplied as a 2.5 mL solution. Xalatan® is a commercially available product. Ingredients of Xalatan® include benzalkonium chloride (preservative), sodium chloride, sodium dihydrogen phosphate monohydrate, disodium hydrogen phosphate anhydrous, and water.

IOP has a well-characterized diurnal pattern, peaking in the early morning hours (Liu et al. 1999); therefore, the time points for IOP measurement in this study are fixed. In addition, diurnal IOP is monitored at selected visits in this study to gather information on the effects of the sustained release L-PPDS on the IOP pattern. The primary and secondary IOP variables are summarized using means, standard deviations, minimums, medians, maximums, and 95% CIs. In addition, frequency distributions of some IOP variables may also be prepared.

The relationship between the IOP change from baseline and other variables, such as TBUT, tear volume, or other demographic characteristics, is evaluated. The difference in the IOP change from baseline between treatment

groups is summarized using means, standard deviations, minimums, maximums, and 95% CI.

Four Week Preliminary Results:

Sixty patients diagnosed with OAG/OH with a mean age of 65 years (30 to 90 years) were enrolled. The mean baseline IOP was 24.5 $\pm$ 2.4 mmHg for the patient population. Of the 60 patients enrolled, 47 (78%) completed Week-4 follow-up, had an IOP measurement at Week 4, and retained the L-PPDS in both eyes through Week 4. In this interim data analysis, 13 patients (22%) discontinued L-PPDS treatment early: 11 patients (18%) due to loss of L-PPDS and 2 patients (3%) due to inadequate IOP control.

Preliminary four week interim results from the ongoing Phase II clinical trial of the 44 microgram Latanoprost Punctal Plug Delivery System (L-PPDS) show that the mean change in intraocular pressure (IOP) from baseline was -3.52 $\pm$2.77 mmHg (range of -8.9 to 3.5, excluding two subjects who lost or had L-PPDS removed from both eyes) at the Week 4 visit. 36% of patients had an IOP decrease from baseline greater than or equal to 5 mmHg.

The L-PPDS were well-tolerated over the testing period. Based on four week preliminary data, the overall adverse events range from 1.7% to 11.7%. The most common adverse events are eye itching (commonly seen with initial punctal plug wear and usually a part of adaptation) and eye irritation (11.7% and 8.3%, respectively). Increased lacrimation (tear production) and ocular discomfort are reported in 6.7% and 1.7% of patients with the 44-$\mu$g L-PPDS. Superficial punctate keratitis was reported in one patient (1.7%). No conjunctival or ocular hyperemia was observed. Week 4 patient-reported comfort and tearing scores for the 44 microgram L-PPDS are as follows: 88% of patients rated L-PPDS comfort as 'no awareness' or 'mild awareness,' while 76% of patients rated tearing as 'none.'

**Table 2**

| **Ocular Adverse Events** | **% of Subjects (N=60)** |
|---|---|
| Lacrimation increased | 4 (6.7) |
| Eye pruritus (itching)** | 7 (11.7) |
| Ocular discomfort** | 1 (1.7) |
| Eye irritation | 5 (8.3) |
| Conjunctival hyperemia | 0 |
| Conjunctival hemorrhage | 2 (3.3) |
| Vision blurred | 2 (3.3) |
| Device migration | 0 |
| Foreign body sensation | 0 |
| Granuloma | 0 |
| Superficial punctate keratitis | 1 (1.7) |
| All others | <2 |
| **Seen typically with initial punctal plug wear and generally a part of adaptation | |

**Table 3**

| **Week 4** | **Percentage of Eyes (%) (N=120 eyes)** |
|---|---|
| **Comfort:** | |
| No awareness | 66 |
| Mild awareness | 22 |
| Moderate awareness | 8 |
| Mild discomfort | 1 |
| Moderate discomfort | - |
| Severe discomfort | - |

(continued)

| Week 4 | Percentage of Eyes (%) (N=120 eyes) |
|---|---|
| **Comfort:** | |
| Not done | 3 |
| **Tearing:** | |
| None | 76 |
| Occasional | 16 |
| Mild | 4 |
| Moderate | - |
| Severe | - |
| Not done | 3 |

**Example 2**

An open-label, randomized, parallel-group study is conducted in approximately 40 subjects with ocular hypertension (OH) or open angle glaucoma (OAG). After an appropriate washout period from previous treatment (no washout if treatment naïve), subjects are enrolled and randomized (1:1) to study treatment with either a 44 microgram Latanoprost Punctal Plug Delivery System (L-PPDS) or a 44 microgram Latanoprost Punctal Plug Delivery System plus Artificial Tears containing Benzalkonium Chloride (L-PPDS + AT-BAK). Subjects are followed for 6 weeks to monitor safety and efficacy.

After treatment with the L-PPDS, subjects are given Xalatan® (latanoprost ophthalmic solution, 0.005%) for a 4 week run-out period in order to confirm a response to topical prostaglandin treatment.

Introduction:

The L-PPDS formulation in this example includes 44μg of latanoprost and uses a proprietary, punctal plug design that has been designed to have improved retention characteristics. This is an open-label study to gather preliminary safety and efficacy data on the 44 μg strength L-PPDS composed of the new punctal plug design.

The total amount of latanoprost in the L-PPDS for this study (44 μg) is equivalent to the amount in 29 drops of Xalatan®, and is intended to be delivered over several months.

An additional purpose of this study is to explore the effects of concomitant administration of artificial tears containing BAK (AT-BAK), for example, on the IOP response to the L-PPDS.

Study Procedures and Assessments:

Each subject will have the L-PPDS inserted bilaterally into the lower punta. An artificial tear solution that contains the preservative BAK is dispensed to subjects who are randomized to receive HypoTears™ (preserved with 0.01% BAK; Novartis Ophthalmics) as adjunctive treatment to the L-PPDS. Subjects are instructed to instill 2 drops, at least 5 minutes apart, into each eye two times per day: every morning and every evening. Subjects may not use any other medications to lower IOP during the study or other lubricants or artificial tears. Other products containing benzalkonium chloride are prohibited.

The primary IOP efficacy variable is IOP change from baseline. The secondary IOP efficacy variables is IOP and percentage IOP change from baseline.

Six Week Preliminary Results:

Sixty-five subjects were screened for enrollment, and out of these, 40 subjects were randomized to study treatment with either the L-PPDS alone (n=20) or L-PPDS + AT-BAK (n=20).

In the L-PPDS alone treatment group, 11 subjects completed the study treatment period and 9 subjects discontinued treatment prematurely, due to the following reasons: loss of the L PPDS from both eyes (5 subjects), inadequate IOP control (2 subjects), and other reasons (2 subjects). The disposition of subjects in the L-PPDS + AT-BAK treatment group was similar with 11 subjects completing the study treatment period and 9 subjects discontinuing treatment prematurely. The reasons for premature treatment discontinuation in the L-PPDS + AT-BAK group were: loss of the L PPD from both eyes (7 subjects), inadequate IOP control (1 subject), and other (1 subject). Table 4 presents the preliminary IOP results of the study (ITT).

**TABLE 4. Preliminary Mean IOP Change From Baseline and Percentage IOP Change From Baseline (ITT)**

| Visit | L-PPDS Alone | L-PPDS + AT-BAK | TOTAL |
|---|---|---|---|
| **Baseline** | | | |
| n | 19 | 20 | 39 |
| Mean IOP (mmHg) | 25.44 ± 2.78 | 24.14 ± 2.34 | 24.78 ± 2.62 |
| **Week 1** | | | |
| n | 19 | 20 | 39 |
| Mean IOP (mmHg) | 22.36 ± 4.15 | 19.59 ± 3.17 | 20.94 ± 3.89 |
| Mean change from baseline (mmHg) | -3.09 ± 2.85 | -4.56 ± 2.82 | -3.84 ± 2.89 |
| Percentage change from baseline (%) | -12.35 ± 10.80 | -18.80 ± 11.62 | -15.66 ± 11.55 |
| **Week 2** | | | |
| n | 18 | 17 | 35 |
| Mean IOP (mmHg) | 21.17 ± 3.77 | 20.00 ± 3.63 | 20.60 ± 3.69 |
| Mean change from baseline (mmHg) | -3.96 ± 2.34 | -4.28 ± 2.44 | -4.11 ± 2.36 |
| Percentage change from baseline (%) | -16.02 ± 9.29 | -17.87 ± 9.99 | -16.92 ± 9.54 |
| **Week 3** | | | |
| n | 16 | 15 | 31 |
| Mean IOP (mmHg) | 22.40 ± 4.08 | 19.45 ± 2.74 | 20.98 ± 3.75 |
| Mean change from baseline (mmHg) | -2.83 ± 3.06 | -4.76 ± 1.93 | -3.76 ± 2.72 |
| Percentage change from baseline (%) | -11.47 ± 12.12 | -19.60 ± 7.98 | -15.41 ± 10.97 |
| **Week 4** | | | |
| n | 14 | 13 | 27 |
| Mean IOP (mmHg) | 21.79 ± 3.48 | 20.04 ± 2.21 | 20.94 ± 3.02 |
| Mean change from baseline (mmHg) | -3.09 ± 2.08 | -4.36 ± 2.30 | -3.70 ± 2.24 |
| Percentage change from baseline (%) | -12.84 ± 8.47 | -17.46 ± 8.87 | -15.06 ± 8.82 |
| **Week 6** | | | |
| n | 11 | 11 | 22 |
| Mean IOP (mmHg) | 21.79 ± 2.09 | 19.90 ± 1.89 | 20.85 ±2.17 |
| Mean change from baseline (mmHg) | -3.34 ± 1.52 | -4.40 ± 2.20 | -3.87 ± 1.92 |
| Percentage change from baseline (%) | -13.31 ± 5.26 | -17.82 ± 7.48 | -15.57 ± 6.72 |

Preliminary analysis of the data indicated that mean IOP decreased from baseline in both treatment groups throughout the study. There was an approximately 3 to 4 mmHg decrease from baseline observed in the L-PPDS Alone treatment group and an approximately 4 to 5 mmHg decrease in the L-PPDS + AT-BAK group. This equated to percentage decreases from baseline of approximately 12% to 16% in the L-PPDS Alone group and a 17% to 19% decrease in the L-PPDS + AT-BAK treatment group.

From the preliminary analysis, the most frequent adverse events reported in this study were local adverse events that are already known to be associated with topical ophthalmic application of latanoprost, such as conjunctival hyperemia, and eye pruritis (10% incidence for each event). There were no systemic safety concerns noted.

Preliminary results indicated there was a higher incidence of eye pruritis in subjects treated concomitantly with the L PPDS and AT-BAK (15% of subjects and 7 events vs 5% of subjects and 1 event). Eye pruritis was not considered associated to treatment in the subject treated with the L-PPDS alone.

**Example 3**

A partially masked study is conducted in approximately 10 evaluable subjects with ocular hypertension (OH) or open angle glaucoma (OAG). After an appropriate washout period from previous treatment (or no washout if treatment naïve), subjects are fitted in both eyes with Latanoprost Punctal Plug Delivery System (L-PPDS) in both the upper and lower puncta. In each subject, the right eye receives a total latanoprost dose of 44 μg (L-PPDS with latanoprost dose of 44 μg in lower punctum and punctal plug with no drug core in the upper punctum), and the left eye receives a total latanoprost dose of 65 μg (L-PPDS with latanoprost dose of 44 μg in lower punctum and L-PPDS with latanoprost dose of 21 μg in upper punctum). Subjects are followed for safety and IOP response for 6 weeks.

L-PPDS and punctal plugs with no drug core are not replaced during the study. Subjects remain on study as long as L-PPDS and/or punctal plugs are retained in both upper and lower puncta of one eye. After treatment with the L-PPDS, subjects are given Xalatan® (latanoprost ophthalmic solution, 0.005%) in order to confirm a response to topical prostaglandin treatment.

<u>Introduction:</u>

This Example is a partially masked study to gather preliminary IOP response data on a higher latanoprost dose of 65 μg.

The purpose of this study is to evaluate L-PPDS with 65 μg latanoprost (equivalent to the amount in about 43 drops of Xalatan®). The study includes 6 weeks of follow-up, so the amount of latanoprost in the L-PPDS is only slightly lower than that in the number of Xalatan® drops a subject would receive over the same time period. The study uses L-PPDS in both puncta (44 μg latanoprost in the lower punctum and 21 μg latanoprost in the upper punctum) to achieve the 65 μg latanoprost dose. This study also compares the 65 μg latanoprost dose with the 44 μg latanoprost dose, while both puncta are occluded to mitigate any effect of double punctal occlusion on IOP response.

<u>Study Procedures and Assessments:</u>

This is a partially masked study, in that IOP is measured by an independent evaluator. Such masking is planned to reduce bias in the comparison between the different latanoprost doses given. Otherwise, the study is open-label (i.e., the principal investigator and subjects know the treatments given).

IOP variables are analyzed by latanoprost dose, as well as the difference between the 2 eyes within the same subject. For analyses using the intent to treat (ITT) data set, all data from all subjects with at least 1 follow-up IOP measurement is included. For analyses using the evaluable (EVAL) data set, if at any time a L-PPDS is lost (spontaneously extruded or migrated out of place), the IOP data from that eye is excluded starting at the time of first loss. If the L-PPDS is removed by the Investigator, the IOP data from that eye is excluded starting at the visit after removal.

The primary and secondary IOP variables are summarized using means, standard deviations, minimums, medians, maximums, and 95% CIs. In addition, frequency distributions of some IOP variables may also be prepared.

**Example 4**

An open-label study of a Latanoprost Punctal Plug Delivery System (L-PPDS) containing 81 micrograms latanoprost is conducted in subjects with Ocular Hypertension (OH) or Open-Angle Glaucoma (OAG). After an appropriate washout period from previous treatment (or no washout if treatment naïve), approximately 40 subjects are fitted with the L-PPDS and followed for safety and efficacy for 12 weeks.

After treatment with the L-PPDS, subjects are given Xalatan® (latanoprost ophthalmic solution, 0.005%) for a 4 week run-out period to confirm a response to topical prostaglandin treatment.

<u>Introduction:</u>

The L-PPDS formulation in this Example includes 81 μg of latanoprost and uses a proprietary, punctal plug design that has been designed to have improved retention characteristics. This is an open-label study to gather safety and efficacy data on the 81 μg strength L-PPDS composed of the new punctal plug design.

The total amount of latanoprost in the L-PPDS for this study (81 μg) is equivalent to the amount in 54 drops of Xalatan®, and is intended to be delivered over several months.

<u>Study Procedures and Assessments:</u>

The trial and treatment punctal plugs are composed of medical grade silicone, polyimide, and cyanoacrylate

medical grade adhesive.

At the start of the study, each subject has the L-PPDS inserted bilaterally into the lower puncta and inspected thereafter at each visit. The L-PPDS is removed at the Week 12 visit. Following completion of L-PPDS treatment, subjects are dispensed Xalatan® and instructed to instill 1 drop into each eye in the evening. Xalatan® (latanoprost ophthalmic solution, 0.005%) is supplied as a 2.5 mL solution. Xalatan® is a commercially available product. Ingredients of Xalatan® include benzalkonium chloride (preservative), sodium chloride, sodium dihydrogen phosphate monohydrate, disodium hydrogen phosphate anhydrous, and water.

If extrusion occurs during the study, a subject may have one L-PPDS replaced per eye. If a subject loses two L-PPDS from the same eye, the eye will remain untreated and the subject followed according to schedule until Week 6 (or loss of the second L-PPDS in the contralateral eye). At the discretion of the medical professional, the L-PPDS may be removed from the contralateral eye and the Xalatan® run-out period can begin immediately.

IOP has a well-characterized diurnal pattern, peaking in the early morning hours (Liu et al. 1999); therefore, the time points for IOP measurement in this study are fixed. In addition, diurnal IOP is monitored at selected visits in this study to gather information on the effects of the sustained release L-PPDS on the IOP pattern. The primary and secondary IOP variables are summarized using means, standard deviations, minimums, medians, maximums, and 95% CIs. In addition, frequency distributions of some IOP variables may also be prepared.

The relationship between the IOP change from baseline and other variables, such as safety, TBUT, tear volume, or other demographic characteristics, is evaluated. The difference in the IOP change from baseline between treatment groups is summarized using means, standard deviations, minimums, maximums, and 95% CI.

## Example 5

Manufacture of Latanoprost/Silicone Mixture:

The silicone formulation (MED6385) is a two part system. Part A contains the silicone and crosslinker while Part B contains the tin catalyst to promote crosslinking. The two parts are combined in a final ratio of 200:1 (Part A:Part B). The required amounts of Latanoprost, the selected excipient (DMPC or EPG), the crosslinker, MED6385 Part A and B are weighed onto a glass slide and mixed for approximately 2 minutes using a plastic mini spatula. Quantities used in the preparation of specific examples are shown below.

Preparation of syringe extrusion system:

Sections of tubing are threaded through a plastic luer adaptor and glued in place using a cyanoacrylate adhesive. A 1mL syringe is modified by cutting the tip of the plunger flush. The previously assembled tubing/adaptor piece is inserted into the syringe barrel and threaded through the luer outlet and fitted in place.

Extrusion into Polyimide Tubing:

After the silicone/latanoprost mixing is complete, the mixture is loaded in the barrel of the syringe extrusion system. The plunger is inserted and excess air is removed. The syringe is then loaded into the extrusion apparatus.

After setup, the extrusion system activated and a silicone latanoprost mixture is extruded down the length of the polyimide tubing. Once the mixture reaches the bottom of the tubing, the tubing is cut using a razor blade and clamped on both ends.

Curing:

The clamped section of tubing is placed in a humidity chamber to be cured.

Release Profiles:

Samples, including those shown in FIGS. 9-12, were analyzed for elution by immersion in a PBS (phosphate buffered saline) elution medium. Temperature was maintained at 37o C with 100 cpm shaking. The elution medium was changed daily (except weekends) for the first two weeks and weekly thereafter. Elution media samples were analyzed by reversed-phase chromatography. Samples were prepared for HPLC analysis by adding 200 μL of internal standard solution (2.4pg/mL of butylated hydroxyanisole in isopropanol) and vortexing to mix. The amount of latanoprost eluted was determined using a reversed phase HPLC UV detection using a Waters SunFire C18, 3.5μm, 3.0 x 100mm column and detection conditions of the following: 1.0 mL/min flow rate, 200 μL injection volume, 210 nm detection wavelength, using gradient of solvents over a run time of 6 minutes (time zero = 63% mobile phase A, 37% mobile phase B, time

4.9 minutes = 35%A, 65%B, time 5 minutes = 63%A, 37%B) comprising 0.1% Phosphoric Acid in Water (mobile Phase A) and ACN (mobile phase B), 50°C column temperature set point.

L-PDDS Formulation 1 of Latanoprost 1 Silicone:

| Component | Ratio w/w API/composition | Wt% of component | API/implant (μg) |
|---|---|---|---|
| latanoprost | 1.000 | 39.1 | 95 |
| Silicone MED 6385 part A | 1.31 | 51.4 | 124.7 |
| Silicone MED 6385 part B | 0.01 | 0.4 | 0.9 |
| Crosslinker (TEOS) | 0.05 | 1.8 | 4.4 |
| DMPC | 0.19 | 7.3 | 17.8 |
| * density 1.29**density 0.916 | | | |

L-PDDS Formulation 2 of Latanoprost 1 Silicone:

| Component | Ratio w/w API/composition | Wt% of component | API/implant (μg) |
|---|---|---|---|
| latanoprost | 1.000 | 39.1 | 95 |
| Silicone MED 6385 part A | 1.31 | 51.4 | 124.7 |
| Silicone MED 6385 part B * | 0.01 | 0.4 | 0.9 |
| Crosslinker (TEOS) ** | 0.05 | 1.8 | 4.4 |
| EPG | 0.19 | 7.3 | 17.8 |
| * density 1.29**density 0.916 | | | |

FIG. 10 shows a release profile over time for these two formulations with respect to a formulation lacking an excipient.

**Example 6 - Comparison of Drug Loading in Presence or Absence of Excipients:**

The Silicone Part B, crosslinker, API and excipients (DMPC or EPG) are weighed onto a glass slide. The materials are mixed for 2 - 5 minutes until completely mixed. Then add Silicone Part A and mix for 2 minutes. The mixture is the extruded into the polyimide tubing at approximately 5°C. The extrusion is then cured.

Results are shown in FIG. 13.

Control:

The components (Silicone Part A and B, crosslinker and API) are weighed onto a glass slide. The materials are mixed for 2 minutes and then extruded into the polyimide tubing at approximately 5°C. The extrusion is then cured.

Results are shown in FIG. 14.

**Example 7 - Open-Label, Phase 2 Study of Formulations 1 and 2 of the Latanoprost Punctal Plug Delivery System:**

An open-label, Phase 2 study of a Latanoprost Punctal Plug Delivery System (L-PPDS) lacrimal implant containing a drug core including 95 micrograms (μg) of latanoprost is conducted in subjects with Ocular Hypertension (OH) or Open-Angle Glaucoma (OAG). The L-PPDS used is one of two formulations-Formulation 1 or Formulation 2. After an appropriate washout period from a previous treatment (or no washout if treatment naive), approximately 30 subjects are fitted with the L-PPDS, Formulation 1 and another approximately 30 subjects are fitted with the L-PPDS, Formulation 2 (approximately 60 subjects in total).

The subjects are followed for safety and efficacy for 6 weeks. Safety is monitored with one or more of intraocular pressure (IOP), Snellen best-corrected visual acuity (BCVA), biomicropscopy, subject tearing and comfort assessments, automated perimetry, and Funduscopy. Tear characteristics are evaluated with Schirmer's and tear break-up time (TBUT) tests throughout the study. Ocular surface evaluations are done using lissamine green staining. Additionally, photographs are taken at baseline to document the placement of the L-PPDSs.

After treatment with the L-PPDS, subjects are given Xalatan® (latanoprost ophthalmic solution, 0.005%) for a 4 week run-out period to confirm a response to topical prostaglandin treatment.

Introduction:

The L-PPDS formulations in this Example include 95 μg of latanoprost and use a proprietary, punctal plug design that has been designed to have improved retention characteristics. This is an open-label study to gather preliminary safety and efficacy data on the 95 μg strength L-PPDS composed of the new punctal plug design and Formulations 1 and 2.

The total amount of latanoprost in the L-PPDS for this study (95 μg) is equivalent to the amount in about 62-63 drops of Xalatan®, and is intended to be delivered over several months.

Study Procedures and Assessments:

The trial and treatment punctal plugs are composed of medical grade silicone, polyimide, and cyanoacrylate medical grade adhesive.

At the start of the study, each subject has the L-PPDS inserted bilaterally into the lower puncta and inspected thereafter at each visit. If an L-PPDS is spontaneously extruded, up to 1 replacement L-PPDS per subject is allowed. The L-PPDS is removed at the Week 6 visit.

Subjects receive study treatment at Day 0 and have follow-up visits at Weeks 1, 2, 3, 4, and 6.

The primary efficacy variable is the change from baseline in IOP measurements.

**Bibliography**

Allen RC. Medical management of glaucoma. In: Albert DM, Jakobiec FA, eds. Principles and Practice of Ophthalmology. Philadelphia, PA: WB Saunders and Co.; 1994:1569-1588.

AlphaMed lacrimal implant [product label]. Wauwatosa, WI: AlphaMed.

American Academy of Ophthalmology. Primary Open-Angle Glaucoma, Preferred Practice Pattern. San Francisco: American Academy of Ophthalmology, 2005. Available at: http://www.aao.org/ppp. Accessed October 22, 2008.

Anderson DR, Chauhan B, Johnson C, Katz J, Patella VM, Drance SM. Criteria for progression of glaucoma in clinical management and in outcome studies. Am J Ophthalmol 2000; 130(6):827-829.

Anderson DR. Collaborative normal tension glaucoma study. Curr Opin Ophthalmol. 2003;14(2):86-90.

Bailey IL, Lovie JE. New design principles for visual acuity letter charts. Am J Optom Physiol Opt 1976 Nov; 53(11):740-745.

Balaram M, Schaumberg DA, Dana MR. Efficacy and tolerability outcomes after punctal occlusion with silicone plugs in dry eye syndrome. Am J Ophthalmol. 2001;131(1):30-36.

Baudouin C, Rouland JF, Nordmann JP, Bron A, Pelen F. Efficacy of first- or second-line latanoprost on intraocular pressure and ocular symptoms in subjects with open-angle glaucoma or ocular hypertension [in French]. J Fr Ophtalmol. 2006;29(6):615-624.

Bengtsson B, Heijl A. A long-term prospective study of risk factors for glaucomatous visual field loss in subjects with ocular hypertension. J Glaucoma. 2005;14(2):135-138.

Brown MM, Brown GC, Spaeth GL. Improper topical self-administration of ocular medication among subjects with glaucoma. Can J Ophthalmol. 1984:1;2-5.

Callender M, Morrison PE. A quantitative study of human tear proteins before and after adaption to non-flexible contact lenses. Am J Optom Physiol Opt. 1974;51(12):939-945.

Coleman AL. Glaucoma. Lancet 1999;354:1803-10.

Dasgupta S, Oates V, Bookhart BK, Vaziri B, Schwartz GF, Mozaffari E. Population-based persistency rates for topical glaucoma medications measured with pharmacy claims data. Am J Manag Care. 2002;8(suppl 10):S255-S261.

Diestelhorst M, Schaefer CP, Beusterien KM, et al. Persistency and clinical outcomes associated with latanoprost and beta-blocker monotherapy: evidence from a European retrospective cohort study. Eur J Ophthalmol. 2003;13(suppl 4):S21-S29.

Fiscella RG, Geller JL, Gryz LL, Wilensky J, Viana M. Cost considerations of medical therapy for glaucoma. Am J Ophthalmol. 1999;128(4):426-433.

Fremont AM, Lee PP, Mangione CM, et al. Patterns of care for open-angle glaucoma in managed care. Arch Ophthalmol. 2003;121(6):777-783.

Fukano Y, Asada H, Kimura A, Kawazu K. Influence of benzalkonium chloride on the penetration of latanoprost into rabbit aqueous humor after ocular instillations. AAPS Journal. 2006;8(S2). http://www.aapsj.org/abstracts/AM_2006/AAPS2006-001397.pdf. Accessed October 22, 2008

Gordon MO, Beiser JA, Brandt JD, et al. The Ocular Hypertension Treatment Study: baseline factors that predict the onset of primary open-angle glaucoma. Arch Ophthalmol. 2002;120(6):714-720; discussion 829-830.

Gordon MO, Kass MA. The Ocular Hypertension Treatment Study: design and baseline description of the participants. Arch Ophthalmol 1999; 117(5):573-583.

Goskonda VR, Hill RA, Khan MA, Reddy IK. Permeability of chemical delivery systems across rabbit corneal (SIRC) cell line and isolated corneas: a comparative study. Pharm Dev Technol. 2000;5(3):409-416.

Gurwitz JH, Glynn RJ, Monane M, et al. Treatment for glaucoma: adherence by the elderly. Am J Public Health. 1993;83(5):711-716.

Heijl A, Leske MC, Bengtsson B, Hyman L, Bengtsson B, Hussein M, for the Early Manifest Glaucoma Trial Group. Reduction of intraocular pressure and glaucoma progression: results from the Early Manifest Glaucoma Trial. Arch Ophthalmol. 2002;120(10):1268-1279.

Higginbotham EJ, Gordon MO, Beiser JA, et al. The Ocular Hypertension Treatment Study: topical medication delays or prevents primary open-angle glaucoma in African American individuals. Arch Ophthalmol. 2004;122(6):813-820.

Horwath-Winter J, Thaci A, Gruber A, Boldin I. Long-term retention rates and complications of silicone punctal plugs in dry eye. Am J Ophthalmol. 2007;144(3):441-444.

HypoTears® Safety Information. Novartis Ophthalmics, East Hanover, NJ. http://www.miochol.org/hcp/products/hypotears-hcp-si.jsp. Accessed: October 22, 2008

Javitt JC, Metrick S, Wang F: Costs of glaucoma in the United States. Invest Ophthalmol Vis Sci 1995; 36:S429.

Kass MA, Gordon MO, Kymes SM. Incorporating the results of the Ocular Hypertension Treatment Study into clinical practice. Arch Ophthalmol. 2005;123(7):1021-1022.

Kass MA, Heuer DK, Higginbotham EJ, et al. The Ocular Hypertension Treatment Study: a randomized trial determines that topical ocular hypotensive medication delays or prevents the onset of primary open-angle glaucoma. Arch Ophthalmol 2002;120(6):701-713.

Kim BM, Osmanovic SS, Edward DP. Pyogenic granulomas after silicone punctal plugs: a clinical and histopathologic study. Am J Ophthalmol. 2005;139(4):678-684.

Kobelt-Nguyen G, Gerdtham UG, Alm A: Costs of treating primary open-angle glaucoma and ocular hypertension: a retrospective, observational two-year chart review of newly diagnosed subjects in Sweden and the United States. J Glaucoma 1998; 7:95.

Leibowitz HM, Krueger DE, Maunder LR, et al. The Framingham Eye Study monograph: An ophthalmological and epidemiological study of cataract, glaucoma, diabetic retinopathy, macular degeneration, and visual acuity in a general population of 2631 adults, 1973-1975. Surv Ophthalmol. 1980;24(Suppl):335-610.

Leonard R. Statistics on Vision Impairment: A Resource Manual (5th Edition). New York: Arlene R. Gordon Research Institute of Lighthouse International; 2002.

Leske MC, Heijl A, Hyman L, Bengtsson B, Komaroff E. Factors for progression and glaucoma treatment: the Early Manifest Glaucoma Trial. Curr Opin Ophthalmol. 2004; 15(2): 102-106.

Leung EW, Medeiros FA, Weinreb RN. Prevalence of ocular surface disease in glaucoma subjects. J Glaucoma. 2008;17(5):350-355.

Lichter PR, Musch DC, Gillespie BW, et al, and the CIGTS Study Group. Interim clinical outcomes in the Collaborative Initial Glaucoma Treatment Study comparing initial treatment randomized to medications or surgery. Ophthalmology. 2001;108(11):1943-1953.

Liu JHK, Kripke DF, Twa MD, et al. Twenty-four-hour pattern of intraocular pressure in the aging population. Invest Ophthalmol Vis Sci. 1999:40(12):2912-2917.

Maier PC, Funk J, Schwarzer G, Antes G, Falck-Ytter YT. Treatment of ocular hypertension and open angle glaucoma: meta-analysis of randomised controlled trials. BMJ. 2005;331(7509):134. Epub 2005 Jul 1.

Mindel JS. Pharmacokinetics. In: Tasman W, Jaeger EA, eds. Duane's Foundations of Clinical Ophthalmology. Vol. 3: Lippincott Williams & Wilkins, 1995:1-17.

Musch DC, Lichter PR, Guire KE, Standardi CL, The CIGTS Study Group. The Collaborative Initial Glaucoma Treatment Study Design, Methods, and Baseline Characteristics of Enrolled Subjects. Ophthalmology 1999; 106:653-662.

Nakamura T, Yamada M, Teshima M, et al. Electrophysiological characterization of tight junctional pathway of rabbit

cornea treated with ophthalmic ingredients. Biol Pharm Bull. 2007;30(12):2360-2364.
Nordstrom BL, Friedman DS, Mozaffari E, Quigley HA, Walker AM. Persistence and adherence with topical glaucoma therapy. Am J Ophthalmol. 2005;140(4):598-606.
Norell SE, Granström PA. Self-medication with pilocarpine among outsubjects in a glaucoma clinic. Br J Ophthalmol. 1980 Feb;64(2):137-41.
O'Donoghue EP, and the UK and Ireland Latanoprost Study Group. A comparison of latanoprost and dorzolamide in subjects with glaucoma and ocular hypertension: a 3 month, randomised study. Br J Ophthalmol. 2000;84:579-582.
Osterberg L, Blaschke T. Adherence to Medication. N Engl J Med. 2005:353(5):487-497.
Parrish RK, Palmberg P, Sheu WP, XLT Study Group. A comparison of latanoprost, bimatoprost, and travoprost in subjects with elevated intraocular pressure: a 12-week, randomized, masked-evaluator multicenter study. Am J Ophthalmol. 2003;135(5):688-703.
Patel SS, Spencer CM. Latanoprost. A review of its pharmacological properties, clinical efficacy and tolerability in the management of primary open-angle glaucoma and ocular hypertension. Drugs Aging. 1996;9(5):363-378.
Pawar PK, Majumdar DK. Effect of formulation factors on in vitro permeation of moxifloxacin from aqueous drops through excised goat, sheep, and buffalo corneas. AAPS PharmSciTech. 2006;7(1):E13.
Preferred practice pattern - Primary Open-Angle Glaucoma. American Academy of Ophthalmology. (Limited Revision). 2005.
Quigley HA: The number of persons with glaucoma worldwide. Br J Ophthalmol 1996; 80:389.
Reardon G, Schwartz GF, Mozaffari E. Subject persistency with pharmacotherapy in the management of glaucoma. Eur J Ophthalmol. 2003;13(suppl 4):S44-S52.
Regnier, et al. Ocular Effects of Topical 0.03% Latanoprost Solution in Normotensive Feline Eyes. Vet Ophthalmol 2006; 9(1):39-43.
Rossetti L, Gandolfi S, Traverse C, et al. An evaluation of the rate of nonresponders to latanoprost therapy. J Glaucoma. 2006:15(3):238-243.
Rouland JF, Berdeaux G, Lafuma A. The economic burden of glaucoma and ocular hypertension: implications for subject management: a review. Drugs Aging 2005;22(4):315-321.
Sakamoto A, Kitagawa K, Tatami A. Efficacy and retention rate of two types of silicone punctal plugs in subjects with and without Sjögren's Syndrome. Cornea. 2004;23(3):249-254.
Scholz M, Lin JE, Lee VH, Keipert S. Pilocarpine permeability across ocular tissues and cell cultures: influence of formulation parameters. J Ocul Pharmacol Ther. 2002;18(5):455-468.
Schwartz GF, Reardon G, Mozaffari E. Persistency with latanoprost or timolol in primary open angle glaucoma suspects. Am J Ophthalmol. 2004;137(suppl 1):S13-S16.
Shaya FT, Mullins CD, Wong W, Cho J. Discontinuation rates of topical glaucoma medications in a managed care population. Am J Manag Care. 2002;8(suppl 10):S271-S277.
Silliman NP. Xalatan Ophthalmic Solution 0.005% (50 mg/mL) (Pharmacia) 06/06/1996 Approval: Statistical Review. 1995.
Spooner JJ, Bullano MF, Ikeda LI, et al. Rates of discontinuation and change of glaucoma therapy in a managed care setting. Am J Manag Care. 2002;8(suppl 10):S262-S270.
The AGIS Investigators. The Advanced Glaucoma Intervention Study (AGIS): 7. The relationship between control of intraocular pressure and visual field deterioration. Am J Ophthalmol. 2000;130(4):429-440.
Thomas JV. Primary open angle glaucoma. In: Albert DM, Jakobiec FA, eds. Principles and Practice of Ophthalmology. Philadelphia, PA: WB Saunders and Co.; 1994:1342-1345.
Thylefors B, Negrel A-D, Pararajasegaram R, Dadzie KY: Global data on blindness. Bull World Health Org 1995; 73:115-121.
Touitou, Elka, Barry, Brian W. Enhancement in Drug Delivery. Taylor & Francis Group; 2007: 527-548.
Urquhart J. The odds of the three nons when an aptly prescribed medicine isn't working: non-compliance, non-absorption, non-response. Br J Clin Pharmacol. 2002;54(2):212-220.
Waldock A, Snape J, Graham CM. Effects of glaucoma medications on the cardiorespiratory and intraocular pressure status of newly diagnosed glaucoma subjects. Br J Ophthalmol. 2000;84(7):710-713.
Whitcup SM, et al. A randomized, doube masked, multicenter clinical trial comparing latanoprost and timolol for the treatment of glaucoma and acular hypertension. Br J Ophthalmol 2003; 87:57-62.
Winfield AJ, et al. A study of the causes of non-compliance by subjects prescribed eyedrops. Br J Ophthalmol. 1990 Aug;74(8):477-80.
Woodward DF, et al. Pharmalogical Characterization of a Novel Antiglaucoma Agent, (AGN 192024). J Pharmacology and Experimental Therapeutics 2003; 305(2):772-785.
Xalatan® (latanoprost ophthalmic solution) (50 μg/mL) Product Monograph. Pfizer Canada Inc.; 2003. http://www.pfizer.ca/english/our%20products/prescription%20pharmaceuticals/default.asp?s=1 &id=18&doc=enmonograph. Accessed October 22, 2008.

Xalatan® (latanoprost ophthalmic solution) (50 μg/mL) Product Monograph. Pfizer Canada Inc.; 2003. http://www.pfizer.ca/english/our%20products/prescription%20pharmaceuticals/default.asp?s=1 &id=18&doc=enmonograph. Accessed October 22, 2008.
Xalatan® (latanoprost ophthalmic solution) 0.005% (50 μg/mL) prescribing information. Division of Pfizer Inc. New York, NY: Pharmacia & Upjohn Company; 2007. http://www.xalatan.com/consumer/prescribinginfo.asp. Accessed October 1, 2007.

**Claims**

1. An implant (200) configured for disposition within or adjacent to a body cavity, tissue, duct, or fluid, the implant comprising:

   a drug core (214) comprising:

   (a) a matrix (254, 362, 454) including a polymer;
   (b) a therapeutic agent (252, 360, 452) contained within the matrix, and
   (c) an excipient dissolved or dispersed within the matrix, the excipient configured to any of,

   (1) modify a release rate of the therapeutic agent into the body cavity, tissue, duct, or fluid relative to a comparable release rate in the absence of an excipient;
   (2) increase a loading of the therapeutic agent substantially uniformly dissolved or dispersed within the matrix, relative to a comparable loading of the therapeutic agent that is substantially uniformly dissolved or dispersed, in the absence of an excipient;
   (3) increase retention of the agent at or adjacent to a site of release in a living body or increase penetration of adjacent body tissue by the agent, or both, relative to the retention or penetration or both from a comparable implant in the absence of the excipient;

   or any combination thereof,

   wherein an amount of the therapeutic agent in a volumetric portion of the matrix is similar to an amount of the therapeutic agent in any other equal volumetric portion of the matrix; **characterized in that** the polymer of the matrix is a non-biodegradable polymer; and
   wherein the implant is an ocular implant, adapted for disposition through a punctum and into a canaliculus of a human eye for release of the therapeutic agent therefrom.

2. The implant (200) of claim 1, wherein the therapeutic agent is substantially uniformly and homogeneously dissolved in the matrix or the agent at least partially forms solid or liquid inclusions, the inclusions having an average diameter less than about 50 microns, the inclusions being substantially uniformly dispersed throughout the matrix on a sub-millimeter scale.

3. The implant (200) of any one of claims 1-2, wherein the matrix comprises a silicone, optionally crosslinked, or a polyurethane polymer.

4. The implant (200) of any one of claims 1-2, comprising a sheath (256, 366, 456) partially surrounding the drug core (214), at least a portion of the sheath intermediate a drug core surface and a wall of the implant body lumen.

5. The implant (200) of claim 1, wherein the therapeutic agent (252, 360, 452) contained in the drug core (214) is a prostaglandin analogue, for example, latanoprost, adapted for treatment of glaucoma.

6. The implant (200) of claim 2, wherein a release rate into tear fluid of the therapeutic agent (252, 360, 452) from the implant when disposed through the punctum and into the canaliculus of the eye is increased relative to a release rate in the absence of the excipient.

7. The implant (200) of claim 1, wherein the therapeutic agent (252, 360, 452) is present at a higher loading or concentration within the matrix (254, 362, 454) of the drug core (214) in the presence of the excipient than could be achieved with a comparably homogeneous dispersion in a comparable matrix in the absence of an excipient.

8. The implant (200) of claim 7, wherein inclusions of the therapeutic agent (252, 360, 452) in the drug core (214) are present in the matrix (254, 362, 454), and the inclusions are of a more uniform size and are more uniformly dispersed in the matrix relative to a size and dispersion of inclusions within a matrix in a comparable drug core with a comparable loading of the agent in the absence of the excipient.

9. The implant (200) of claim 1, wherein the excipient is adapted to enhance corneal penetration of the therapeutic agent.

10. The implant (200) of claim 1, wherein the excipient is adapted to enhance retention of the therapeutic agent on the surface of the eye or within the tissue of the eye.

11. The implant (200) of claim 1, wherein the amount of the therapeutic agent (252, 360, 452) in a volumetric portion of the matrix (254, 362, 454) varies from the amount of the therapeutic agent in any other equal volumetric portion of the matrix by no greater than about 30%.

12. The implant (200) of claim 1, wherein the drug core (214) comprises about 0.1 wt% to about 50 wt% of the agent.

13. The implant (200) of claim 4, wherein the sheath (256, 366, 456) comprises a polymer comprising at least one of polyimide, PMMA, or PET, wherein the polymer is extruded or cast; or a metal comprising stainless steel or titanium.

14. The implant (200) of claim 1, further comprising a second excipient, an inert filler material, a salt, a surfactant, a dispersant, a second polymer, an oligomer, or a combination thereof.

15. The implant (200) of claim 1, further comprising an implant body (202) adapted to receive the drug core (214) therewithin for placement within the body cavity, tissue, duct, or fluid.

## Patentansprüche

1. Implantat (200), das zur Anordnung innerhalb oder benachbart zu einer Körperhöhle, einem Gewebe, einem Gang oder einem Fluid konfiguriert ist, wobei das Implantat Folgendes umfasst:

   einen Arzneimittelkern (214), der Folgendes umfasst:

   (a) eine Matrix (254, 362, 454), die ein Polymer enthält;
   (b) ein in der Matrix enthaltenes therapeutisches Mittel (252, 360, 452) und
   (c) einen Hilfsstoff, der in der Matrix gelöst oder dispergiert ist, wobei der Hilfsstoff zu einem der Folgenden konfiguriert ist:

   (1) Modifizieren einer Freisetzungsrate des therapeutischen Mittels in die Körperhöhle, das Gewebe, den Gang oder das Fluid relativ zu einer vergleichbaren Freisetzungsrate in Abwesenheit eines Hilfsstoffs;
   (2) Erhöhen einer Ladung des therapeutischen Mittels, das im Wesentlichen gleichmäßig in der Matrix gelöst oder dispergiert ist, relativ zu einer vergleichbaren Ladung des therapeutischen Mittels, das im Wesentlichen gleichmäßig gelöst oder dispergiert ist, in Abwesenheit eines Hilfsstoffs;
   (3) Erhöhen der Retention des Mittels an oder benachbart zu einer Freisetzungsstelle in einem lebenden Körper oder Erhöhen der Penetration benachbarten Körpergewebes durch das Mittel oder beides relativ zu der Retention oder Penetration oder beidem von einem vergleichbaren Implantat in Abwesenheit des Hilfsstoffs;

   oder eine beliebige Kombination davon,
   wobei eine Menge des therapeutischen Mittels in einem volumetrischen Teil der Matrix ähnlich einer Menge des therapeutischen Mittels in jedem anderen gleichen volumetrischen Teil der Matrix ist; **dadurch gekennzeichnet, dass** das Polymer der Matrix ein nicht biologisch abbaubares Polymer ist; und
   wobei das Implantat ein Augenimplantat ist, das zur Anordnung durch einen Punctum und in einen Canaliculus eines menschlichen Auges zur Freisetzung des therapeutischen Mittels daraus geeignet ist.

2. Implantat (200) nach Anspruch 1, wobei das therapeutische Mittel im Wesentlichen gleichmäßig und homogen in der Matrix gelöst ist oder das Mittel mindestens teilweise feste oder flüssige Einschlüsse bildet, wobei die Einschlüsse

einen durchschnittlichen Durchmesser von weniger als etwa 50 Mikrometer aufweisen, wobei die Einschlüsse im Wesentlichen gleichmäßig in der gesamten Matrix im Submillimeterbereich dispergiert sind.

3. Implantat (200) nach einem der Ansprüche 1-2, wobei die Matrix ein Silikon, optional vernetzt, oder ein Polyurethan-Polymer umfasst.

4. Implantat (200) nach einem der Ansprüche 1-2, umfassend eine Hülle (256, 366, 456), die den Arzneimittelkern (214) teilweise umgibt, wobei mindestens ein Teil der Hülle zwischen einer Arzneimittelkernoberfläche und einer Wand des Implantatkörperlumens liegt.

5. Implantat (200) nach Anspruch 1, wobei das therapeutische Mittel (252, 360, 452), das in dem Arzneimittelkern (214) enthalten ist, ein Prostaglandin-Analogon, beispielsweise Latanoprost, ist, das zur Behandlung von Glaukom geeignet ist.

6. Implantat (200) nach Anspruch 2, wobei eine Freisetzungsrate des therapeutischen Mittels (252, 360, 452) aus dem Implantat in die Tränenflüssigkeit, wenn es durch das Punctum und in den Canaliculus des Auges angeordnet ist, relativ zu einer Freisetzungsrate in Abwesenheit des Hilfsstoffs erhöht ist.

7. Implantat (200) nach Anspruch 1, wobei das therapeutische Mittel (252, 360, 452) in Gegenwart des Hilfsstoffs in einer höheren Ladung oder Konzentration innerhalb der Matrix (254, 362, 454) des Arzneimittelkerns (214) vorliegt, als mit einer vergleichbar homogenen Dispersion in einer vergleichbaren Matrix in Abwesenheit eines Hilfsstoffs erreicht werden könnte.

8. Implantat (200) nach Anspruch 7, wobei Einschlüsse des therapeutischen Mittels (252, 360, 452) in dem Arzneimittelkern (214) in der Matrix (254, 362, 454) vorliegen und die Einschlüsse von einer gleichmäßigeren Größe sind und gleichmäßiger in der Matrix dispergiert sind relativ zu einer Größe und Dispersion von Einschlüssen innerhalb einer Matrix in einem vergleichbaren Arzneimittelkern mit einer vergleichbaren Ladung des Mittels in Abwesenheit des Hilfsstoffs.

9. Implantat (200) nach Anspruch 1, wobei der Hilfsstoff geeignet ist, die Hornhautpenetration des therapeutischen Mittels zu verbessern.

10. Implantat (200) nach Anspruch 1, wobei der Hilfsstoff dazu geeignet ist, die Retention des therapeutischen Mittels auf der Oberfläche des Auges oder innerhalb des Gewebes des Auges zu verbessern.

11. Implantat (200) nach Anspruch 1, wobei die Menge des therapeutischen Mittels (252, 360, 452) in einem volumetrischen Teil der Matrix (254, 362, 454) von der Menge des therapeutischen Mittels in jedem anderen gleichen volumetrischen Teil der Matrix um nicht mehr als etwa 30 % variiert.

12. Implantat (200) nach Anspruch 1, wobei der Arzneimittelkern (214) etwa 0,1 Gew.-% bis etwa 50 Gew.-% des Mittels umfasst.

13. Implantat (200) nach Anspruch 4, wobei die Hülle (256, 366, 456) ein Polymer umfasst, das mindestens eines von Polyimid, PMMA oder PET umfasst, wobei das Polymer extrudiert oder gegossen ist; oder ein Metall, das Edelstahl oder Titan umfasst.

14. Implantat (200) nach Anspruch 1, ferner einen zweiten Hilfsstoff, ein inertes Füllmaterial, ein Salz, ein Tensid, ein Dispergiermittel, ein zweites Polymer, ein Oligomer oder eine Kombination davon umfassend.

15. Implantat (200) nach Anspruch 1, ferner einen Implantatkörper (202) umfassend, der dazu geeignet ist, den Arzneimittelkern (214) darin zur Platzierung innerhalb der Körperhöhle, des Gewebes, des Gangs oder des Fluids aufzunehmen.

## Revendications

1. Implant (200) conçu pour être disposé à l’intérieur ou à proximité d’une cavité corporelle, d’un tissu, d’un conduit ou d’un fluide, l’implant comprenant :

un noyau de médicament (214) comprenant :

(a) une matrice (254, 362, 454) comprenant un polymère ;
(b) un agent thérapeutique (252, 360, 452) contenu dans la matrice, et
(c) un excipient dissous ou dispersé dans la matrice, l'excipient étant conçu pour une quelconque opération parmi

(1) la modification de la vitesse de libération de l'agent thérapeutique dans la cavité corporelle, le tissu, le conduit ou le fluide par rapport à une vitesse de libération comparable en l'absence d'un excipient ;
(2) l'augmentation de la charge de l'agent thérapeutique sensiblement uniformément dissous ou dispersé dans la matrice, par rapport à une charge comparable de l'agent thérapeutique qui est sensiblement uniformément dissous ou dispersé, en l'absence d'un excipient ;
(3) l'augmentation de la rétention de l'agent au niveau ou à proximité d'un site de libération dans un corps vivant ou l'augmentation de la pénétration du tissu corporel adjacent par l'agent, ou les deux, par rapport à la rétention ou à la pénétration ou aux deux à partir d'un implant comparable en l'absence de l'excipient ;

ou toute combinaison de celles-ci,

la quantité de l'agent thérapeutique dans une partie volumétrique de la matrice étant similaire à la quantité de l'agent thérapeutique dans toute autre partie volumétrique égale de la matrice ; **caractérisé en ce que** le polymère de la matrice est un polymère non biodégradable ; et
ledit implant étant un implant oculaire, adapté pour être disposé à travers un point lacrymal et dans un canalicule d'un oeil humain pour en libérer l'agent thérapeutique.

2. Implant (200) selon la revendication 1, ledit agent thérapeutique étant dissous de manière sensiblement uniforme et homogène dans la matrice ou ledit agent formant au moins partiellement des inclusions solides ou liquides, lesdites inclusions comportant un diamètre moyen inférieur à environ 50 microns, lesdites inclusions étant sensiblement uniformément dispersées dans toute la matrice à une échelle inférieure au millimètre.

3. Implant (200) selon l'une quelconque des revendications 1 à 2, ladite matrice comprenant une silicone, éventuellement réticulée, ou un polymère polyuréthane.

4. Implant (200) selon l'une quelconque des revendications 1 à 2, comprenant une gaine (256, 366, 456) entourant partiellement le noyau de médicament (214), au moins une partie de la gaine se trouvant entre une surface du noyau de médicament et une paroi de la lumière du corps de l'implant.

5. Implant (200) selon la revendication 1, ledit agent thérapeutique (252, 360, 452) contenu dans le noyau de médicament (214) étant un analogue de prostaglandine, par exemple le latanoprost, adapté au traitement d'un glaucome.

6. Implant (200) selon la revendication 2, la vitesse de libération dans le liquide lacrymal de l'agent thérapeutique (252, 360, 452) à partir de l'implant lorsqu'il est disposé à travers le point lacrymal et dans le canalicule de l'oeil étant augmentée par rapport à la vitesse de libération en l'absence de l'excipient.

7. Implant (200) selon la revendication 1, ledit agent thérapeutique (252, 360, 452) étant présent à une charge ou à une concentration plus élevée dans la matrice (254, 362, 454) du noyau de médicament (214) en présence de l'excipient que celle qui pourrait être obtenue avec une dispersion comparablement homogène dans une matrice comparable en l'absence d'un excipient.

8. Implant (200) selon la revendication 7, lesdites inclusions de l'agent thérapeutique (252, 360, 452) dans le noyau de médicament (214) étant présentes dans la matrice (254, 362, 454), et lesdites inclusions étant d'une taille plus uniforme et étant plus uniformément dispersés dans la matrice par rapport à une taille et à une dispersion des inclusions au sein d'une matrice dans un noyau de médicament comparable avec une charge comparable de l'agent en l'absence de l'excipient.

9. Implant (200) selon la revendication 1, ledit excipient étant adapté à l'amélioration de la pénétration cornéenne de l'agent thérapeutique.

**10.** Implant (200) selon la revendication 1, ledit excipient étant adapté à l'amélioration de la rétention de l'agent thérapeutique à la surface de l'oeil ou dans le tissu de l'oeil.

**11.** Implant (200) selon la revendication 1, ladite quantité de l'agent thérapeutique (252, 360, 452) dans une partie volumétrique de la matrice (254, 362, 454) variant de la quantité de l'agent thérapeutique dans toute autre partie volumétrique égale de la matrice de pas plus d'environ 30 %.

**12.** Implant (200) selon la revendication 1, ledit noyau de médicament (214) comprenant environ 0,1 % en poids à environ 50 % en poids de l'agent.

**13.** Implant (200) selon la revendication 4, ladite gaine (256, 366, 456) comprenant un polymère comprenant au moins un composant parmi le polyimide, le PMMA ou le PET, ledit polymère étant extrudé ou coulé ; ou un métal comprenant de l'acier inoxydable ou du titane.

**14.** Implant (200) selon la revendication 1, comprenant en outre un second excipient, un matériau de charge inerte, un sel, un tensioactif, un dispersant, un second polymère, un oligomère ou une combinaison de ceux-ci.

**15.** Implant (200) selon la revendication 1, comprenant en outre un corps d'implant (202) adapté à la réception du noyau de médicament (214) en son sein pour le placement dans la cavité corporelle, le tissu, le conduit ou le fluide.

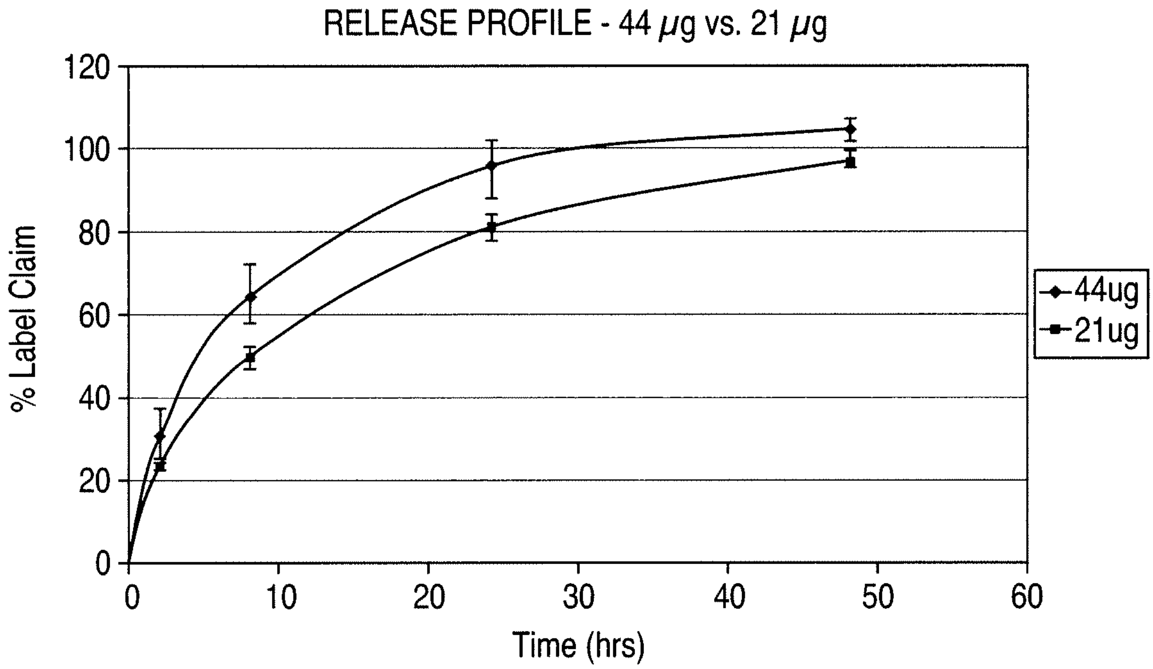
RELEASE PROFILE - 44 μg vs. 21 μg
120
100
80
60
40
20
0
% Label Claim
0
10
20
30
40
50
60
Time (hrs)
44ug
21ug

FIG. 1

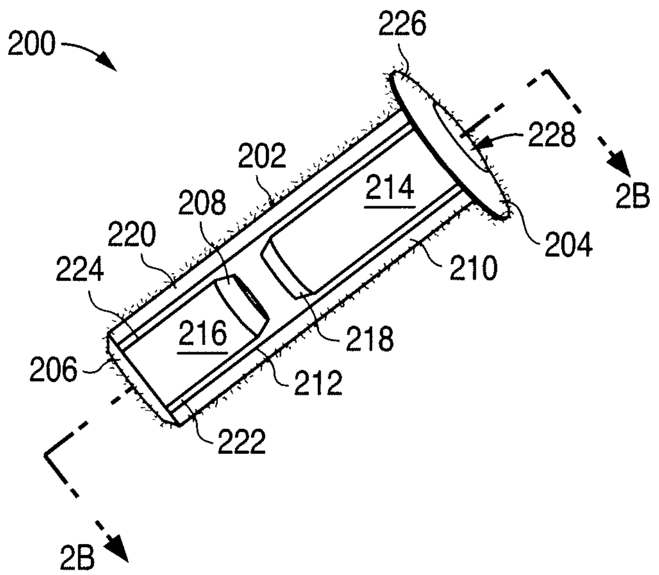
200
226
228
202
214
2B
208
220
224
204
210
216
218
206
212
222
2B

FIG. 2A

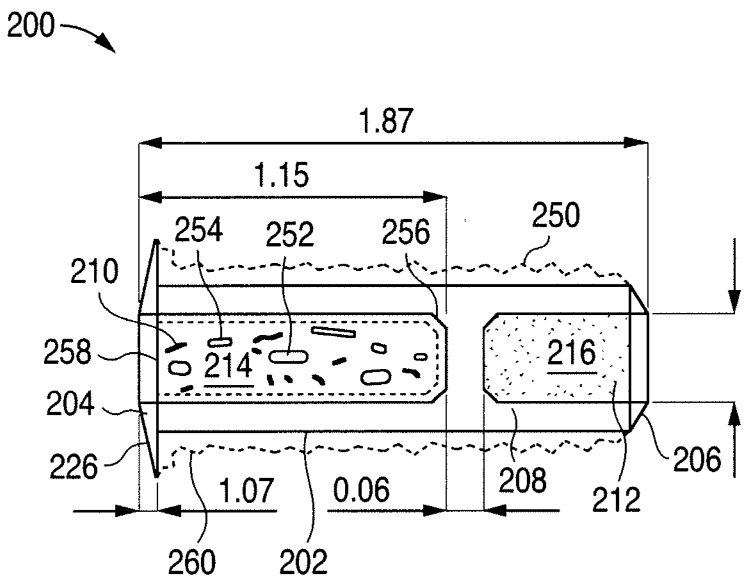
200
1.87
1.15
254
252
256
250
210
258
214
216
204
226
206
1.07
0.06
208
212
260
202

FIG. 2B

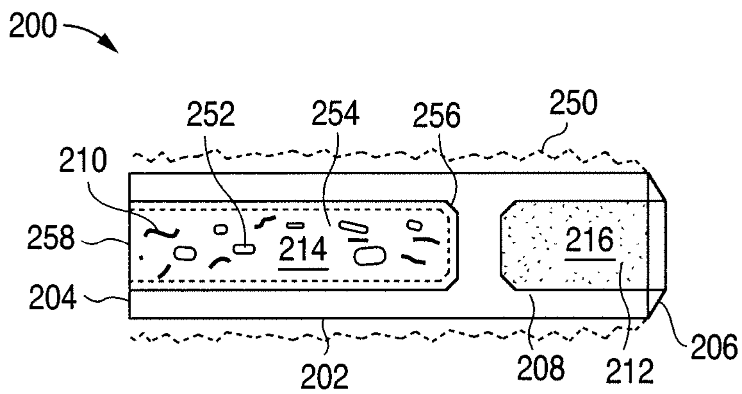
200
252
254
256
250
210
258
214
216
204
202
208
212
206

FIG. 2C

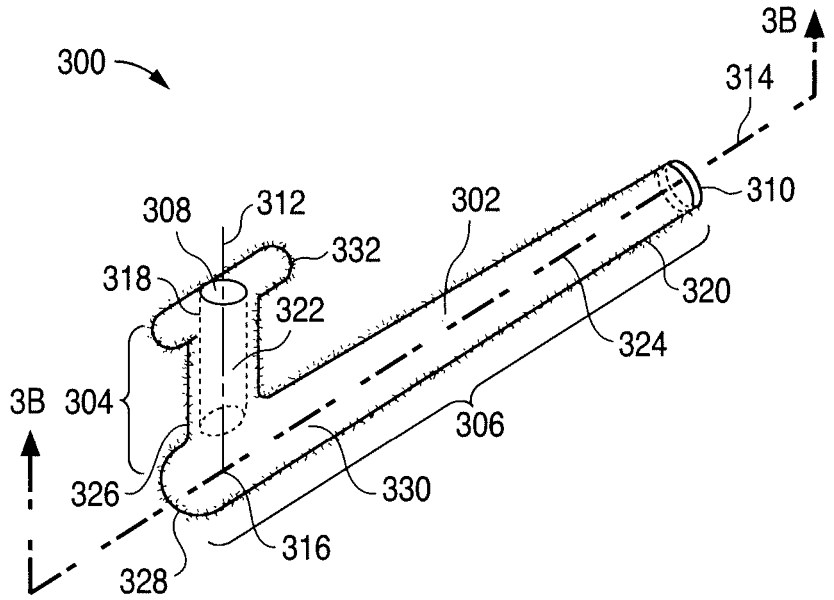

300
3B
314
310
302
312
308
332
318
320
322
324
304
306
326
330
316
328


FIG. 3A

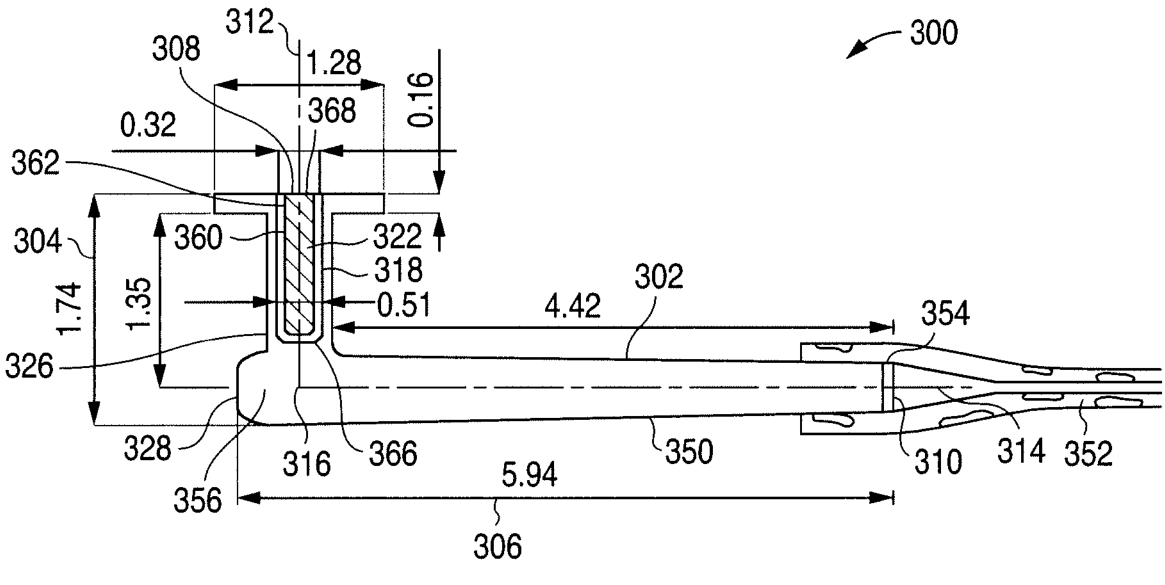

312
300
308
1.28
0.16
368
0.32
362
304
360
322
318
1.74
1.35
0.51
302
4.42
354
326
328
316
366
350
310
314
352
356
5.94
306


FIG. 3B

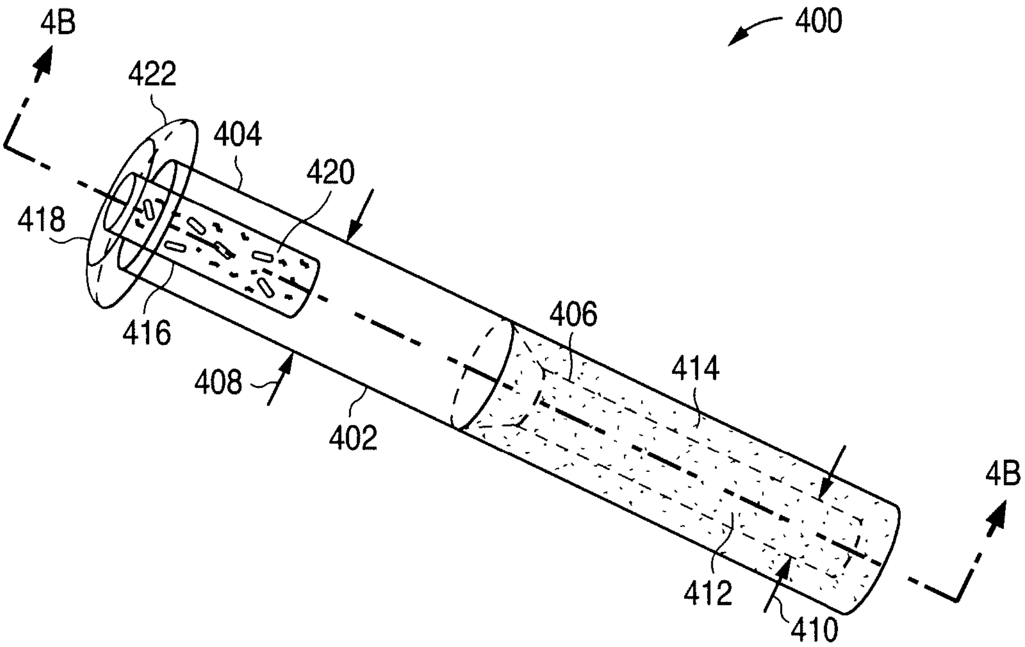


FIG. 4A

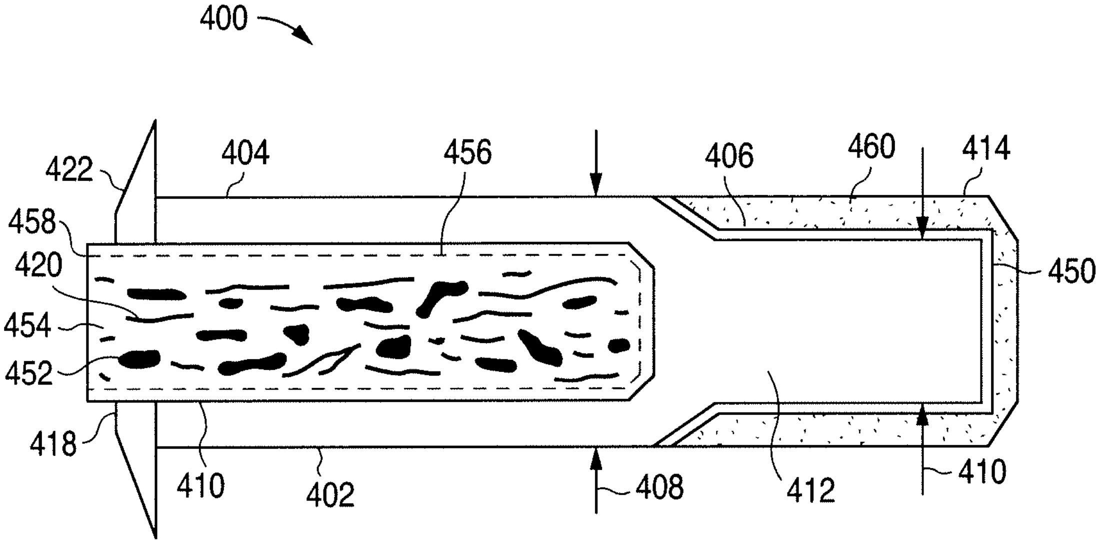
400
422
404
456
406
460
414
458
420
450
454
452
418
410
402
408
412
410

FIG. 4B

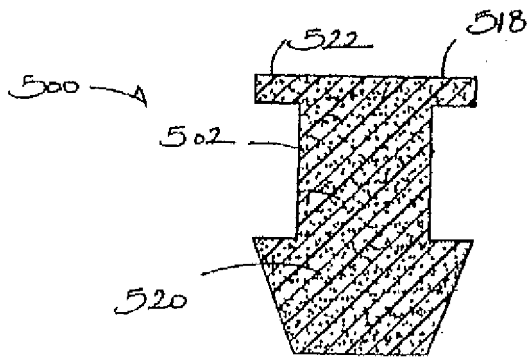


FIG. 5

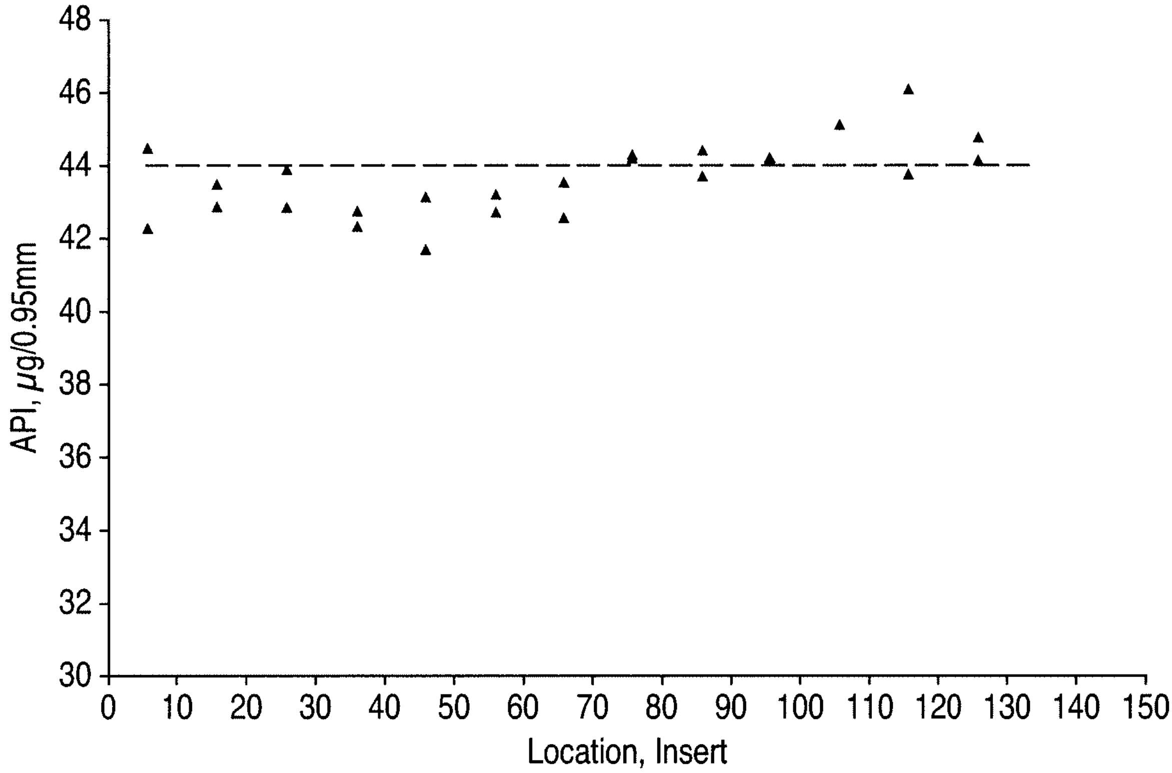
48
46
44
42
40
38
36
34
32
30
API, μg/0.95mm
0
10
20
30
40
50
60
70
80
90
100
110
120
130
140
150
Location, Insert

FIG. 6

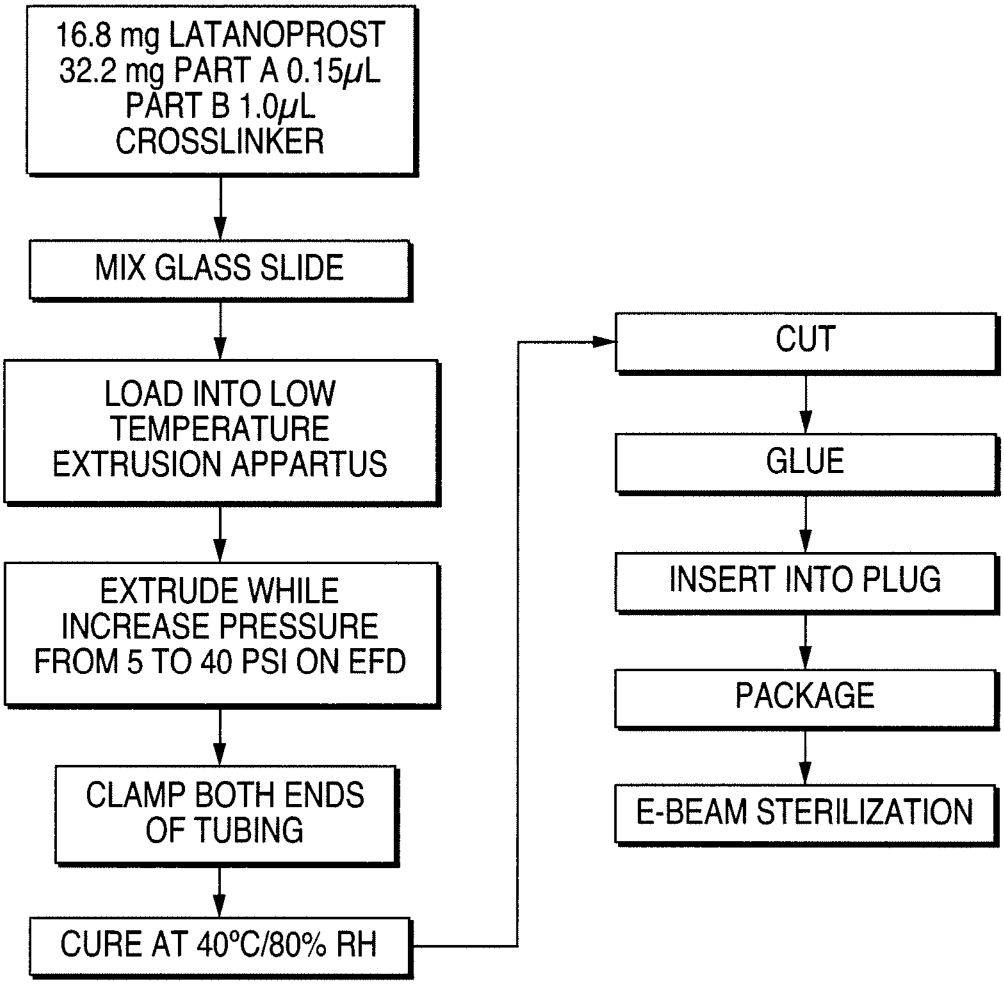
16.8 mg LATANOPROST
32.2 mg PART A 0.15μL
PART B 1.0μL
CROSSLINKER
MIX GLASS SLIDE
LOAD INTO LOW
TEMPERATURE
EXTRUSION APPARTUS
EXTRUDE WHILE
INCREASE PRESSURE
FROM 5 TO 40 PSI ON EFD
CLAMP BOTH ENDS
OF TUBING
CURE AT 40°C/80% RH
CUT
GLUE
INSERT INTO PLUG
PACKAGE
E-BEAM STERILIZATION

FIG. 7

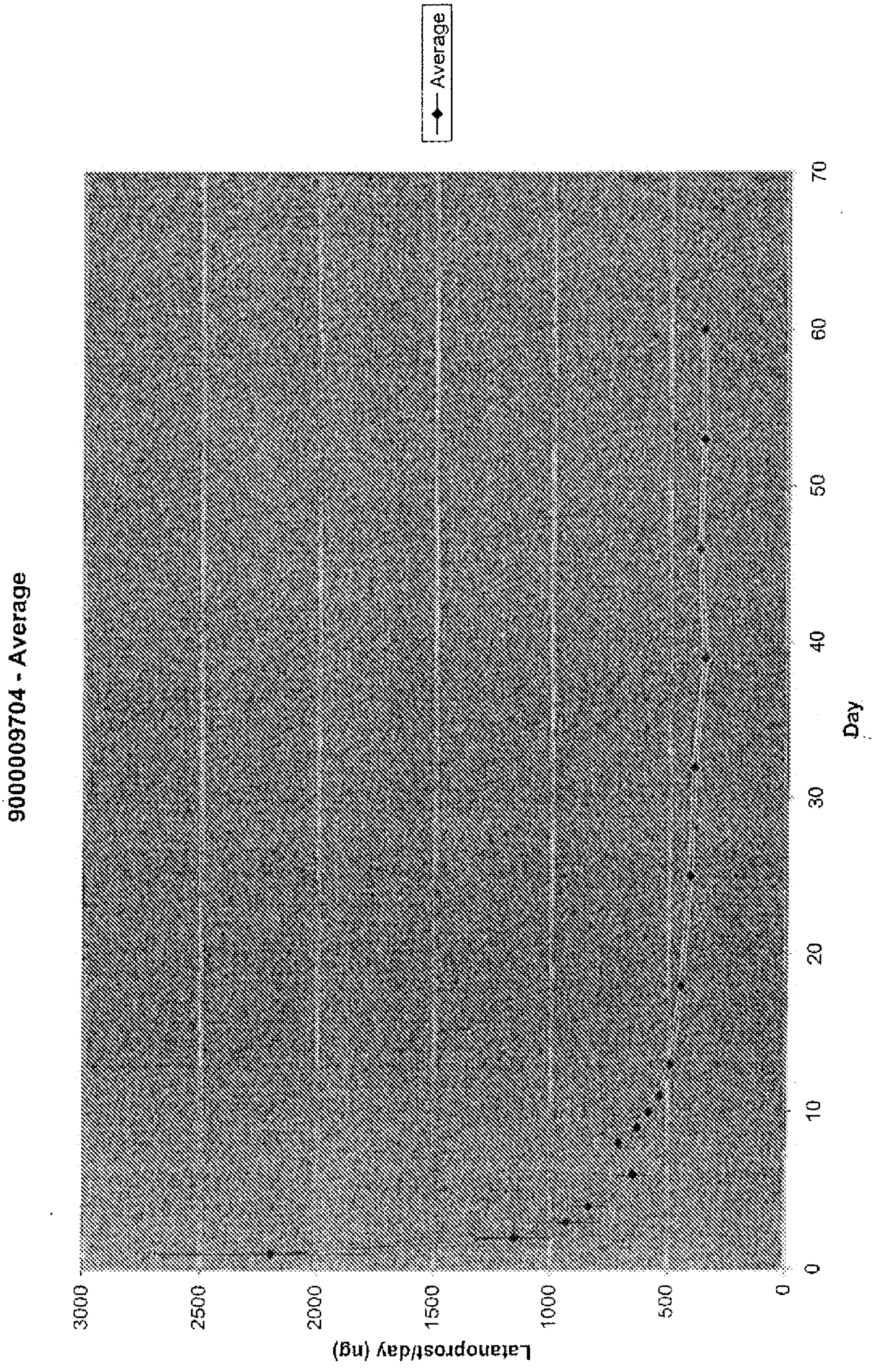
9000009704 - Average
Average
Latanoprost/day (ng)
3000
2500
2000
1500
1000
500
0
0
10
20
30
40
50
60
70
Day

FIG. 8A

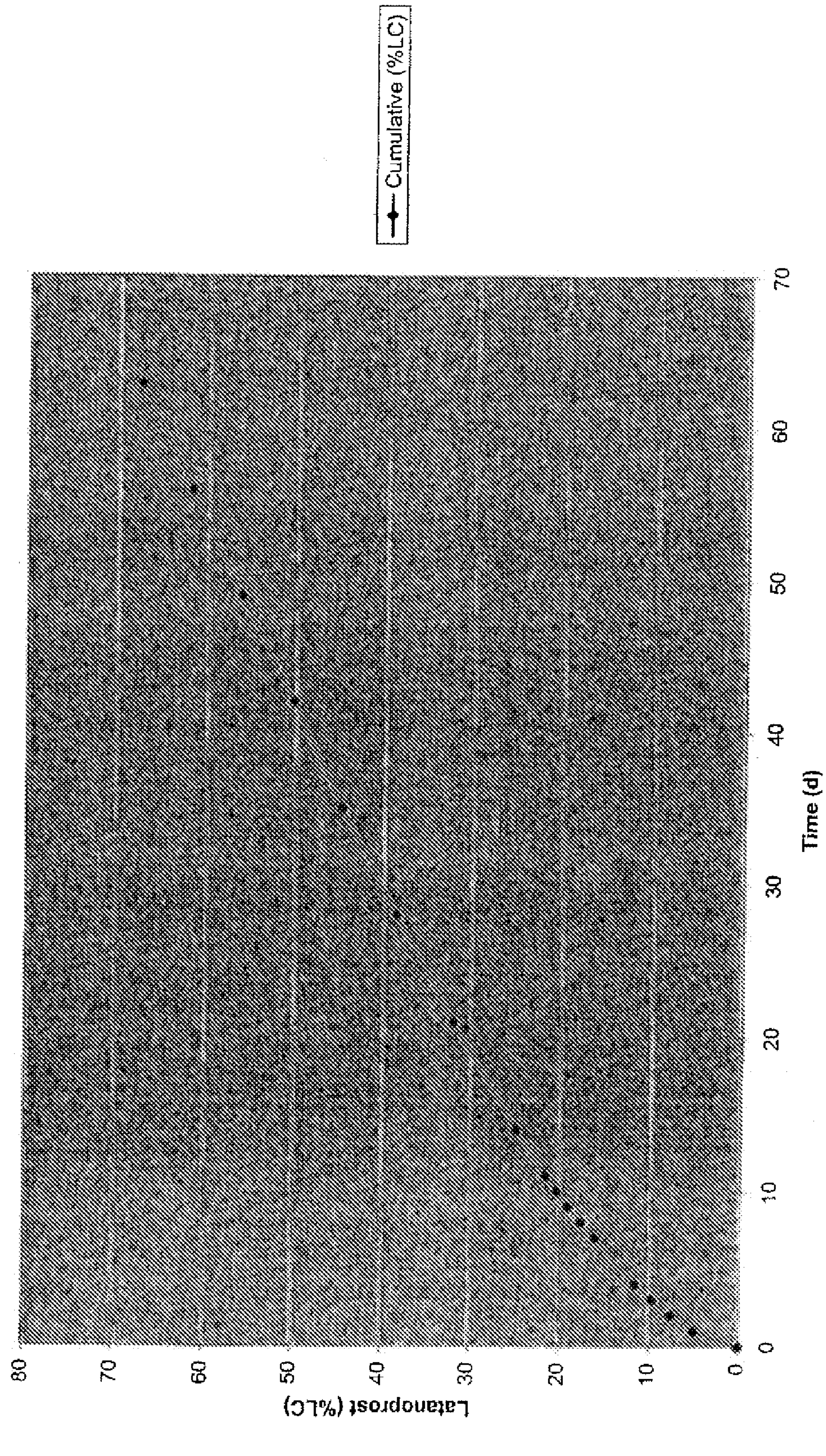
Cumulative (%LC)
Latanoprost (%LC)
0
10
20
30
40
50
60
70
80
Time (d)
0
10
20
30
40
50
60
70

FIG. 8B

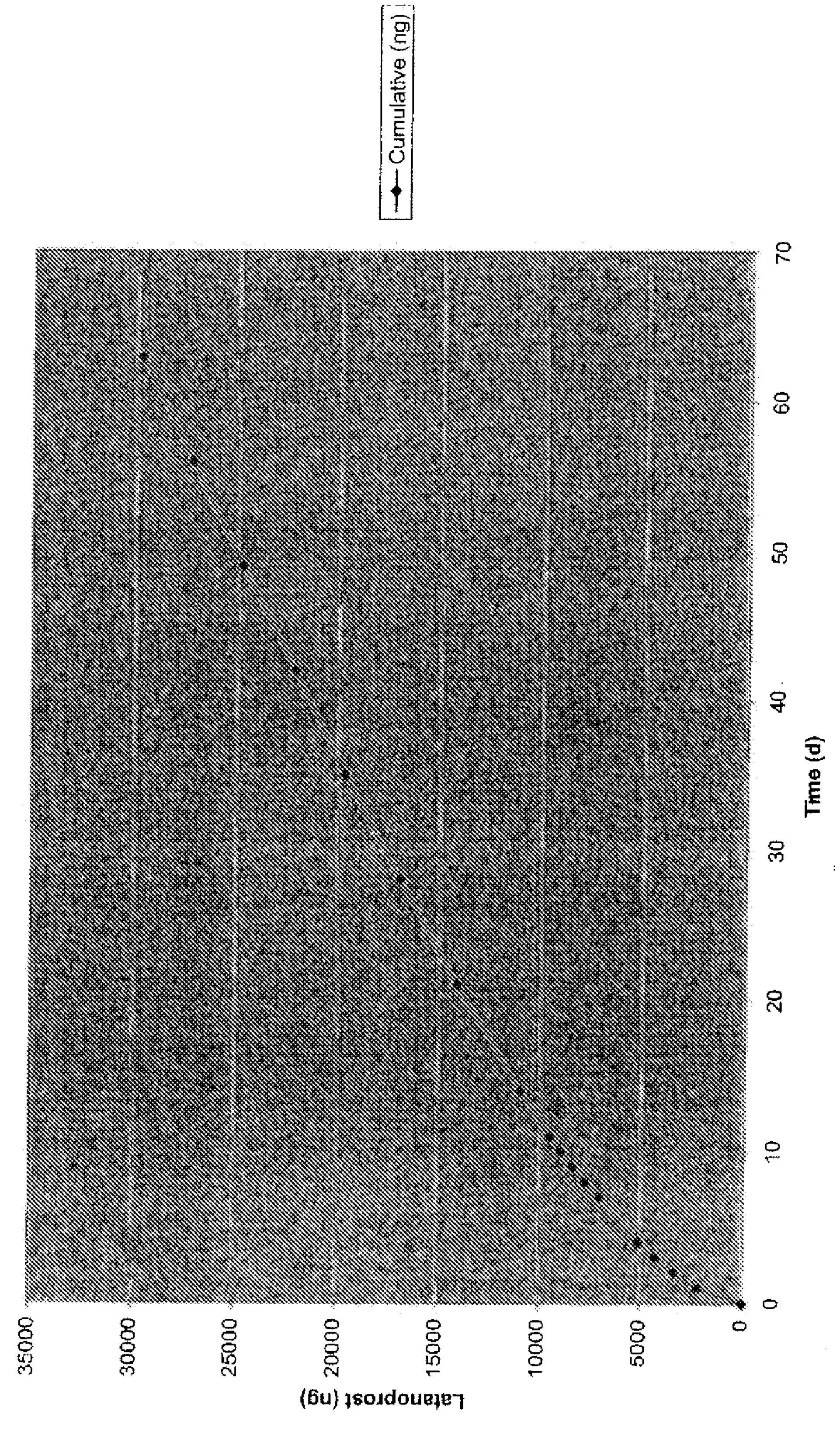
Cumulative (ng)
Latanoprost (ng)
35000
30000
25000
20000
15000
10000
5000
0
0
10
20
30
40
50
60
70
Time (d)

FIG. 8C

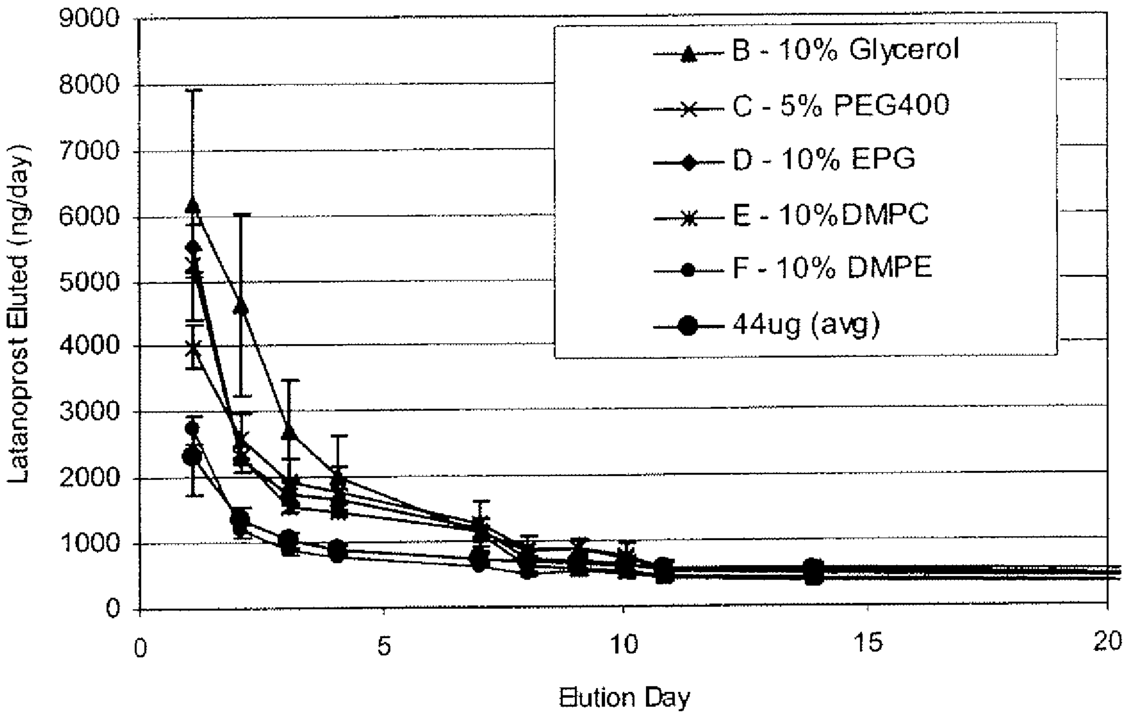
9000
8000
7000
6000
5000
4000
3000
2000
1000
0
Latanoprost Eluted (ng/day)
0
5
10
15
20
Elution Day
B - 10% Glycerol
C - 5% PEG400
D - 10% EPG
E - 10%DMPC
F - 10% DMPE
44ug (avg)

FIG. 9

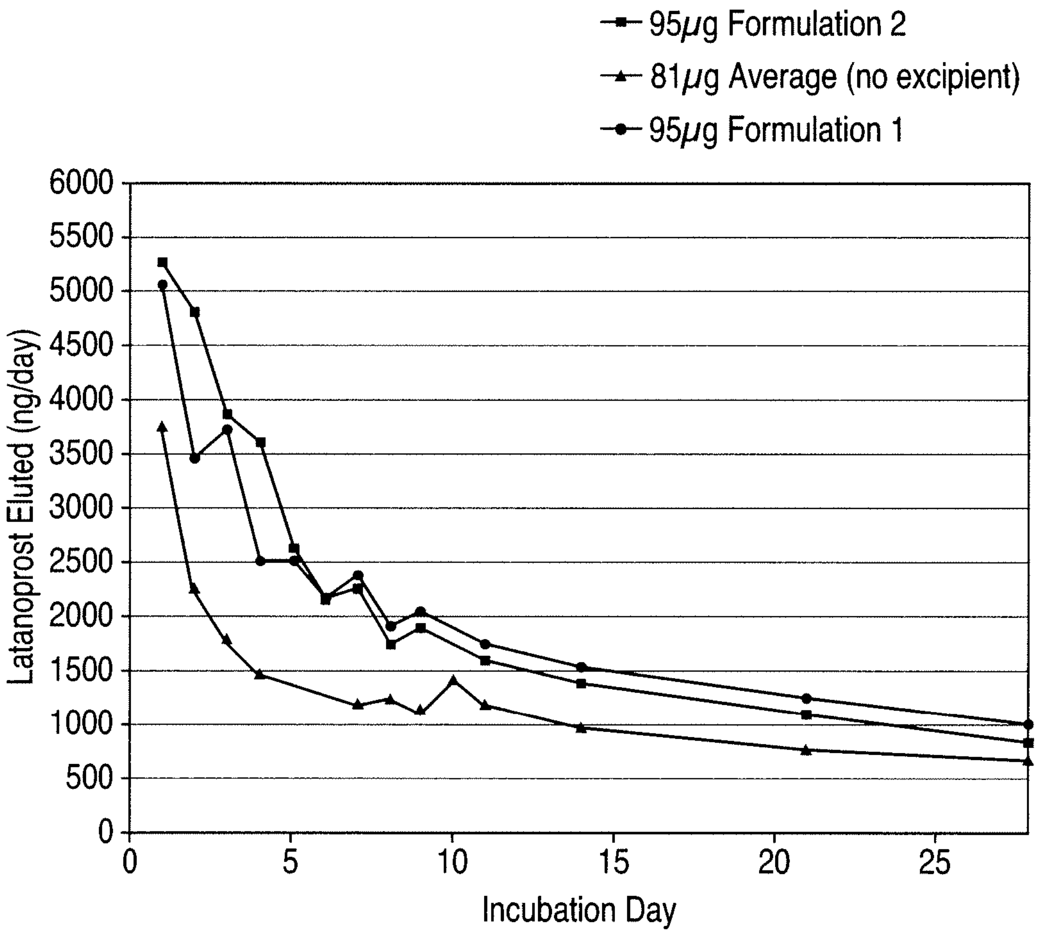
95μg Formulation 2
81μg Average (no excipient)
95μg Formulation 1
Latanoprost Eluted (ng/day)
6000
5500
5000
4500
4000
3500
3000
2500
2000
1500
1000
500
0
0
5
10
15
20
25
Incubation Day

FIG. 10

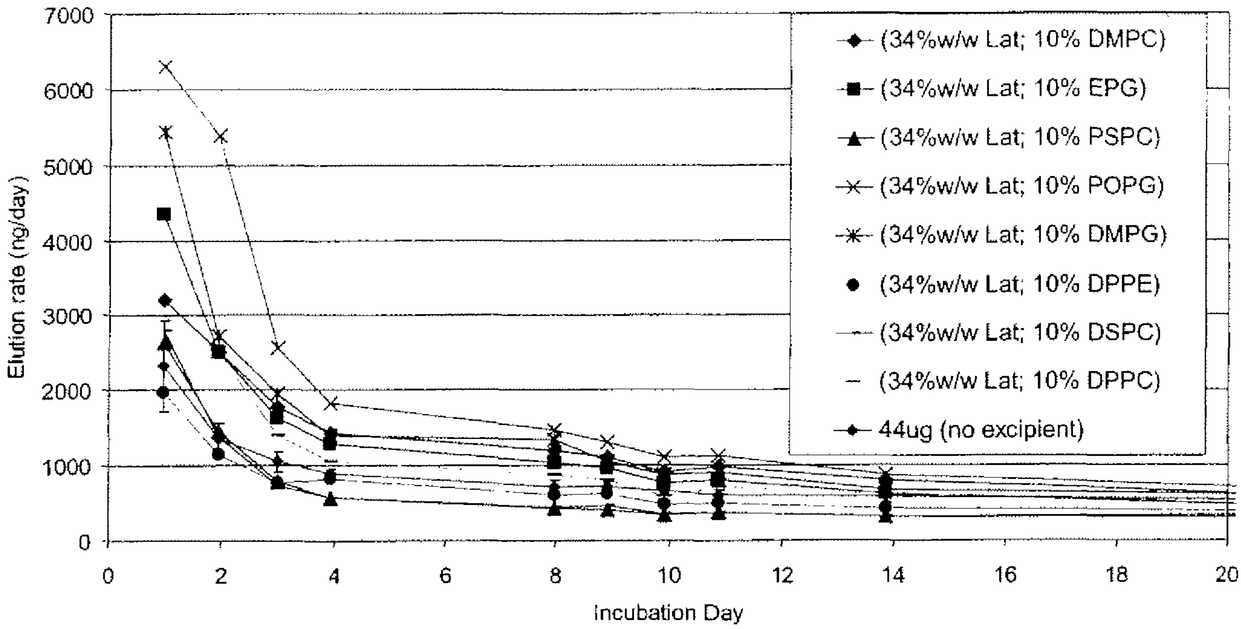
7000
6000
5000
4000
3000
2000
1000
0
Elution rate (ng/day)
0
2
4
6
8
10
12
14
16
18
20
Incubation Day
(34%w/w Lat; 10% DMPC)
(34%w/w Lat; 10% EPG)
(34%w/w Lat; 10% PSPC)
(34%w/w Lat; 10% POPG)
(34%w/w Lat; 10% DMPG)
(34%w/w Lat; 10% DPPE)
(34%w/w Lat; 10% DSPC)
(34%w/w Lat; 10% DPPC)
44ug (no excipient)

FIG. 11

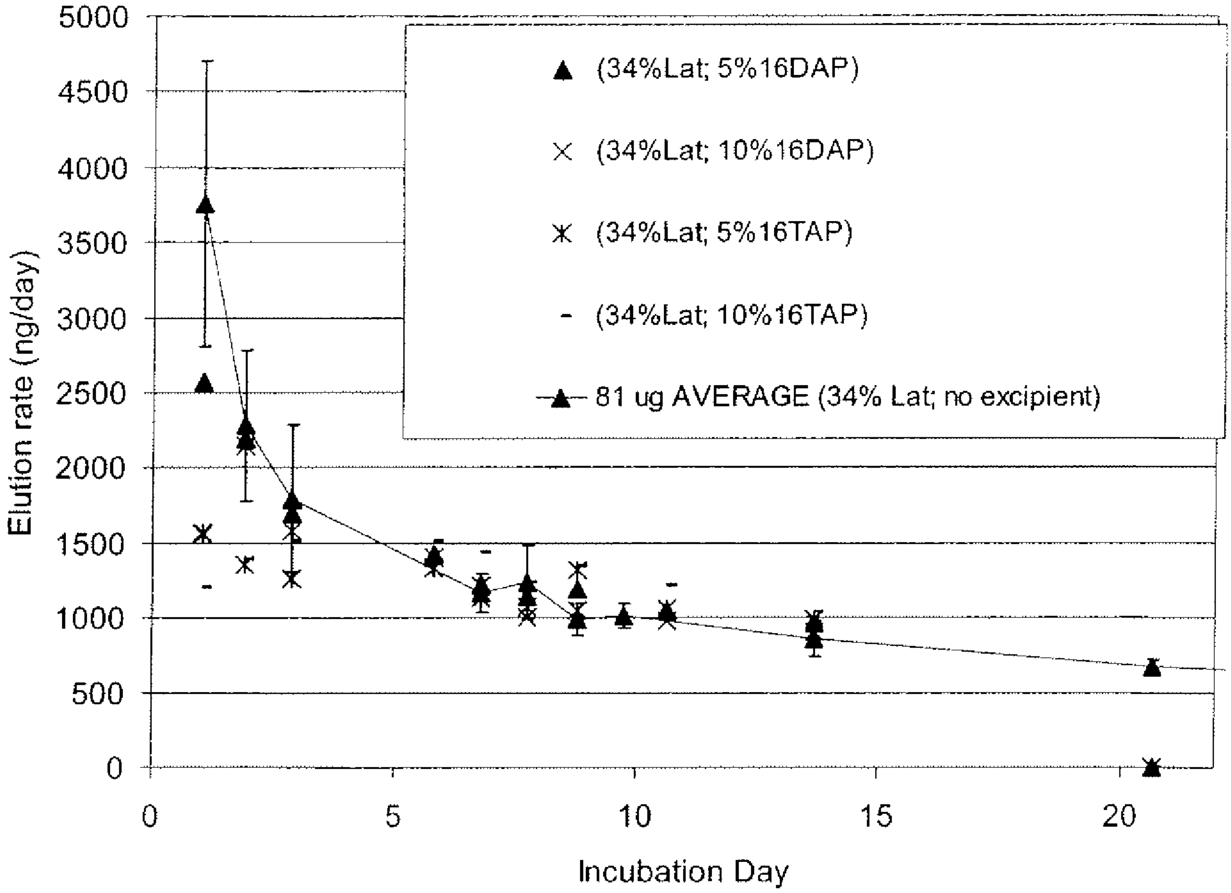
5000
4500
4000
3500
3000
2500
2000
1500
1000
500
0
Elution rate (ng/day)
0
5
10
15
20
Incubation Day
(34%Lat; 5%16DAP)
(34%Lat; 10%16DAP)
(34%Lat; 5%16TAP)
(34%Lat; 10%16TAP)
81 ug AVERAGE (34% Lat; no excipient)

FIG. 12

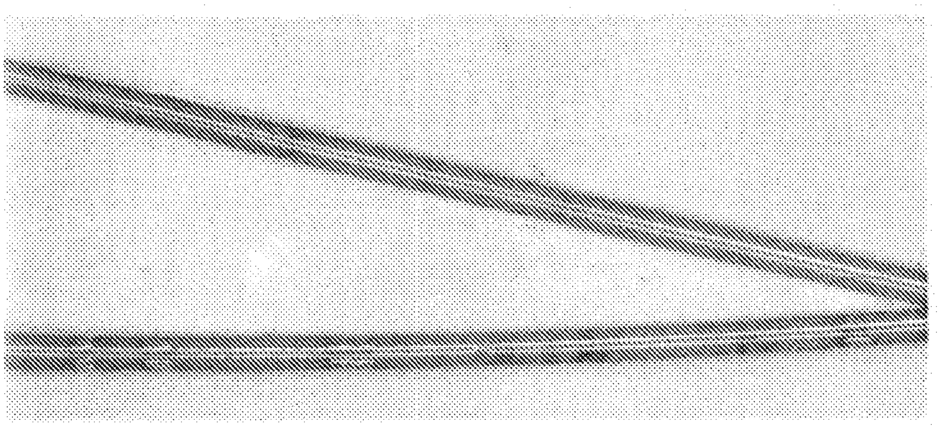

FIG. 13

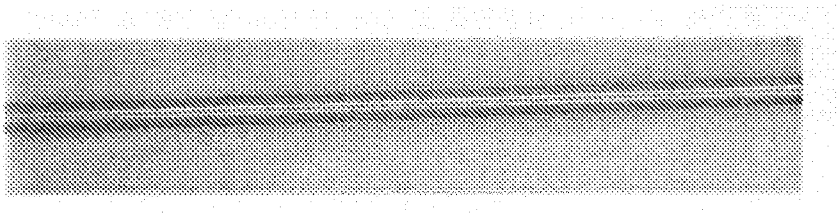

FIG. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2006022366 A
- WO 07115261 A
- US 97084007
- US 87186406
- US 69553707
- US 78777506
- US 69554507
- US 60970696 B
- US 97436707
- US 97069907
- US 97070907
- US 97072007
- US 97075507
- US 97082007
- US 61049347
- US 61049360 A
- US 61209630
- US 2755023P A
- US 61036816
- US 61049337
- US 61049329
- US 61049317
- US 61050901 A
- US 23198908
- US 61134271
- US 23198608
- US 82504704
- WO 2006014434 A
- US 2007065789 W
- WO 2007115259 A
- US 2008010487 W
- US 2008010479 W
- US 61139456
- WO 61049317 A, Jain
- WO 10825047 A, Odrich
- US 4281654 A, Shell
- US 20020055701 A
- US 772693 A
- US 20050129731 A
- US 979977 A
- US 20050048121 A
- US 861881 A
- US 20040170685 A
- US 788747 A
- US 60970709 B
- US 97069607
- US 61049360
- US 2755023 A
- US 61050901
- US 23198608 A
- WO 2009035562 A

### Non-patent literature cited in the description

- **TOUITOU, ELKA ; BARRY, BRIAN W.** Enhancement in Drug Delivery. Taylor & Francis Group, 2007, 527-548
- Orange Book. FDA Orange Book
- Medical management of glaucoma. **ALLEN RC.** Principles and Practice of Ophthalmology. WB Saunders and Co, 1994, 1569-1588
- Primary Open-Angle Glaucoma. Preferred Practice Pattern. American Academy of Ophthalmology, 2005
- **ANDERSON DR ; CHAUHAN B ; JOHNSON C ; KATZ J ; PATELLA VM ; DRANCE SM.** Criteria for progression of glaucoma in clinical management and in outcome studies. *Am J Ophthalmol,* 2000, vol. 130 (6), 827-829
- **ANDERSON DR.** Collaborative normal tension glaucoma study. *Curr Opin Ophthalmol.,* 2003, vol. 14 (2), 86-90
- **BAILEY IL ; LOVIE JE.** New design principles for visual acuity letter charts. *Am J Optom Physiol Opt,* November 1976, vol. 53 (11), 740-745
- **BALARAM M ; SCHAUMBERG DA ; DANA MR.** Efficacy and tolerability outcomes after punctal occlusion with silicone plugs in dry eye syndrome. *Am J Ophthalmol.,* 2001, vol. 131 (1), 30-36
- **BAUDOUIN C ; ROULAND JF ; NORDMANN JP ; BRON A ; PELEN F.** Efficacy of first- or second-line latanoprost on intraocular pressure and ocular symptoms in subjects with open-angle glaucoma or ocular hypertension [in French. *J Fr Ophtalmol.,* 2006, vol. 29 (6), 615-624
- **BENGTSSON B ; HEIJL A.** A long-term prospective study of risk factors for glaucomatous visual field loss in subjects with ocular hypertension. *J Glaucoma.,* 2005, vol. 14 (2), 135-138

- **BROWN MM ; BROWN GC ; SPAETH GL.** Improper topical self-administration of ocular medication among subjects with glaucoma. *Can J Ophthalmol.,* 1984, vol. 1, 2-5
- **CALLENDER M ; MORRISON PE.** A quantitative study of human tear proteins before and after adaption to non-flexible contact lenses. *Am J Optom Physiol Opt.,* 1974, vol. 51 (12), 939-945
- **COLEMAN AL.** Glaucoma. *Lancet,* 1999, vol. 354, 1803-10
- **DASGUPTA S ; OATES V ; BOOKHART BK ; VAZIRI B ; SCHWARTZ GF ; MOZAFFARI E.** Population-based persistency rates for topical glaucoma medications measured with pharmacy claims data. *Am J Manag Care,* 2002, vol. 8 (10), S255-S261
- **DIESTELHORST M ; SCHAEFER CP ; BEUSTERIEN KM et al.** Persistency and clinical outcomes associated with latanoprost and beta-blocker monotherapy: evidence from a European retrospective cohort study. *Eur J Ophthalmol,* 2003, vol. 13 (4), S21-S29
- **FISCELLA RG ; GELLER JL ; GRYZ LL ; WILENSKY J ; VIANA M.** Cost considerations of medical therapy for glaucoma. *Am J Ophthalmol.,* 1999, vol. 128 (4), 426-433
- **FREMONT AM ; LEE PP ; MANGIONE CM et al.** Patterns of care for open-angle glaucoma in managed care. *Arch Ophthalmol,* 2003, vol. 121 (6), 777-783
- **FUKANO Y ; ASADA H ; KIMURA A ; KAWAZU K.** Influence of benzalkonium chloride on the penetration of latanoprost into rabbit aqueous humor after ocular instillations. *AAPS Journal,* 2006, vol. 8 (S2, http://www.aapsj.org/abstracts/AM_2006/AAPS2006-001397.pdf
- **GORDON MO ; BEISER JA ; BRANDT JD et al.** The Ocular Hypertension Treatment Study: baseline factors that predict the onset of primary open-angle glaucoma. *Arch Ophthalmol.,* 2002, vol. 120 (6), 714-720, 829-830
- **GORDON MO ; KASS MA.** The Ocular Hypertension Treatment Study: design and baseline description of the participants. *Arch Ophthalmol,* 1999, vol. 117 (5), 573-583
- **GOSKONDA VR ; HILL RA ; KHAN MA ; REDDY IK.** Permeability of chemical delivery systems across rabbit corneal (SIRC) cell line and isolated corneas: a comparative study. *Pharm Dev Technol.,* 2000, vol. 5 (3), 409-416
- **GURWITZ JH ; GLYNN RJ ; MONANE M et al.** Treatment for glaucoma: adherence by the elderly. *Am J Public Health.,* 1993, vol. 83 (5), 711-716
- **HEIJL A ; LESKE MC ; BENGTSSON B ; HYMAN L ; BENGTSSON B ; HUSSEIN M.** Early Manifest Glaucoma Trial Group. Reduction of intraocular pressure and glaucoma progression: results from the Early Manifest Glaucoma Trial. *Arch Ophthalmol.,* 2002, vol. 120 (10), 1268-1279
- **HIGGINBOTHAM EJ ; GORDON MO ; BEISER JA et al.** The Ocular Hypertension Treatment Study: topical medication delays or prevents primary open-angle glaucoma in African American individuals. *Arch Ophthalmol.,* 2004, vol. 122 (6), 813-820
- **HORWATH-WINTER J ; THACI A ; GRUBER A ; BOLDIN I.** Long-term retention rates and complications of silicone punctal plugs in dry eye. *Am J Ophthalmol.,* 2007, vol. 144 (3), 441-444
- HypoTears® Safety Information. Novartis Ophthalmics, 22 October 2008
- **JAVITT JC ; METRICK S ; WANG F.** Costs of glaucoma in the United States. *Invest Ophthalmol Vis Sci,* 1995, vol. 36, S429
- **KASS MA ; GORDON MO ; KYMES SM.** Incorporating the results of the Ocular Hypertension Treatment Study into clinical practice. *Arch Ophthalmol.,* 2005, vol. 123 (7), 1021-1022
- **KASS MA ; HEUER DK ; HIGGINBOTHAM EJ et al.** The Ocular Hypertension Treatment Study: a randomized trial determines that topical ocular hypotensive medication delays or prevents the onset of primary open-angle glaucoma. *Arch Ophthalmol,* 2002, vol. 120 (6), 701-713
- **KIM BM ; OSMANOVIC SS ; EDWARD DP.** Pyogenic granulomas after silicone punctal plugs: a clinical and histopathologic study. *Am J Ophthalmol.,* 2005, vol. 139 (4), 678-684
- **KOBELT-NGUYEN G ; GERDTHAM UG ; ALM A.** Costs of treating primary open-angle glaucoma and ocular hypertension: a retrospective, observational two-year chart review of newly diagnosed subjects in Sweden and the United States. *J Glaucoma,* 1998, vol. 7, 95
- **LEIBOWITZ HM ; KRUEGER DE ; MAUNDER LR et al.** The Framingham Eye Study monograph: An ophthalmological and epidemiological study of cataract, glaucoma, diabetic retinopathy, macular degeneration, and visual acuity in a general population of 2631 adults, 1973-1975. *Surv Ophthalmol.,* 1980, vol. 24, 335-610
- **LEONARD R. ; ARLENE R.** Statistics on Vision Impairment: A Resource Manual. Gordon Research Institute of Lighthouse International, 2002
- **LESKE MC ; HEIJL A ; HYMAN L ; BENGTSSON B ; KOMAROFF E.** Factors for progression and glaucoma treatment: the Early Manifest Glaucoma Trial. *Curr Opin Ophthalmol.,* 2004, vol. 15 (2), 102-106
- **LEUNG EW ; MEDEIROS FA ; WEINREB RN.** Prevalence of ocular surface disease in glaucoma subjects. *J Glaucoma,* 2008, vol. 17 (5), 350-355

- **LICHTER PR ; MUSCH DC ; GILLESPIE BW et al.** CIGTS Study Group. Interim clinical outcomes in the Collaborative Initial Glaucoma Treatment Study comparing initial treatment randomized to medications or surgery. *Ophthalmology,* 2001, vol. 108 (11), 1943-1953
- **LIU JHK ; KRIPKE DF ; TWA MD et al.** Twenty-four-hour pattern of intraocular pressure in the aging population. *Invest Ophthalmol Vis Sci.,* 1999, vol. 40 (12), 2912-2917
- **MAIER PC ; FUNK J ; SCHWARZER G ; ANTES G ; FALCK-YTTER YT.** Treatment of ocular hypertension and open angle glaucoma: meta-analysis of randomised controlled trials. *BMJ,* 01 July 2005, vol. 331 (7509), 134
- Pharmacokinetics. **MINDEL JS.** Duane's Foundations of Clinical Ophthalmology. Lippincott Williams & Wilkins, 1995, vol. 3, 1-17
- **MUSCH DC ; LICHTER PR ; GUIRE KE ; STANDARDI CL.** CIGTS Study Group. The Collaborative Initial Glaucoma Treatment Study Design, Methods, and Baseline Characteristics of Enrolled Subjects. *Ophthalmology,* 1999, vol. 106, 653-662
- **NAKAMURA T ; YAMADA M ; TESHIMA M et al.** Electrophysiological characterization of tight junctional pathway of rabbit cornea treated with ophthalmic ingredients. *Biol Pharm Bull.,* 2007, vol. 30 (12), 2360-2364
- **NORDSTROM BL ; FRIEDMAN DS ; MOZAFFARI E ; QUIGLEY HA ; WALKER AM.** Persistence and adherence with topical glaucoma therapy. *Am J Ophthalmol.,* 2005, vol. 140 (4), 598-606
- **NORELL SE ; GRANSTRÖM PA.** Self-medication with pilocarpine among outsubjects in a glaucoma clinic. *Br J Ophthalmol.,* February 1980, vol. 64 (2), 137-41
- **O'DONOGHUE EP.** A comparison of latanoprost and dorzolamide in subjects with glaucoma and ocular hypertension: a 3 month, randomised study. *Br J Ophthalmol.,* 2000, vol. 84, 579-582
- **OSTERBERG L ; BLASCHKE T.** Adherence to Medication. *N Engl J Med.,* 2005, vol. 353 (5), 487-497
- **PARRISH RK ; PALMBERG P ; SHEU WP.** A comparison of latanoprost, bimatoprost, and travoprost in subjects with elevated intraocular pressure: a 12-week, randomized, masked-evaluator multicenter study. *Am J Ophthalmol,* 2003, vol. 135 (5), 688-703
- **PATEL SS ; SPENCER CM.** Latanoprost. A review of its pharmacological properties, clinical efficacy and tolerability in the management of primary open-angle glaucoma and ocular hypertension. *Drugs Aging,* 1996, vol. 9 (5), 363-378
- **PAWAR PK ; MAJUMDAR DK.** Effect of formulation factors on in vitro permeation of moxifloxacin from aqueous drops through excised goat, sheep, and buffalo corneas. *AAPS PharmSciTech,* 2006, vol. 7 (1), E13
- Preferred practice pattern - Primary Open-Angle Glaucoma. American Academy of Ophthalmology, 2005
- **QUIGLEY HA.** The number of persons with glaucoma worldwide. *Br J Ophthalmol,* 1996, vol. 80, 389
- **REARDON G ; SCHWARTZ GF ; MOZAFFARI E.** Subject persistency with pharmacotherapy in the management of glaucoma. *Eur J Ophthalmol.,* 2003, vol. 13 (4), S44-S52
- **REGNIER et al.** Ocular Effects of Topical 0.03% Latanoprost Solution in Normotensive Feline Eyes. *Vet Ophthalmol,* 2006, vol. 9 (1), 39-43
- **ROSSETTI L ; GANDOLFI S ; TRAVERSE C et al.** An evaluation of the rate of nonresponders to latanoprost therapy. *J Glaucoma,* 2006, vol. 15 (3), 238-243
- **ROULAND JF ; BERDEAUX G ; LAFUMA A.** The economic burden of glaucoma and ocular hypertension: implications for subject management: a review. *Drugs Aging,* 2005, vol. 22 (4), 315-321
- **SAKAMOTO A ; KITAGAWA K ; TATAMI A.** Efficacy and retention rate of two types of silicone punctal plugs in subjects with and without Sjögren's Syndrome. *Cornea,* 2004, vol. 23 (3), 249-254
- **SCHOLZ M ; LIN JE ; LEE VH ; KEIPERT S.** Pilocarpine permeability across ocular tissues and cell cultures: influence of formulation parameters. *J Ocul Pharmacol Ther.,* 2002, vol. 18 (5), 455-468
- **SCHWARTZ GF ; REARDON G ; MOZAFFARI E.** Persistency with latanoprost or timolol in primary open angle glaucoma suspects. *Am J Ophthalmol.,* 2004, vol. 137 (1), S13-S16
- **SHAYA FT ; MULLINS CD ; WONG W ; CHO J.** Discontinuation rates of topical glaucoma medications in a managed care population. *Am J Manag Care.,* 2002, vol. 8 (10), S271-S277
- **SILLIMAN NP.** Xalatan Ophthalmic Solution 0.005% (50 mg/mL) (Pharmacia) 06/06/1996. *Approval: Statistical Review,* 1995
- **SPOONER JJ ; BULLANO MF ; IKEDA LI et al.** Rates of discontinuation and change of glaucoma therapy in a managed care setting. *Am J Manag Care,* 2002, vol. 8 (10), S262-S270
- The AGIS Investigators. The Advanced Glaucoma Intervention Study (AGIS): 7. The relationship between control of intraocular pressure and visual field deterioration. *Am J Ophthalmol.,* 2000, vol. 130 (4), 429-440
- Primary open angle glaucoma. **THOMAS JV.** Principles and Practice of Ophthalmology. WB Saunders and Co, 1994, 1342-1345

- **THYLEFORS B ; NEGREL A-D ; PARARAJASEGARAM R ; DADZIE KY.** Global data on blindness. *Bull World Health Org,* 1995, vol. 73, 115-121
- **URQUHART J.** The odds of the three nons when an aptly prescribed medicine isn't working: non-compliance, non-absorption, non-response. *Br J Clin Pharmacol.,* 2002, vol. 54 (2), 212-220
- **WALDOCK A ; SNAPE J ; GRAHAM CM.** Effects of glaucoma medications on the cardiorespiratory and intraocular pressure status of newly diagnosed glaucoma subjects. *Br J Ophthalmol.,* 2000, vol. 84 (7), 710-713
- **WHITCUP SM et al.** A randomized, doube masked, multicenter clinical trial comparing latanoprost and timolol for the treatment of glaucoma and acular hypertension. *Br J Ophthalmol,* 2003, vol. 87, 57-62
- **WINFIELD AJ et al.** A study of the causes of non-compliance by subjects prescribed eyedrops. *Br J Ophthalmol,* August 1990, vol. 74 (8), 477-80
- **WOODWARD DF et al.** Pharmalogical Characterization of a Novel Antiglaucoma Agent, (AGN 192024). *J Pharmacology and Experimental Therapeutics,* 2003, vol. 305 (2), 772-785
- Xalatan® (latanoprost ophthalmic solution) (50 μg/mL). Product Monograph. Pfizer Canada Inc, 2003
- Xalatan® (latanoprost ophthalmic solution) 0.005% (50 μg/mL) prescribing information. Pharmacia & Upjohn Company, 01 October 2007